# EUROPEAN PATENT APPLICATION

(11) **EP 1 598 428 A1**
(43) Date of publication of application: **23.11.2005**
(21) Application number: 04011790.5
(22) Date of filing: 18.05.2004
(51) Int. Cl.: C12Q 1/68, A61K 48/00, A01K 67/027, G01N 33/53

(54) **Methods and kits to detect Hereditary angioedema type III**

(71) Applicant: Dewald, Georg, 53115 Bonn (DE)
(72) Inventor: Dewald, Georg, 53115 Bonn (DE)
(74) Representative: Vossius & Partner

(57) **Abstract**

The present invention relates to a method of diagnosing hereditary angioedema type III (HAE III) or a predisposition thereto in a subject being suspected of having developed or of having a predisposition to develop a hereditary angioedema type III or in a subject being suspected of being a carrier for hereditary angioedema type III, the method comprising determining in vitro from a biological sample of said subject the presence or absence of a disease-associated mutation in a nucleic acid molecule regulating the expression of or encoding coagulation factor XII; wherein the presence of such a mutation is indicative of a hereditary angioedema type III or a predisposition thereto. The present invention also relates to a method of diagnosing hereditary angioedema type III (HAE III) or a predisposition thereto in a subject being suspected of having developed or of having a predisposition to develop a hereditary angioedema type III or in a subject being suspected of being a carrier for hereditary angioedema type III, the method comprising assessing the presence, amount and/or activity of coagulation factor XII in said subject and including the steps of: (a) determining from a biological sample of said subject in vitro, the presence, amount and /or activity of: (i) a (poly)peptide encoded by the coagulation factor XII gene; (ii) a substrate of the (poly)peptide of (i); or (iii) a (poly)peptide processed by the substrate mentioned in (ii); (b) comparing said presence, amount and/or activity with that determined from a reference sample; and (c) diagnosing, based on the difference between the samples compared in step (b), the pathological condition of a hereditary angioedema type III or a predisposition thereto. The present invention also relates to a method of identifying a compound modulating coagulation factor XII activity which is suitable as a medicament or a lead compound for a medicament for the treatment and/or prevention of hereditary angioedema type III, the method comprising the steps of: (a) in vitro contacting a coagulation factor XII (poly)peptide or a functionally related (poly)peptide with the potential modulator; and (b) testing for modulation of coagulation factor XII activity, wherein modulation of coagulation factor XII activity is indicative of a compound's suitability as a medicament for the treatment and/or prevention of hereditary angioedema type III. Furthermore, the present invention relates to gene therapy methods and to a kit for diagnosing hereditary angioedema type III.

## Description

The present invention relates to a method of diagnosing hereditary angioedema type III (HAE III) or a predisposition thereto in a subject being suspected of having developed or of having a predisposition to develop a hereditary angioedema type III or in a subject being suspected of being a carrier for hereditary angioedema type III, the method comprising determining in vitro from a biological sample of said subject the presence or absence of a disease-associated mutation in a nucleic acid molecule regulating the expression of or encoding coagulation factor XII; wherein the presence of such a mutation is indicative of a hereditary angioedema type III or a predisposition thereto. The present invention also relates to a method of diagnosing hereditary angioedema type III (HAE III) or a predisposition thereto in a subject being suspected of having developed or of having a predisposition to develop a hereditary angioedema type III or in a subject being suspected of being a carrier for hereditary angioedema type III, the method comprising assessing the presence, amount and/or activity of coagulation factor XII in said subject and including the steps of: (a) determining from a biological sample of said subject in vitro, the presence, amount and /or activity of: (i) a (poly)peptide encoded by the coagulation factor XII gene; (ii) a substrate of the (poly)peptide of (i); or (iii) a (poly)peptide processed by the substrate mentioned in (ii); (b) comparing said presence, amount and/or activity with that determined from a reference sample; and (c) diagnosing, based on the difference between the samples compared in step (b), the pathological condition of a hereditary angioedema type III or a predisposition thereto. The present invention also relates to a method of identifying a compound modulating coagulation factor XII activity which is suitable as a medicament or a lead compound for a medicament for the treatment and/or prevention of hereditary angioedema type III, the method comprising the steps of: (a) in vitro contacting a coagulation factor XII (poly)peptide or a functionally related (poly)peptide with the potential modulator; and (b) testing for modulation of coagulation factor XII activity, wherein modulation of coagulation factor XII activity is indicative of a compound's suitability as a medicament or a lead compound for a medicament for the treatment and/or prevention of hereditary angioedema type III. Furthermore, the present invention relates to gene therapy methods and to a kit for diagnosing hereditary angioedema type III.

Several documents are cited throughout the text of this specification. The disclosure content of the documents cited herein (including any manufacturer's specifications, instructions, etc.) is herewith incorporated by reference.

All or any combination of steps (including single steps only) carried out in the method of the present invention and cited throughout this specification can be carried out in any combination of in vivo, ex vivo or in vitro.

The conventional or classic forms of hereditary angioedema (HAE) are known to be autosomal dominant disorders. Two types are recognized (Rosen et al. 1965, Science 148: 957-958), both related to a C1 inhibitor deficiency caused by mutations in the C1 inhibitor gene (Bissler et al. 1997, Proc. Assoc. Am. Physicians 109: 164-173; Zuraw & Herschbach 2000, J. Allergy Clin. Immunol. 105: 541-546; Bowen et al. 2001, Clin. Immunol. 98: 157-163). The defective gene produces either no C1 inhibitor (HAE type I) or a dysfunctional C1 inhibitor (HAE type II). Recently, a further type of hereditary angioedema has been described (Bork et al. 2000, Lancet 356: 213-217; Binkley & Davis 2000, J. Allergy Clin. Immunol. 106: 546-550; Martin et al. 2001, J. Allergy Clin. Immunol. 107: 747), and it appears that this new type is closely related, possibly identical, to a disease entity described already in 1986 by Warin et al. (Br. J. Dermatol. 115:731-734) in two sisters. In patients with this new type of inherited/familial angioedema, C1 inhibitor protein levels and C1 inhibitor function (as determined by antigenic and functional assays) are normal. This disease has been termed HAE type III by Bork et al. 2000 (Lancet 356: 213-217). The genetic defect underlying hereditary angioedema type III is still unknown. Until now, this disease has been reported exclusively in women, but from pedigree analysis one must postulate the existence of male carriers (Binkley & Davis 2000; Martin et al. 2001). Inheritance is assumed to be autosomally dominant (Binkley and Davis, 2000; Martin et al., 2001; Binkley and Davis 2001, J. Allergy Clin. Immunol. 107: 747-748), male-to-male transmission has been observed in one family (Martin et al., 2001). Nevertheless, the possibility of genetic heterogeneity, including the possibility of X-chromosomal inheritance eventually in some families, has been discussed (Bork et al. 2000; Martin et al. 2001; Binkley & Davis 2001). The clinical manifestation of HAE type III, in particular regarding frequency and intensity of symptoms, appears to be quite variable, and penetrance of the disease can be reduced (Bork et al. 2000, Lancet 356: 213-217; Bork et al. 2003, Am. J. Med. 114: 294-298). One therefore might speculate that some patients diagnosed as 'idiopathic angioedema' eventually are affected by hereditary angioedema type III, and that the disease has not (yet) manifested in any of their relatives (Bork et al. 2003, Am. J. Med. 114: 294-298). In about two thirds of women affected with HAE type III angioedema symptoms are precipitated or exacerbated by oral contraceptives or hormone replacement therapy (Bork et al. 2003), pregnancy might also be an important precipitating factor, at least in some families (Binkley & Davis 2000). It is assumed that exogenous, respectively endogenous estrogens are responsible for these precipitating or exacerbating effects (Binkley & Davis 2000, 2001; Bork et al. 2000, 2003). Based on presently available information, the clinical presentation of HAE type III appears to be highly similar to HAE type I or II, except for the unique occurrence in women.

The deficiency of functional C1 esterase inhibitor provides a useful means of detecting hereditary angioedema types I and II, as the substitution with a pharmaceutical preparation of human C1 inhibitor does provide a useful means of treating as well as of preventing these types of hereditary angioedema. In contrast, an effective method for the detection, treatment, and prevention of hereditary angioedema type III still remains to be identified.

Thus, the technical problem underlying the present invention was to provide means and methods for diagnosis of hereditary angioedema type III or a predisposition thereto, as well as for prevention and treatment of hereditary angioedema type III.

The solution to this technical problem is achieved by providing the embodiments characterized in the claims.

Accordingly, the present invention relates to a method of diagnosing hereditary angioedema type III (HAE III) or a predisposition thereto in a subject being suspected of having developed or of having a predisposition to develop a hereditary angioedema type III or in a subject being suspected of being a carrier for hereditary angioedema type III, the method comprising determining in vitro from a biological sample of said subject the presence or absence of a disease-associated mutation in a nucleic acid molecule regulating the expression of or encoding coagulation factor XII; wherein the presence of such a mutation is indicative of a hereditary angioedema type III or a predisposition thereto.

The term "nucleic acid" or "nucleic acid molecule" refers to DNA or RNA, including genomic DNA, cDNA, mRNA, hnRNA etc as well as chimeras thereof. Included are artificially modified nucleic acid molecules carrying chemically modified bases. All nucleic acid molecules may be either single or double stranded.

In principle, the detection of at least one disease-associated mutation such as 2, 3, 4, 5, 6, 7, 8, 9, 10 or more mutations or combinations of various different mutations in at least one allele is an indication that the subject to be diagnosed either with respect to a potentially existing disease predisposition or susceptibility or because of being affected by the disease is a carrier. In general, if a disease-associated mutation is dominant, it may be causative for the onset or progress of the disease and a diagnosis of heterozygosity as only of its presence in the genome at all, will be indicative of the subject being prone to developing the disease if it does not already suffer from it. A recessive character of a mutation will more likely indicate that only its homozygous occurrence will have a direct impact on the onset or progress of the disease, whereas its occurrence in heterozygous form will rather qualify the subject as a carrier only, unless other concomitantly occurring mutations contribute to the onset or progress of the disease.

The term "diagnosing" means assessing whether or not an individual or a subject has a specific mutation linked with hereditary angioedema type III and concluding from the presence of said mutation that the individual or subject has a hereditary angioedema type III (HAE III), or a predisposition thereto, a predisposition to develop a hereditary angioedema type III or is a carrier for hereditary angioedema type III.

The term "hereditary angioedema" refers to an inherited abnormality to cause skin swellings, gastrointestinal symptoms (abdominal pain attacks) due to edema of the intestinal wall, or edema of the tongue, or laryngeal edema, which may ultimately result in death by asphyxiation. Until recently two forms of hereditary angioedema (HAE), type I and type II, were recognized (Rosen et al. 1965, Science 148: 957-958); both are inherited in an autosomal dominant fashion and are related to a C1 inhibitor deficiency caused by mutations in the C1 inhibitor gene (Bissler et al. 1997, Proc. Assoc. Am. Physicians 109: 164-173; Zuraw & Herschbach 2000, J. Allergy Clin. Immunol. 105: 541-546; Bowen et al. 2001, Clin. Immunol. 98: 157-163). The defective gene produces either no C1 inhibitor (HAE type I) or a dysfunctional C1 inhibitor (HAE type II). With respect to hereditary angioedema due to C1 inhibitor deficiency, it is generally assumed that bradykinin (and eventually related kinins) is an important mediator of angioedema development (Nussberger et al. 1998, Lancet 351: 1693-1697; Kaplan et al. 2002, J. Allergy Clin. Immunol. 109 : 195-209; Han et al. 2002, J. Clin. Invest. 109 : 1057-1063; Cugno et al. 2003, Int. Immunopharmacol. 3: 311-317). However, also a kinin derived from complement component C2 - following an eventually increased or uncontrolled activation of the classical complement pathway - has been considered to be of pathophysiological significance (Donaldson et al. 1977, Trans. Assoc. Am. Physicians 90: 174-183; Strang et al. 1988, J. Exp. Med. 168: 1685-1698).

The term "hereditary angioedema type III", as used throughout the description of the present invention, relates to the disease described by Bork et al. 2000 (Lancet 356: 213-217) and Binkley and Davis 2000 (J. Allergy Clin. Immunol. 106: 546-550), the disease described by Warin et al. 1986 (Br. J. Dermatol. 115: 731-734) probably being closely related or even identical. Type III of hereditary angioedema is characterized by similar symptoms as observed in HAE I and II, however, the blood plasma level and activity of C1 esterase inhibitor are normal and until now the disease has exclusively been reported in women. According to the present invention, hereditary angioedema type III may include several potential subtypes: Binkley & Davis 2001 (J. Allergy Clin. Immunol. 107: 747-748) suggested to differentiate between (1) an estrogen-sensitive type (in which symptoms worsen but are not strictly dependent on high estrogen levels) and (2) an estrogen-dependent type (in which there is an absolute dependence of symptoms). In addition, according to data presented by Bork et al. (2003, 2000), it appears that in almost one third of patients symptoms are not influenced by estrogen exposure; these patients may represent a third subtype (3) of HAE type III regarding estrogen-sensitivity. A fourth subtype (4) of HAE type III appears to be the disease described by Warin et al. 1986: a familial type of recurrent angioedema with normal C1 inhibitor levels, oestrogen-induced, but with occasional symptoms of urticaria. A fifth subtype (5) may be 'HAE type III with negative family history', a term that is understood here as being equivalent to 'idiopathic angioedema', i.e. a recurrent angioedema that cannot be attributed to any of the C1 inhibitor-related forms of hereditary or acquired angioedema or to any of the known drug-induced and physical causes: certain patients classified as cases of "idiopathic angioedema" may, in fact, represent patients with HAE type III, namely HAE type III patients where the disease has not yet manifested in any relative (e.g. because of reduced penetrance), or HAE type III patients with a negative family history because of a *de novo* mutation. Finally, a sixth subtype (6) of HAE type III may be manifested in men, eventually only in the presence of certain (genetic or environmental) precipitating factors.

The term "predisposition", in accordance with the present invention, refers to a genetic condition that (a) increases the risk for the development of a disease or promotes or facilitates the development of a disease and/or that (b) facilitates to pass on to the offspring specific alleles of a gene increasing the risk for or promoting the development of such condition or disease.

The term "biological sample", in accordance with the present invention, relates to the specimen taken from a mammal. Preferably, said specimen is taken from hair, skin, mucosal surfaces, body fluids, including blood, plasma, serum, urine, saliva, sputum, tears, liquor cerebrospinalis, semen, synovial fluid, amniotic fluid, breast milk, lymph, pulmonary sputum, bronchial secretion, or stool.

The term "mutation" comprises, inter alia, substitutions, additions, insertions, inversions, duplications or deletions within nucleic acid molecules, wherein one or more nucleotide positions can be affected by a mutation. These mutations occur with respect to the wild-type nucleic acid sequence. As the "wild-type" or "normal" nucleic acid sequence of the coagulation factor XII gene is considered herein the sequence (bases 1 to 10616) given under GenBank acc. no. AF 538691 and, with respect to extended flanking sequences, the sequence given in the July 2003 human reference sequence of the UCSC Genome Browser, v.53 (vide infra). A mutation may affect preferably up to 1, 2 , 3 , 4 , 5 , 6 , 7 , 8 , 9 ,10 or even of up to 20, 30, 40, 50, or up to 1000 nucleotides. However, it is also conceivable that even larger sequences are affected. Therefore, the term "mutation" also relates to, e.g., a nucleotide deletion, substitution or insertion of up to 10000 or up to 20000 nucleotides, also comprising the situation when the entire coding, non-coding and/or regulating sequence of a gene is affected. Mutations can involve coding or non-coding gene regions. The term "non-coding" preferably relates to introns, to the non-coding parts of exons, to 5'- and 3'-flanking regulatory sequences, thus also to expression control sequences including control elements such as promoter, enhancer, silencer, transcription terminator, polyadenylation site. It is well known to the person skilled in the art that mutations in these regions of a gene can have a substantial impact on gene expression, eventually also with respect to specific tissues. For example, mutations in these sites can result in a nearly complete shutdown of gene expression or in a drastic overexpression. However, mutations in non-coding regions can also exert important effects by altering the splicing process; such mutations, for example, can affect the intron consensus sequences at the splice and branch sites, sometimes they activate cryptic sites, or create ectopic splice sites.

On the other hand, a mutation can also reside in the coding region of a gene and severely affect the protein's structural and/or functional characteristics, for example by causing amino acid substitutions. However, even so-called silent or synonymous mutations must not necessarily be silent. For example, mutations within exonic splicing enhancers or silencers may affect mRNA splicing, which may for example alter protein structure or cause phenotypic variability and variable penetrance of mutations elsewhere in the gene (Liu H.-X. et al. 2001, Nature Genet. 27: 55-58; Blencowe 2000, TIBS 25: 106-110; Verlaan et al. 2002, Am. J. Hum. Genet. 70; Pagani et al. 2003, Hum. Mol. Genet. 12: 1111-1120).

However, it is well known in the art that not any deviation from a given reference sequence must necessarily result in a disease condition or a predisposition thereto. For example the gene encoding human coagulation factor XII is known to occur in a number of variations comprising polymorphisms or polymorphic variants such as those deposited in the databank of Seattle (http://pga.gs.washington.edu, University of Washington, 'Seattle SNPs').

The term "polymorphism" or "polymorphic variant" means a common variation in the sequence of DNA among individuals (NHGRI glossary). "Common" means that there are two or more alleles that are each present at a frequency of at least 1% in a population. Usually it is understood, that polymorphisms, or at least the majority of polymorphisms, represent variations that are benign, functionally neutral, not having an adverse effect on gene function. However, it is also clear that polymorphic variants exist which can have an impact with respect to the development of a disease. This impact can be not only a disease-predisposing one, but, in certain cases, it can also be a protective effect reducing the risk of disease manifestation.

Taking into account the existence of polymorphic variants, it is reasonable to consider the existence of numerous alternative wild-type sequences. For various purposes of the present invention, for example for the design of nucleotide probes and primers and also for the design of oligonucleotides to be used therapeutically, it will be important to carefully take into account the existence of such variant sequences.

Although the term "mutation" basically describes any alteration or change in a gene from its natural state, it is often understood as a disease-causing change, as a change that causes a disorder or the inherited susceptibility to a disorder.

For the skilled artisan and under certain circumstances, the terms "polymorphic variant" ("polymorphism") and "mutation" have the same connotation and refer to the same molecular phenomenon, namely alteration in or deviation from a paradigmatic wild-type sequence.

For the purpose of the present invention, the term "disease-associated mutation" refers to a mutation in a nucleic acid molecule regulating the expression of or encoding coagulation factor XII and which is linked with hereditary angioedema type III or a predisposition thereto. In accordance with the present invention, a "disease-associated mutation" is preferably a rare mutation, preferably with a frequency <1%, and more preferably a mutation with an important disease-causing effect, a dominant mutation. Nevertheless, in accordance with the present invention, it is also envisaged that polymorphic variants exist that can have an influence on disease predisposition and/or the onset or progress of a disease (vide infra), and which, thus, also represent a "disease-associated mutation". It is important to note that an affected individual may carry more than one disease-associated mutation. In order to determine whether or not a mutation is disease-associated, the person skilled in the art may for example compare the frequency of a specific sequence change in patients affected by HAE type III with the frequency of this sequence change in appropriately chosen control individuals, preferably individuals who never showed any angioedema symptoms, and conclude from a statistically significantly deviating frequency in the patient group that said mutation is a disease-associated mutation. The person skilled in the art knows how to design such a comparison of patients and controls. For example, patients and controls should be matched for age and sex. Controls could be individuals assumed to be healthy, like blood donors, but also a population-based control sample appears to be possible, although it is appreciated that among such samples there might be a small percentage of individuals included who have a predisposition for the disease. Thus, preferably, controls should be individuals who never experienced any angioedema symptoms According to the present invention, the term "statistically significant" describes a mathematical measure of difference between groups. The difference is said to be statistically significant if it is greater than what might be expected to happen by chance alone. Preferably, a P-value < 0.10, more preferred a P-value < 0.05, even more preferred, a P-value < 0.01, calculated without using any corrections like those for multiple testing, is considered to be indicative of a significant difference.

In cases where more than one mutation is present in a nucleic acid molecule, wherein said mutation is linked with HAE type III, it may suffice to detect the presence of one mutation only or of a lower number of mutations than are actually present in the nucleic acid molecule and associated with HAE type III. Normally, it is not relevant for the purpose of diagnosis, whether such associated mutations are solely indicative, thus having for example a bystander effect, and not causative or whether they are causative for the onset or progress of the disease.

GenBank accession number AF538691 lists a consensus sequence of the human coagulation factor XII gene and a number of polymorphic variants observed in Caucasian and Negroid individuals. For a large part, these and potentially existing other polymorphic variants may be functionally neutral. Nevertheless, it is possible that at least some polymorphic variants are not neutral, i.e. that they can exhibit functional, quantitative or qualitative consequences like, for example, influencing directly the susceptibility or predisposition for the development of HAE type III or modulating the pathogenic effect of another mutation associated with hereditary angioedema type III.

For example, it is envisaged that a common polymorphism (46C/T) in the 5'-UTR (in exon 1) of the human coagulation factor XII gene can be of importance for the present invention. It is known that this polymorphism is significantly associated with the plasma concentration of coagulation factor XII (Kanaji et al. 1998, Blood 91: 2010-2014), the T allele being associated with a decreased translation efficiency; in functional and antigenic assays, individuals with the genotype C/C show 170% of the concentration seen in pooled normal plasma, whereas in individuals with the genotype T/T the factor XII plasma concentration is 80% of that seen in pooled normal plasma. In accordance with the present invention, one, therefore, may consider the C allele being a risk factor whose presence can increase the risk for the development of angioedema, for example in case that it is present in one haplotype with a dominant disease-associated mutation.

Thus, in a less preferred alternative, it is conceivable that, in fact, some of said polymorphic variants represent a disease-associated mutation. It is also envisaged that such a situation might arise from linkage disequilibrium phenomena. With these limitations in mind, the deposited consensus sequence mentioned above, is considered herein to represent the "wild-type" sequence.

It is important to note that the term "nucleic acid molecule regulating the expression of or encoding coagulation factor XII" preferably comprises the complete genomic sequence of the coagulation factor XII gene including extended flanking regulatory sequences (vide infra) as well as sequences or nucleic acid molecules which are physically unrelated to the coagulation factor XII gene but which exert regulatory effects on the expression of coagulation factor XII. The term "nucleic acid molecule regulating the expression of or encoding coagulation factor XII" also refers to portions of the above sequences, for example the promoter of said gene.

The term "regulating the expression" means influencing, including increasing or decreasing transcription or translation. Accordingly, increasing or decreasing means producing more or less, respectively, RNA or (poly)peptides. The term "regulating the expression" also refers to influencing splicing processes, as well as the tissue-specific expression of a gene. The skilled person knows that expression may be regulated, for example, by enhancer or silencer sequences, splicing signals as well as other sequences which affect splicing processes, binding of transcription factors, polyadenylation sequences, transport signals, transcription terminator and the like. It is also envisaged that nucleic acid sequences physically unrelated to the coagulation factor XII gene locus can participate in the regulation of the expression of coagulation factor XII, and thus may have an impact on the development of angioedema symptoms. For example, a gene locus on the short arm of chromosome 10, around marker D10S1653, envisaged to be located within the nucleotide sequence comprising nucleotides chr10:10,554,416 to chr10:18,725,506 (UCSC Genome Browser/July 2003) has been demonstrated to affect coagulation factor XII plasma level (Soria et al. 2002, Am. J. Hum. Genet. 70:567-574) and may, thus, also affect disease susceptibility or disease development.

Sequences "encoding coagulation factor XII" refer to the coding sequence of the coagulation factor XII gene. Said term relates to the genomic coding sequence as well as the coding sequence in a RNA or cDNA molecule.

The term "coagulation factor XII" relates to coagulation factor XII, which is a serine protease circulating in plasma as a single-chain inactive zymogen of approximately 80kDa. Particularly preferred in accordance with the present invention is the coagulation factor XII corresponding to the mRNA sequence given under GenBank accession no. NM_000505.2 and encoded by the nucleic acid molecule deposited under GenBank accession number AF538691 which is considered by the present invention as the wild-type coagulation factor XII gene sequence and which includes 5' promoter sequences (up to 1581 bp upstream from exon 1), coding and non-coding exon sequences, intronic sequences, and 3' flanking regulatory sequences, including 1598 bp downstream from the end of exon 14 which corresponds to the end of the coagulation factor XII mRNA as given under GenBank accession number NM_000505.2. With respect to genomic sequences further extending into upstream and downstream direction, the sequence considered here to represent the wild-type sequence may be taken from the July 2003 human reference sequence of the UCSC Genome Browser, v.53, namely from the reverse complement sequence of chr5:176,807,093 - 176,821,530 (representing 4000 bp upstream of exon 1 and 3000 bp downstream of exon 14). The GenBank entry AF538691 relates to the gene of Homo sapiens coagulation factor XII (Hageman factor) (F12) of which several variants are known in the art (vide supra). The term "coagulation factor XII" also relates to sequences with an identity of at least 70%, 75%, 80%, 85%, 90%, 95%, 96%, 97%, 98%, or 99% when compared with the sequence of GenBank accession number AF538691. In addition, the present invention also relates to various protein isoforms corresponding to different transcripts produced by alternative splicing (for example, those shown in "http://www.ncbi.nih.gov/IEB/Research/Acembly/av.cgi?db=human&l=F12"). Further, the present invention also relates to species homologues in other animals, preferably mammals including rat, mouse, guinea pig, pig, cattle or rabbit. Polymorphic variants of coagulation factor XII may also comprise variants with large deletions in, for example, intron regions. Said variants may nevertheless encode a coagulation factor XII (poly)peptide of wild-type sequence. It is important to note that when aligned to the sequence of AF538691, the calculated sequence identity may be considerably lower than expected for normal polymorphic variation. Thus, preferred in accordance with the present invention are biologically active variants and also fragments of coagulation factor XII encoded by a nucleic acid molecule with a sequence identity of at least 70%, 75%, 80%, 85%, 90%, 95%, 96%, 97%, 98%, or 99% when compared with the sequence of databank accession number AF538691. Sequence identity may be determined by using the Bestfit® program (Wisconsin Sequence Analysis Package, Version 8 for Unix, Genetics Computer Group, University Research Park, 575 Science Drive, Madison, WI 53711). Bestfit® uses the local homology algorithm of Smith and Waterman to find the best segment of homology between two sequences (*Advances in Applied Mathematics* 2:482-489 (1981)). When using Bestfit® or any other sequence alignment program to determine whether a particular sequence is, for instance, 95% identical to a reference sequence, the parameters are set, of course, such that the percentage of identity is calculated over the full length of the reference nucleotide sequence and that gaps in homology of up to 5% of the total number of nucleotides in the reference sequence are allowed. The identity between a first sequence and a second sequence, also referred to as a global sequence alignment, is determined using the FASTDB computer program based on the algorithm of Brutlag and colleagues (Comp. *App. Biosci.* 6:237-245 (1990)). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identity are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the length of the subject nucleotide sequence, whichever is shorter.

The present invention is related to the observation that patients affected by hereditary angioedema type III show no deficiency of C1 esterase inhibitor. According to the present invention, the symptoms observed in patients affected by hereditary angioedema type III can be associated with a mutation in a nucleic acid molecule regulating the expression of or encoding coagulation factor XII.

Such mutations may comprise, but are not limited to, for example (1) a mutation that favours, directly or indirectly, the production of a vasoactive kinin, (2) a mutation that alters the interaction of coagulation factor XII with activating surfaces or with a cell surface receptor or a cell surface receptor complex or with another physiologically interacting molecule, (3) a mutation that results in an increased stability of coagulation factor XII and/or an increased stability of its mRNA, (4) a mutation that results in an increased activity of coagulation factor XII, (5) a mutation that results in an alteration of substrate specificity of coagulation factor XII, (6) a mutation that results in an aberrant proteolytic processing of coagulation factor XII or (7) a mutation that results in an irregular interaction with C1 esterase inhibitor.

Further, without being bound by any theory, it is believed in accordance with the invention, that certain mutations or variations within certain regions of the coagulation factor XII gene may be mutations that affect the splicing, the expression, the structure and/or function of the GPRK6 (G protein-coupled receptor kinase 6) gene or a GPRK6 protein, respectively. GPRK6 has a direct functional relationship for example with the β2-adrenergic receptor, the vasoactive intestinal polypeptide type-1 (VPAC1) receptor, and the calcitonin gene-related peptide (CGRP) receptor (Shetzline et al. 2002, J. Biol. Chem. 277: 25519-25526; Aiyar et al. 2000, Eur. J. Pharmacol. 403: 1-7), thus possibly also being involved in the regulation of mechanisms underlying angioedema pathogenesis. The GPRK6 gene is located ~15 kb telomeric from the coagulation factor XII gene, being encoded on the opposite strand. There appear to exist certain splice variants/isoforms of GPRK6 (c.f. AceView and UCSC Genome Browser; GenBank acc nos. BX355118, BX463737, B1604127 [isoform h]) that arise from or are related to genomic sequences within the coagulation factor XII gene or its extended promoter region.

As stated above, factor XII (i.e. coagulation factor XII) is preferably a serine protease produced by the liver, circulating in human plasma as a single-chain inactive zymogen at a concentration of approximately 30 µg/ml. From expression data one has to assume a coagulation factor XII production also by other tissues, possibly as isoforms. Coagulation factor XII has a molecular weight of about 80 kDa on SDS gel electrophoresis and was originally cloned and sequenced by Cool et al. 1985 (J. Biol. Chem. 260: 13666-13676) and by Que & Davie 1986 (Biochemistry 25: 1525-1528). The human coagulation factor XII gene is located on chromosome 5, at 5q35.3 (Royle et al. 1988, Somat. Cell Mol. Genet. 14: 217-221), it is approximately 12 kb in size and consists of 14 exons and 13 introns (Cool & MacGillivray 1987, J. Biol. Chem. 262: 13662-13673). The mature plasma protein consists of 596 amino acids (following a leader peptide of 19 residues) and is organized in several domains. From N-terminus to C-terminus, these domains are: a fibronectin type-II domain, an epidermal growth factor-like domain, a fibronectin type-I domain, another epidermal growth factor-like domain, a kringle domain, a proline-rich region, and a serine-protease catalytic region.

*In vitro* activation of factor XII occurs on negatively charged surfaces (including glass, kaolin, Celite, dextran sulfate, and ellagic acid), by autoactivation, by proteolytic cleavage, by conformational change, or by some combination of these mechanisms (Pixley & Colman 1993, Methods Enzymol. 222: 51-65). Further activating substances include sulfatides, chondroitin sulfate, endotoxin, some mast cell proteoglycans, and also aggregated Aβ protein of Alzheimer's disease. In vivo, the subendothelial vascular basement membrane and/or the stimulated endothelial cell surface might be important for factor XII activation (Pixley & Colman 1993). On endothelial cell membranes, urokinase plasminogen activator receptor, gC1qR (the receptor that binds to the globular heads of complement C1q), and cytokeratin 1 might be involved in the interaction with factor XII (Joseph K. et al. 1996, Proc. Natl. Acad. Sci. USA 93: 8552-8557; Joseph K. et al. 2001, Thromb. Haemost. 85: 119-124; Mahdi et al. 2002, Blood 99 : 3585-3596).

Primary activation of factor XII is due to cleavage of the molecule at a critical Arg₃₅₃-Val₃₅₄ bond contained within a disulfide bridge, mediated for example by kallikrein or plasmin (or factor XIIa itself). The resultant factor XIIa (α-coagulation factor XIIa) is thus a two-chain, disulfide-linked 80-kDa enzyme consisting of a heavy chain (353 residues; 50 kDa) and a light chain (243 residues; 28 kDa). The heavy chain binds to negatively charged surfaces, the light chain represents the serine protease part of the molecule containing the canonical Asp₄₄₂, His₃₉₃, Ser₅₄₄ triad. Two subsequent cleavages are responsible for the formation of the two forms of factor XIIf (Kaplan et al. 2002, J. Allergy Clin. Immunol. 109: 195-209): these cleavages occur at Arg334-Asn335 and Arg343-Leu344 and result in the formation of "factor XII fragment", FXIIf, also called β-FXIIa. FXIIf consists of the light chain of factor XIIa, corresponding to the serine protease domain, and a very small piece, either 19 or 9 amino acids in length, of the original heavy chain. Factor XIIf lacks the binding site for the activating surface as well as the ability of factor XIIa to convert factor XI to factor XIa. However, FXIIf is still a potent activator of prekallikrein. In summary, activation of the factor XII zymogen results in an enzyme with decreasing size, a decrease in surface-binding properties, and a decrease in coagulant activity, but retained, eventually increased kinin-forming capacity (Colman & Schmaier 1997, Blood 90: 3819-3843).

The present invention's disclosure allows to specifically identify individuals with (a) mutation(s) in a nucleic acid molecule encoding coagulation factor XII or regulating the expression of coagulation factor XII and link this/these mutation(s) with the individual's hereditary angioedema type III or its predisposition to develop HAE type III or to pass on to their offspring (a) specific mutation(s) which is/are associated with an increased risk for the development of HAE type III. Said nucleic acid molecule may be DNA or RNA.

Any method including those known to the person skilled in the art may be used to determine the presence or absence of such a mutation.

In a preferred embodiment of the present invention's method of diagnosing, said determination comprises hybridizing under stringent conditions to said nucleic acid molecule at least one pair of nucleic acid probes, the first probe of said pair being complementary to the wild-type sequence of said nucleic acid molecule and the second probe of said pair being complementary to the mutant sequence of said nucleic acid molecule, wherein a perfect match, the presence of stable hybridization, between (i) the first hybridization probe and the target nucleic acid molecule indicates the presence of a wild-type sequence, and (ii) the second hybridization probe and the target nucleic acid molecule, indicates the presence of a mutant sequence, wherein the first hybridization probe and the second hybridization probe allow a differential detection. Preferably, said mutant sequence is a disease-associated mutant sequence.

The term "hybridizing under stringent conditions", as used in the description of the present invention, is well known to the skilled artesian and corresponds to conditions of high stringency or selectivity. Appropriate stringent hybridization conditions for each sequence may be established by a person skilled in the art on well-known parameters such as temperature, composition of the nucleic acid molecules, salt conditions etc.; see, for example, Sambrook et al., "Molecular Cloning, A Laboratory Manual"; ISBN: 0879695765, CSH Press, Cold Spring Harbor, 2001, or Higgins and Hames (eds.), "Nucleic acid hybridization, a practical approach", IRL Press, Oxford 1985, see in particular the chapter "Hybridization Strategy" by Britten & Davidson, 3 to 15. Stringent hybridization conditions are, for example, conditions comprising overnight incubation at 42° C in a solution comprising: 50% formamide, 5x SSC (750 mM NaCl, 75 mM trisodium citrate), 50 mM sodium phosphate (pH 7.6), 5x Denhardt's solution, 10% dextran sulfate, and 20 micrograms/ml denatured, sheared salmon sperm DNA, followed by washing the filters in 0.1x SSC at about 65°. Other stringent hybridization conditions are for example 0.2 x SSC (0.03 M NaCl, 0.003 M sodium citrate, pH 7) at 65°C.

Depending on the particular conditions, for example the base composition of the probe, the person skilled in the art may have to vary, for example the salt concentration and temperature in order to find conditions which (a) prevent the hybridization of probes differing from the target nucleic acid molecule in only one position and (b) still allow hybridization of probes which completely match the same region of the target nucleic acid molecule. However, said conditions can be established by standard procedures known to the person skilled in the art and by routine experimentation.

The probe of hybridization is usually a nucleic acid molecule containing one or more labels. The label can be located at the 5' and/or 3' end of the nucleic acid molecule or be located at an internal position. Preferred labels include, but are not limited to, fluorochromes, e.g. carboxyfluorescein (FAM) and 6-carboxy-X-rhodamine (ROX), fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein (JOE), 6-carboxy-2',4',7',4,7-hexachlorofluorescein (HEX), 5-carboxyfluorescein (5-FAM) or N,N,N',N'-tetramethyl-6-carboxyrhodamine (TAMRA), radioactive labels, e.g. ³²P, ³⁵S, ³H; etc. The label may also be a two stage system, where the probe is conjugated to biotin, haptens, etc. having a high affinity binding partner, e.g. avidin, specific antibodies, etc., where the binding partner is conjugated to a detectable label.

As stated above, two probes used as a pair must allow a differential detection. This can be accomplished, for example, by labeling the probes with two different labels that can be differentiated in a detection process.

The hybridization probe is usually a nucleic acid molecule of about 20 to about 2000 bases in length. When used for hybridization reactions such as southern or northern blot reactions, the probe can be an oligonucleotide or primer which are typically in the range of about 15 to 50 bases in length or can be considerably longer and may range from about 50 bases to about 2000 bases. The term "oligonucleotide", when used in an amplification reaction, refers to a nucleic acid molecule of typically 15 to 50 bases in length with sufficient complementarity to allow specific hybridization to a nucleic acid sequence encoding or regulating the expression of coagulation factor XII. Preferably, an oligonucleotide used for hybridization or amplification is about 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 bases in length. However, probes of about 100, 150, 200, 250, 300, 350, 400, 450, 500, 550, 600, 650, 700, 750, 800, 850, 900, 950 or 1000 bases are also contemplated by the present invention. Moreover, according to the particular conditions chosen for hybridization, the nucleotide probe may even be several hundred or thousand bases longer. Said probe or oligonucleotide may be composed of DNA or RNA. When used as a hybridization probe, it may be, e.g., desirable to use nucleic acid analogs, in order to improve the stability and binding affinity. The term "nucleic acid" shall be understood to encompass such analogs. A number of modifications have been described that alter the chemistry of the phosphodiester backbone, sugars or heterocyclic bases. Among useful changes in the backbone chemistry are phosphorothioates; phosphorodithioates, where both of the non-bridging oxygens are substituted with sulfur; phosphoroamidites; alkyl phosphotriesters and boranophosphates. Achiral phosphate derivatives include, but are not limited to, 3'-O'-5'-S-phosphorothioate, 3'-S-5'-O-phosphorothioate, 3'-CH2-5'-O-phosphonate and 3'-NH-5'-O-phosphoroamidate. Peptide nucleic acids replace the entire phosphodiester backbone with a peptide linkage. Sugar modifications are also used to enhance stability and affinity. The a-anomer of deoxyribose may be used, where the base is inverted with respect to the natural b-anomer. The 2'-OH of the ribose sugar may be altered to form 2'-O-methyl or 2'-O-allyl sugars, which provides resistance to degradation without comprising affinity. Modification of the heterocyclic bases must maintain proper base pairing. Some useful substitutions include deoxyuridine for deoxythymidine; 5-methyl-2'-deoxycytidine and 5-bromo-2'-deoxycytidine for deoxycytidine; 5-propynyl-2'-deoxyuridine and 5-propynyl-2'-deoxycytidine for deoxythymidine and deoxycytidine, respectively.

In another preferred embodiment of the present invention's method of diagnosing, said method comprises hybridizing under stringent conditions to said nucleic acid molecule a hybridization probe specific for a mutant sequence. Preferably, said mutant sequence is a disease-associated mutant sequence.

In another preferred embodiment of the present invention, the method of diagnosing comprises a step of nucleic acid amplification and/or nucleic acid sequencing. Preferably, nucleic acid sequencing is DNA sequencing. A widely used method of diagnosing is for example direct DNA sequencing of PCR products containing a mutation to be diagnosed. The term "amplification" or "amplify" means increase in copy number. The person skilled in the art know various methods to amplify nucleic acid molecules, these methods may also be used in the present invention's method of diagnosing. Amplification methods include, but are not limited to, "polymerase chain reaction" (PCR), "ligase chain reaction"(LCR, EPA320308), "cyclic probe reaction" (CPR), "strand displacement amplification" (SDA, Walker et al. 1992, Nucleic Acid Res. 7: 1691-1696), "transcription based amplification systems" (TAS, Kwoh et al. 1989, Proc. Nat. Acad. Sci. USA 86: 1173; Gingeras et al., PCT Application WO 88/10315). Preferably, amplification of DNA is accomplished by using polymerase chain reaction (PCR) [Methods in Molecular Biology, Vol. 226 (Bartlett J. M. S. & Stirling D., eds.): PCR protocols, 2^{nd} edition; PCR Technology: Principles and Applications for DNA Amplification (Erlich H. A., ed.), New York 1992; PCR Protocols: A guide to methods and applications (Innis M. A. et al., eds.), Academic Press, San Diego 1990]. Nucleic acid amplification methods may be particularly useful in cases when the sample contains only minute amounts of nucleic acid. If said nucleic acid is RNA, an RT-PCR might be performed. Subsequently, another amplification step involving PCR may be performed. Alternatively, if said nucleic acid contained in the sample is DNA, PCR may be performed.

The PCR, generally, consists of many repetitions of a cycle which consists of: (a) a denaturing step, which melts both strands of a DNA molecule; (b) an annealing step, which is aimed at allowing the primers to anneal specifically to the melted strands of the DNA molecule; and (c) an extension step, which elongates the annealed primers by using the information provided by the template strand. Generally, PCR can be performed for example in a 50 µl reaction mixture containing 5 µl of 10 x PCR buffer with 1.5 mM MgCl₂, 200 µM of each deoxynucleoside triphosphate, 0.5 µl of each primer (10 µM), about 10 to 100ng of template DNA and 1 to 2.5 units of Taq Polymerase. The primers for the amplification may be labeled or be unlabeled. DNA amplification can be performed, e.g., with a model 2400 thermal cycler (Applied Biosystems, Foster City, CA): 2 min at 94°C, followed by 35 cycles consisting of annealing (30 s at 50°C), extension (1 min at 72°C), denaturing (10 s at 94°C) and a final annealing step at 55°C for 1 min as well as a final extension step at 72°C for 5 min. However, the person skilled in the art knows how to optimize these conditions for the amplification of specific nucleic acid molecules or to scale down or increase the volume of the reaction mix.

A further method of nucleic acid amplification is the "reverse transcriptase polymerase chain reaction" (RT-PCR). This method is used when the nucleic acid to be amplified consists of RNA. The term "reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. Suitable polymerases for use with a DNA template include, for example, E. coli DNA polymerase I or its Klenow fragment, T.sub.4 DNA polymerase, Tth polymerase, and Taq polymerase, a heat-stable DNA polymerase isolated from Thermus aquaticus and developed and manufactured by Hoffmann-La Roche and commercially available from Perkin Elmer. The latter enzyme is widely used in the amplification and sequencing of nucleic acids. The reaction conditions for using Taq polymerase are known in the art and are described, e.g., in: PCR Technology, Erlich, H. A. 1989, Stockton Press, New York; or in: Innis, M. A., D. H. Gelfand, J. J. Sninsky, and T. J. White. 1990, PCR Protocols: A guide to methods and applications. Academic Press, New York. High-temperature RT provides greater primer specificity and improved efficiency. Copending U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT Reaction can be performed, for example, in a 20µl reaction mix containing: 4 µl of 5x ANV-RT buffer, 2 µl of Oligo dT (100 µg/ml), 2µl of 10 mM dNTPs, 1µl total RNA, 10 Units of AMV reverse transcriptase, and H₂O to 20µl final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample.

The term "primer" or "oligonucleotide" refers to a short nucleic acid molecule from about 8 to about 30, eventually to about 50 nucleotides in length, whether natural or synthetic, capable of acting as a point of initiation of nucleic acid synthesis under conditions in which synthesis of a primer extension product complementary to a template nucleic acid strand is induced, i.e., in the presence of four different nucleoside triphosphates or analogues thereof and an agent for polymerisation (i.e., DNA polymerase or reverse transcriptase) in an appropriate buffer and at a suitable temperature. Preferably, a primer is a single-stranded oligodeoxyribonucleotide. The appropriate length of a primer depends on the intended use of the primer but typically ranges for PCR primers and primers used in sequencing reactions from 10 to 25 nucleotides. Short primer molecules generally require cooler temperatures to form sufficiently stable hybrid complexes with the template. A primer need not reflect the exact sequence of the template but must be sufficiently complementary to hybridize specifically with a template, provided its ability to mediate amplification is not compromised. "Hybridize" refers to the binding of two single stranded nucleic acids via complementary base pairing, i.e. A to T (in RNA: U), G to C. The term "primer pair" refers to two primers that hybridize with the + and - strand, respectively, of a double stranded nucleic acid molecule, and allow the amplification of e.g. DNA fragments, as for example in a PCR reaction. A primer can be labeled, if desired, by incorporating a compound detectable by spectroscopic, photochemical, biochemical, immunochemical, or chemical means. For example, useful labels include, but are not limited to, fluorescent dyes, electron-dense reagents, biotin, or small peptides for which antisera or monoclonal antibodies are available. A label can also be used to "capture" the primer, so as to facilitate a selection of amplified nucleic acid or fragments thereof. Carboxyfluorescein (FAM) and 6-carboxy-X-rhodamine (ROX) are preferred labels. However, other preferred labels include fluorochromes, e.g. fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein (JOE), 6-carboxy-2',4',7',4,7-hexachlorofluorescein (HEX), 5-carboxyfluorescein (5-FAM) or N,N,N',N'-tetramethyl-6-carboxyrhodamine (TAMRA), radioactive labels, e.g. ³²P, ³⁵S, ³H; etc. The label may also be a two stage system, where the primer is conjugated to biotin, haptens, etc. having a high affinity binding partner, e.g. avidin, specific antibodies, etc., where the binding partner is conjugated to a detectable label. The label may be conjugated to one or both of the primers.

During said method for diagnosing, a step of nucleic acid sequencing may be performed. Any methods known in the art may be used for sequencing. Preferably, the nucleic acid sequence is determined by a method based on the sequencing techniques of Sanger or Maxam/Gilbert (see for example: Methods in Molecular Biology, Vol. 167 (Graham C. A. & Hill A. J. M., eds.): DNA sequencing protocols. 2^{nd} edition, 2001; Galas D. J. & McCormack S. J., Genomic Technologies: Present and Future. Caister Academic Press, Wymondham, UK, 2002).

In another preferred embodiment of the present invention's method of diagnosing, said method is or comprises an allele discrimination method selected from the group consisting of allele-specific hybridization, allele-specific primer extension including allele-specific PCR, allele-specific oligonucleotide ligation, allele-specific cleavage of a flap probe and/or allele-specific cleavage using a restriction endonuclease. These methods are known to the skilled person and described and further referenced for example by Kwok P-Y & Chen X 2003, Curr. Issues Mol. Biol. 5:43-60; Kwok P-Y 2001, Annu. Rev. Genomics Hum. Genet. 2:235-258; Syvänen, A.-Ch. 2001, Nature Rev. Genet. 2: 930-942.

In yet a further preferred embodiment, the present invention's method of diagnosing may comprise a detection method selected from the group consisting of fluorescence, time-resolved fluorescence, fluorescence resonance energy transfer (FRET), fluorescence polarization, colorimetric methods, mass spectrometry, (chemi)luminescence, electrophoretical detection and electrical detection methods. These methods for the detection of an allele discrimination reaction are known to the skilled person and described and further referenced for example by Kwok P-Y & Chen X 2003, Curr. Issues Mol. Biol. 5:43-60; Kwok P-Y 2001, Annu. Rev. Genomics Hum. Genet. 2:235-258; Syvänen, A.-Ch. 2001, Nature Rev. Genet. 2: 930-942.

In certain cases it might be necessary to detect large deletions, insertions, or duplications. Preferably, this may be done by using methods well known in the art and comprising, for example, Southern blotting methods; quantitative or semiquantitative gene dosage methods including competitive PCR, differential PCR, real-time PCR, multiplex amplifiable probe hybridization; or long-range PCR (Armour et al. 2002, Human Mutation 20: 325-337).

It may often be desirable to obtain, from a single individual, an allelic diagnosis at several regions or positions of the nucleic acid molecule(s) encoding coagulation factor XII or regulating its expression. For this purpose, nucleic acid arrays may be useful, such as those described in: W0 95/11995.

Further, for some purposes it may be desirable to determine the presence of two or more mutations/variations as a haplotype, i.e. to determine which alleles from several mutant/variant positions occur together on one haplotype. This can be achieved by methods known in the art, for example by a segregation analysis within families, and also and preferably by methods allowing molecular haplotyping. For example, a double digest of a single PCR product, containing two mutant/variant positions, with two restriction endonucleases, each one of these two enzymes being able to differentiate the allelic situation at one of the two investigated positions, can yield such haplotype information from the fragment sizes obtained. However, numerous other methods are known to the person skilled in the art (see, for example: Tost et al. 2002, Nucleic Acids Res. 30: e96; Eitan & Kashi 2002, Nucleic Acids Res. 30: e62; Pettersson et al. 2003, Genomics 82: 390-396; Ding et al. 2003, Proc. Natl. Acad. Sci. U.S.A. 100: 7449-7453; Odeberg et al. 2002, Biotechniques 33: 1104,1106,1108; McDonald et al. 2002, Pharmacogenetics 12: 93-99; Woolley et al. 2000, Nature Biotechnol. 18: 760-763), and are envisaged to be applicable for the purposes of the present invention.

In yet another preferred embodiment of the present invention's method of diagnosing, the probe or the subject's nucleic acid molecule is attached to a solid support. Solid supports that may be employed in accordance with the invention include filter material, chips, wafers, microtiter plates, to name a few.

The present invention also relates to a method of diagnosing hereditary angioedema type III (HAE III) or a predisposition thereto in a subject being suspected of having developed or of having a predisposition to develop a hereditary angioedema type III or in a subject being suspected of being a carrier for hereditary angioedema type III, the method comprising assessing the presence, amount and/or activity of coagulation factor XII in said subject and including the steps of: (a) determining from a biological sample of said subject in vitro, the presence, amount and /or activity of: (i) a (poly)peptide encoded by the coagulation factor XII gene; (ii) a substrate of the (poly)peptide of (i); or (iii) a (poly)peptide processed by the substrate mentioned in (ii); (b) comparing said presence, amount and/or activity with that determined from a reference sample; and (c) diagnosing, based on the difference between the samples compared in step (b), the pathological condition of a hereditary angioedema type III or a predisposition thereto. The term "(poly)peptide" refers alternatively to peptide or to (poly)peptides. Peptides conventionally are covalently linked amino acids of up to 30 residues, whereas polypeptides (also referred to herein as "proteins") comprise 31 and more amino acid residues. The term "assessing the amount" or "determining the amount" means assessing or determining the amount of a (poly)peptide encoded by the coagulation factor XII gene, comprising for example the coagulation factor XII precursor or any of its maturation products generated for example by activating processes including autoactivation and proteolytic processing of coagulation factor XII. Therefore, assessing or determining the amount of coagulation factor XII also may refer to determining the amount of (1) mature FXII, (2) FXIIa (80 kDa, arising from the cleavage at Arg353 - Val354); (3) FXIIf (2 subforms: 30 kDa/28.5 kDa; 19-peptide or nonapeptide linked via S-S to the catalytic chain; arising from the cleavage of Arg334 - Asn335 and the additional cleavage of Arg343 - Leu344); (4) a third form of activated factor XII, a 40kDa molecule (mainly produced by autoactivation), in which the serine protease domain is linked to a 12,000-MW fragment of the heavy chain (Kaplan & Silverberg 1987); (5) potential protein isoforms (AceView, http://www.ncbi.nlm.nih.gov/IEB/Research/Acembly/av.cgi?db=33&c=Gene&l=F12); (6) coagulation factor XII forms or fragments that arise from an irregular proteolytic processing, eventually caused by a mutation of the present invention; or (7) a mutant of any one of the forms (1) to (5), including any of the mutants of the present invention. However, "assessing the amount" or "determining the amount" also refers to determining the amount of substrates and/or their activation products of any of the above-mentioned coagulation factor XII forms. Preferably, the ratio of activated and native (non-activated) forms of these substrates is determined. Also included are (poly)peptides processed by these (activated) substrates. These substrates and processed (poly)peptides include, for example, (8) coagulation factor XIa/coagulation factor XI; (9) coagulation factor VIIa/coagulation factor VII; (10) kallikrein/prekallikrein; (11) plasmin/plasminogen; (12) activated complement C1r/C1r; (13) activated complement C1s/C1s; (14) activated hepatocyte growth factor (HGF) / hepatocyte growth factor; (15) activated macrophage stimulating protein (MSP) / macrophage stimulating protein. Also included is (16) the determination of "cleavage products of high-molecular weight kininogen" or the ratio of the "cleavage products of high-molecular weight kininogen" with "high-molecular weight kininogen". Said cleavage products comprise cleaved kininogen, bradykinin and/or other kinins. Furthermore included are (17) cleavage products of complement component C2 / complement component C2; (18) cleavage products of complement component C4 / complement component C4; and (19) activated bradykinin type 2 receptor / bradykinin type 2 receptor.

The term "assessing the activity" or "determining the activity" means determining a biological activity, wherein biological activity refers to (a) the known activities, preferably those of wild-type (poly)peptides, and (b) aberrant activities, including those of mutant coagulation factor XII (poly)peptides which are apparent from comparing the activity of a mutant with that of a wild-type (poly)peptide. The known and aberrant activities may comprise the activity of any of the proteins (1) to (19) mentioned above. The term "assessing the presence" or "determining the presence" means determining which of the aforementioned (poly)peptides or proteins is present in the sample. Said term also refers to determining whether wild-type or a mutant (poly)peptide is present in the sample. Preferably, said (poly)peptide is any of the (poly)peptides (1) to (7) as mentioned above. In some cases, it may also be useful to analyze any of the (poly)peptides (8) to (19) as mentioned above, their native and/or activated forms.

Step (i) of the method, which reads "a (poly)peptide encoded by the coagulation factor XII gene", may comprise the determination of at least one of the (poly)peptides listed above under (1), (2), (3), (4), (5), (6) and (7).

Step (ii) of the method, which reads "a substrate of the (poly)peptide of (i)", may comprise the determination of at least one of the polypeptides listed above under (8), (9), (10), (11), (12), (13), (14), (15) and (16).

Step (iii) of the method, which reads "a (poly)peptide processed by the substrate mentioned in (ii)", may comprise the determination of at least one of the polypeptides listed above under (16), (17), (18), and (19).

This method of diagnosing is based on determining from a sample of an individual to be diagnosed and a reference sample the quantity and/or quality of, for example, any of the proteins listed under (1) to (19) and determining, based on the difference between said samples, a pathological condition in said individual's sample. Said pathological condition is hereditary angioedema type III or a predisposition thereto. The reference sample is a standard sample obtained from a healthy subject or healthy subjects, preferably from a subject or subjects particularly not affected by angioedema symptoms.

Generally, any of the known protein detection methods may be used. These include, for example, immunochemical, antibody-based methods such as ELISA, RIA, Western Blotting, preferably following any kind of electrophoretic separation step, and the like. Such methods are, for example, described by Clark & Hales: Immunoassays. In: Clinical Aspects of Immunology (P. J. Lachmann et al., eds.), vol.2, 5^{th} ed., Boston 1993; or in Weir's Handbook of Experimental Immunology, 5^{th} ed., 1996 (Herzenberg L. et al., eds.); see also e.g. Lämmle et al. 1987 (Semin. Thromb. Hemost. 13: 106-114). Methods for the determination of biological activities of the polypeptides listed above are known in the art. Biological activity can be measured for example by providing substrates for the (poly)peptides and measuring substrate conversion by the methods known in the art. For example, measuring the activity of (pre)kallikrein on a chromogenic substrate, which may be monitored by detecting cleavage of said substrate, has been described by Kluft 1978 (J. Lab. Clin. Med, 91:83-95), Kluft 1988 (Meth. Enzymol. 163: 170-179). Functional assays for measuring prekallikrein have also been described by de la Cadena et al. 1987 (J. Lab. Clin. Med. 109: 601-607) and Silverberg & Kaplan 1988 (Meth. Enzymol. 163: 85-95). A functional assay for high molecular weight kininogen using a chromogenic substrate has been described by Scott et al. 1987 (Thromb. Res. 48: 685-700) and also by Gallimore et al. 2002 (Blood Coagul. Fibrinolysis 13: 561-568).

The present invention also employs methods for determining the amino acid sequence of a (poly)peptide. Such methods are known in the art (see for example: Methods in Molecular Biology, Vol. 211 (Smith B. J., ed.): Protein Sequencing Protocols. 2^{nd} edition, 2002). Preferably, protein sequence analysis is performed by Edman degradation (P. Edman, Acta Chem. Scand. 4: 283 (1950)) or by Matrix-assisted laser desorption/ionisation-time of flight mass spectrometry (MALDI-TOF MS). Hence, by using amino acid sequence analysis, the skilled person may determine whether a wild-type or mutant coagulation factor XII (poly)peptide is present in a sample.

The proteins listed above, include on the one hand coagulation factor XII and its various forms. These are part of a cascade known as, for example, the intrinsic coagulation pathway or contact system or kinin-forming pathway (see e.g. Kaplan et al. 1997, Adv. Immunol. 66: 225-272; Kaplan et al. 2002, J. Allergy Clin. Immunol. 109: 195-209). On the other hand, proteins listed above are proteins which follow coagulation factor XII downstream in said cascade, and, in addition, proteins which are not directly related to the kinin-forming pathway but for which it has been shown that they can be activated by coagulation factor XII, eventually indirectly. It is important to note that mutations of coagulation factor XII may have an impact on these downstream steps in the cascade and, for example, can result in a quantitatively or qualitatively abnormal activation of (poly)peptides located downstream in the cascade. This effect may be measured and may allow for deductions on the nature of the specific coagulation factor XII expressed in the individual under study.

The methods of the present invention are not limited to measuring individual (poly)peptides as listed above, but also refer to the measuring or determination of complexes of said (poly)peptides. Such complexes are for example complexes consisting of activated factor XII and complement C1 inhibitor; or complexes consisting of kallikrein and complement C1 inhibitor; or complexes consisting of kallikrein and alpha2-macroglobulin. Such complexes can be detected, for example, by using ELISA or RIA based techniques (Nuijens et al., 1987 Thromb. Hemost. 58: 778-785; Kaplan et al., 1985, Blood 66: 636-641; Kaplan et al., 1989, Clin. Immunol. Immunopathol. 50 : S41-S51; Dors et al. 1992, Thromb. Haemost. 67 : 644-648).

In a preferred embodiment of the present invention's method, the biological sample consists of or is taken from hair, skin, mucosal surfaces, body fluids, including blood, plasma, serum, urine, saliva, sputum, tears, liquor cerebrospinalis, semen, synovial fluid, amniotic fluid, milk, lymph, pulmonary sputum, bronchial secretion, or stool.

The term "biological sample" relates to the specimen taken from a mammal. Preferably, said specimen is taken from hair, skin, mucosal surfaces, body fluids, including blood, plasma, serum, urine, saliva, sputum, tears, liquor cerebrospinalis, semen, synovial fluid, amniotic fluid, milk, lymph, pulmonary sputum, bronchial secretion, or stool. However, it is important to note that many other samples might be useful for this purpose, for example a sample taken for histological or cytological purposes.

A variety of techniques for extracting nucleic acids from biological samples are known in the art. For example, see those described in Rotbart et al., 1989, in PCR Technology (Erlich ed., Stockton Press, New York) and Han et al. 1987, Biochemistry 26:1617-1625. If the sample is fairly readily disruptable, the nucleic acid need not be purified prior to amplification by the PCR technique, i.e., if the sample is comprised of cells, e.g. peripheral blood lymphocytes or monocytes, lysis and dispersion of the intracellular components may be accomplished merely by suspending the cells in hypotonic buffer. Suitable methods will vary depending on the type of specimen and are well known to the person skilled in the art (see e.g. Sambrook et al., "Molecular Cloning, A Laboratory Manual"; ISBN: 0879695765, CSH Press, Cold Spring Harbor, 2001).

It is apparent that, for analysis of mRNA, cDNA, or protein, the sample must be obtained from a tissue in which coagulation factor XII/the coagulation factor XII gene is expressed, or, respectively, from a tissue or body fluid, in which coagulation factor XII is expressed or in which it is secreted.

In another preferred embodiment, said presence, amount and/or activity is determined by using an antibody or an aptamer, wherein the antibody or aptamer is specific for (a) a (poly)peptide encoded by the coagulation factor XII gene, (b) a substrate of the (poly)peptide of (a), or (c) a (poly)peptide processed by the substrate mentioned in (b). The term "antibody" refers to monoclonal antibodies, polyclonal antibodies, chimeric antibodies, single chain antibodies, or a fragment thereof. Preferably the antibody is specific for a polypeptide listed under (1) to (19). The antibodies may be bispecific antibodies, humanized antibodies, synthetic antibodies, antibody fragments, such as Fab, F(ab₂)', Fv or scFv fragments etc., or a chemically modified derivative of any of these, all comprised by the term "antibody". Monoclonal antibodies can be prepared, for example, by the techniques as originally described in Köhler and Milstein, Nature 256 (1975), 495, and Galfré, Meth. Enzymol. 73 (1981), 3, which comprise the fusion of mouse myeloma cells to spleen cells derived from immunized mammals with modifications developed by the art. Furthermore, antibodies or fragments thereof to the aforementioned (poly)peptides can be obtained by using methods which are described, e.g., in Harlow and Lane "Antibodies, A Laboratory Manual", CSH Press, Cold Spring Harbor, 1998. When derivatives of said antibodies are obtained by the phage display technique, surface plasmon resonance as employed in the BlAcore system can be used to increase the efficiency of phage antibodies Swhich bind to an epitope of the peptide or polypeptide to be analyzed (Schier, Human Antibodies Hybridomas 7 (1996), 97-105; Malmborg, J. Immunol. Methods 183 (1995), 7-13). The production of chimeric antibodies is described, for example, in WO89/09622.

Antibodies may be labelled. Preferably said label is selected from the group consisting of fluorochromes, e.g. fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein (JOE), 6-carboxy-X-rhodamine(ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA), radioactive labels, e.g. ³²P, ³⁵S, ³H; etc. The label may also be a two stage system, where the antibody is conjugated to biotin, haptens, etc. having a high affinity binding partner, e.g. avidin, specific antibodies, etc., where the binding partner is conjugated to a detectable label. In another preferred embodiment of the present invention the label is a toxin, radioisotope, or fluorescent label.

The term "aptamers" refers to RNA and also DNA molecules capable of binding target proteins with high specificity, comparable with the specificity of antibodies. Methods for obtaining or identifying aptamers specific for a desired target are known in the art. Preferably, these methods may be based on the "systematic evolution of ligands by exponential enrichment" (SELEX) process (Ellington and Szostak, Nature, 1990, 346: 818-822; Tuerk and Gold, 1990, Science 249: 505-510; Fitzwater & Polisky, 1996, Methods Enzymol. 267: 275-301). Preferably, said aptamers may be specific for any of the (poly)peptides listed under (1) to (19). The use of aptamers for detection and quantification of polypeptide targets is described in, for example, McCauley et al., 2003, Anal. Biochem., 319:244-250; Jayasena, 1999, Clin.Chem. 45:1628-1650.

In a more preferred embodiment, said antibody or aptamer is specific for a (poly)peptide encoded by the coagulation factor XII gene. Said reagents will allow for assessing the quantity and/or quality of (a) coagulation factor XII (poly)peptide(s), and eventually also for the differentiation between wild-type and mutant, preferably disease-associated mutant coagulation factor XII (poly)peptides. For example, the identification of coagulation factor XII (poly)peptides by an immunoblotting procedure following an electrophoretic separation step, might well allow for the recognition of a mutant coagulation factor XII (poly)peptide. However, regarding the preferred differentiation between wild-type and disease-associated mutant coagulation factor XII (poly)peptides, preferably, said antibody or aptamer is specific for a disease-associated mutant of the present invention. Such an antibody or aptamer would fail to bind to wild-type coagulation factor XII (poly)peptide(s) but bind to a disease-associated mutant with high specificity. This antibody or aptamer would therefore be most useful to discriminate between wild-type and mutant coagulation factor XII (poly)peptides. More preferably, the epitope or target region recognized by the antibody or aptamer comprises the mutant position/region in coagulation factor XII.

Various antibody-based methods for the determination of coagulation factor XII (poly)peptide(s), like radial immunodiffusion, electroimmunoassay according to Laurell, dot immunobinding assay, radioimmunoassay, enzyme immunoassay, enzyme-linked immunosorbent assay, immunoblotting, or alike, have been described or employed for example by Mannhalter et al. 1987 (Fibrinolysis 1: 259-263), Gevers Leuven et al. 1987 (J. Lab. Clin. Med.), Wuillemin et al. 1990 (J. Immunol. Methods 130: 133-140), Saito et al. 1976 (J. Lab. Clin. Med. 88: 506-514), Ford et al. 1996 (J. Immunoassay 17: 119-131), Lämmle et al. 1987 (Semin. Thromb. Hemost. 13: 106-114).

In a preferred embodiment of the present invention, the presence, amount and/or activity of the (poly)peptide(s) encoded by the coagulation factor XII gene is determined in (a) a coagulation assay; or in (b) a functional amidolytic assay; or in (c) a mitogenic assay; or in (d) a binding assay measuring binding of a (poly)peptide encoded by the coagulation factor XII gene to a binding partner.

Coagulant activity of coagulation factor XII may be quantified using methods in which correction of the abnormal clotting time, the prolonged activated partial thromboplastin time, of plasma of a person with a severe hereditary deficiency of coagulation factor XII is measured (see for example: Pixley R. A. & Colman R. W. 1993; Methods in Enzymology 222: 51-65). Functional amidolytic assays for coagulation factor XII using various synthetic chromogenic substrates (for example S2302, S2337, S2222) have been described for example by Vinazzer 1979 (Thrombosis Research 14: 155-166), Tans et al. 1987 (Eur. J. Biochem. 164: 637-642), Gallimore et al. 1987 (Fibrinolysis 1: 123-127), Walshe et al. 1987 (Thrombosis Research 47: 365-371), Kluft 1988 (Methods Enzymol. 163: 170-179), Stürzebecher et al. 1989 (Thrombosis Research 55: 709-715).
Another example for assessing a coagulation factor XII functional activity may be a measurement of the hepatocyte growth factor activating activity of coagulation factor XII (Shimomura et al. 1995, Eur. J. Biochem. 229: 257-261).

Schmeidler-Sapiro et al. 1991 (Proc. Natl. Acad. Sci. U.S.A. 88: 4382-4385) described assay systems allowing to assess a mitogenic activity of coagulation factor XII on HepG2 cells; coagulation factor XII as well as coagulation factor XIIa (kaolin-activated coagulation factor XII) enhanced cell proliferation and thymidine and leucine incorporation in HepG2 cells. Gordon et al. 1996 (Proc. Natl. Acad. Sci. U.S.A. 93: 2174-2179) assessed a growth factor activity of factor XII on several other target cells. Any of the aforementioned methods may be modified and used for determining the activity of (poly)peptides encoded by the coagulation factor XII gene. Various activators can be used in these assays, for example dextran sulfate, kaolin, a cephalin ellagic acid based reagent (Walshe et al. 1987, Thromb. Res. 47: 365-371), or others, and it is conceivable that the extent and/or the nature of activation achieved could be different for disease-associated mutant forms of coagulation factor XII when compared to wild-type coagulation factor XII (poly)peptide(s).

The term "binding partner" refers to a molecule capable of interacting with a (poly)peptide encoded by the coagulation factor XII gene. The binding activity of coagulation factor XII (poly)peptides may be determined by using a binding assay. The skilled person knows from in vitro studies that coagulation factor XII may bind for example to activating surfaces or substances, proteins or protein complexes. The prior art reported for example about the binding of coagulation factor XII to complexes of gC1q-R, cytokeratin 1 and urokinase plasminogen activator receptor present on the surface of endothelial cells (Joseph et al. 1996, Proc. Natl. Acad. Sci. USA 93: 8552-8557; Joseph et al. 2001, Thromb. Haemost. 85: 119-124; Mahdi et al. 2002, Blood 99 : 3585-3596). The binding partner can also be an antibody. Binding assays are described in detail in the prior art and may be used by the skilled person in order to determine whether a sample contains coagulation factor XII (poly)peptide(s) with normal or aberrant binding characteristics. This will allow deductions on the nature of the coagulation factor XII (poly)peptide(s) present in the sample under study.

The present invention also relates to a method of identifying a compound modulating coagulation factor XII activity which is suitable as a medicament or a lead compound for a medicament for the treatment and/or prevention of hereditary angioedema type III, the method comprising the steps of: (a) in vitro contacting a coagulation factor XII (poly)peptide or a functionally related (poly)peptide with the potential modulator; and (b) testing for modulation of coagulation factor XII activity, wherein modulation of coagulation factor XII activity is indicative of a compound's suitability as a medicament or a lead compound for a medicament for the treatment and/or prevention of hereditary angioedema type III.

The term "modulator" or "modulating compound" refers to a compound which alters the activity and/or the expression and/or the secretion of coagulation factor XII. This includes also the modulation of a "functionally related (poly)peptide", thus of (a) (poly)peptide(s) or the expression thereof being related to the function and/or expression and/or secretion of coagulation factor XII, preferably functionally related to coagulation factor XII upstream or downstream within the contact system/kinin pathway. In principle, a modulator can have an activating or an inhibiting effect. It is also envisaged that the modulator can differentially modulate only one or more of the various functions of coagulation factor XII. The modulator can be, for example, a 'small molecule', an aptamer, or an antibody (see below). In accordance with the present invention, the modulator is preferably a compound interacting with a coagulation factor XII (poly)peptide, and, more preferably, an inhibiting compound.

The term "contacting" means bringing in contact the targeted (poly)peptide, preferably a coagulation factor XII (poly)peptide with a potential modulator. Said coagulation factor XII (poly)peptide is preferably a polypeptide selected from any of the aforementioned (poly)peptides (1) to (7). By bringing in contact the (poly)peptide with a potential modulator of activity, the skilled person can test the impact of the modulator on the (poly)peptide's activity. Examples for assays for measuring various activities of coagulation factor XII (poly)peptides, including the binding to activating substances or other binding partners, have been described above and can be used for testing of potential modulators.

Coagulation factor XII (poly)peptide(s) used for contacting with a potential modulator may generate from various sources. For example, coagulation factor XII (poly)peptide(s) may be isolated from human plasma, either from healthy individuals or from patients affected by HAE type III; to this end, various methods known in the art may be used, for example those described by Pixley & Colman 1993 (Methods Enzymol. 222: 51-65). Alternatively, coagulation factor XII (poly)peptide(s) may also be produced synthetically. Further, coagulation factor XII (poly)peptide(s) may be recombinantly expressed. To this end, nucleic acid molecules encoding coagulation factor XII (poly)peptides may be introduced into a host cell. The term "introducing" refers to the process of transfecting or transforming a host cell with such a nucleic acid molecule. Introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals, such as Davis *et al., Basic Methods In Molecular Biology* (1986). Said nucleic acid molecule introduced into the host cell comprises an open reading frame encoding a coagulation factor XII (poly)peptide in expressable form. A typical mammalian expression vector contains the promoter element, which mediates the initiation of transcription of mRNA, the protein coding sequence, and signals required for the termination of transcription and polyadenylation of the transcript. Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109). Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells. Alternatively, the recombinant (poly)peptide can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al. 1991, *Biochem J. 227*:277-279; Bebbington et al. 1992, *Bio*/*Technology 10*:169-175). Using these markers, the mammalian cells are grown in selective medium and the cells with the highest resistance are selected. Chinese hamster ovary (CHO) and NSO cells are often used for the production of proteins. The expression vectors pC1 and pC4 contain the strong promoter (LTR) of the Rous Sarcoma Virus (Cullen *et al. 1985, Molecular and Cellular Biology 5:* 438-447) plus a fragment of the CMV-enhancer (*Boshart et al.1985, Cell 41*:521-530). Multiple cloning sites, e.g., with the restriction enzyme cleavage sites *Bam* HI, *Xba* I and *Asp* 718, facilitate the cloning of the gene of interest. The vectors contain in addition the 3' intron, the polyadenylation and termination signal of the rat preproinsulin gene. As indicated above, the expression vectors will preferably include at least one selectable marker. Such markers include dihydrofolate reductase, G418 or neomycin resistance for eukaryotic cell culture and tetracycline, kanamycin or ampicillin resistance genes for culturing in *E. coli* and other bacteria. Representative examples of appropriate hosts include, but are not limited to, bacterial cells, such as *E. coli*, Streptomyces and *Salmonella typhimurium* cells; fungal cells, such as yeast cells; insect cells such as Drosophila S2 and Spodoptera Sf9 cells; animal cells such as CHO, COS, 293 and Bowes melanoma cells; and plant cells. Appropriate culture mediums and conditions for the above-described host cells are known in the art.

The recombinantly expressed polypeptide may contain additional amino acid residues in order to increase the stability or to modify the targeting of the protein. For instance, a region of additional amino acids, particularly charged amino acids, may be added to the N-terminus of the polypeptide to improve stability and persistence in the host cell, during purification, or during subsequent handling and storage. Also, peptide moieties may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art. A preferred fusion protein comprises a heterologous region from immunoglobulin that is useful to stabilize and purify proteins. For example, EP-A- 0 464 533 (Canadian counterpart 2045869) discloses fusion proteins comprising various portions of constant region of immunoglobulin molecules together with another human protein or part thereof. In many cases, the Fc part in a fusion protein is thoroughly advantageous for use in therapy and diagnosis and thus results, for example, in improved pharmacokinetic properties (EP-A 0 232 262). On the other hand, for some uses it would be desirable to be able to delete the Fc part after the fusion protein has been expressed, detected and purified in the advantageous manner described. This is the case when the Fc portion proves to be a hindrance for example for the catalytic activity of a coagulation factor XII (poly)peptide. In drug discovery, for example, human proteins, such as hIL-5, have been fused with Fc portions for the purpose of high-throughput screening assays to identify antagonists of hIL-5. See, D. Bennett *et al., J. Molecular Recognition 8*:52-58 (1995) and K. Johanson *et al., J. Biol. Chem.* 270:9459-9471 (1995). Coagulation factor XII (poly)peptide(s) can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography and/or hydroxylapatite chromatography. Most preferably, high performance liquid chromatography ("HPLC") is employed for purification.

The step of contacting the recovered coagulation factor XII (poly)peptide with a potential modulator is essentially a step by which the efficacy of a potential modulator is tested. Generally, the coagulation factor XII (poly)peptide is present at conditions assumed to be physiological conditions or in a test solution representing such conditions. When examining, for example, enzymatic activity, the following may be of importance: after optimum substrate and enzyme concentrations are determined, a candidate modulator is added to the reaction mixture at a range of concentrations. The assay conditions ideally should resemble the conditions under which the modulator is to be active, i.e., under physiologic pH, temperature, ionic strength, etc. For example, when the modulator is an inhibitor of protease activity, suitable inhibitors will exhibit strong protease inhibition at concentrations which do not raise toxic side effects in the subject. Inhibitors which compete for binding to the protease's active site may require concentrations equal to or greater than the substrate concentration, while inhibitors capable of binding irreversibly to the protease's active site may be added in concentrations in the order of the enzyme concentration. Substrate conversion, i.e. proteolytic cleavage is conveniently measured by using labelled substrates such as labelled peptides representing the cleavage site of a natural substrate of coagulation factor XII.

One of the more popular protease detection methods is the use of fluorescence resonance energy transfer between a donor fluorophore at one end of the peptide chain, and a quencher at the other end of the peptide chain. These methods were reviewed by Knight "Fluorimetric assays of proteolytic enzymes," Methods in Enzymol. (1995) 248:18-34, the contents of which are incorporated herein by reference. Here, proteolytic cleavage of the peptide link connecting the fluorophore and quencher liberates the quencher to diffuse away from the fluorophore. This results in an increase in fluorescence. A variation on this quencher method is taught by U.S. Pat. Nos. 5,605,809 and 6,037,137. This variation brings a first fluorophore in close proximity to a second fluorophore via a folded peptide backbone. This technique has the advantage that the protease cleavage site need not be immediately adjacent to either of the fluorophores. However it has the disadvantage that to avoid disrupting the folded structure, the length of the protease cleavage site should ideally fall between 2-15 amino acid residues in length. Another very popular method is the use of peptide-quenched fluorescent moieties, such as the 7-amino-4-methylcoumarin (AMC) fluorophore, the 7-amino-4-carbamoylmethylcoumarin fluorophore (Harris, et. al. PNAS 97: 7754-7759 (2000)), or the peptide quenched Rhodamine 110 fluorophore (Mangel et. al., U.S. Pat. No. 4,557,862). Here the intrinsic fluorescence of a fluorophore is quenched by one or more covalently linked peptides, and the fluorescence is restored upon cleavage of the peptide. Although the Rhodamine 110 molecule operates with high efficiency, uses visible light for excitation and emission, and is otherwise an excellent label for fluorescence based protease assays, it has a few drawbacks that limit its use. The Rhodamine 110 molecule is divalent and normally incorporates two peptides of identical sequence, with both "N" terminal peptide groups exposed. This has the drawback that peptides with this polarity can not be incorporated into the interior of a larger peptide chain. Thus this label has primarily been used for protease substrate assays where the Rhodamine 110 molecule effectively represents the final "C" terminal group on the substrate. Variations on Rhodamine 110 molecule methods, suitable for caspase assays, are taught by U.S. Pat. No. 6,248,904.

The test for protease activity of coagulation factor XII (poly)peptides may be performed in solution or with the coagulation factor XII (poly)peptide or the substrate or the modulator arrayed on a solid support, e.g. a microtiter plate. Microarray methods have become widely used for pharmaceutical and biochemical research, and a large number of microarrays are commercially available. Use of peptide microarrays, constructed by photochemical methods, for antibody recognition of peptide patterns was taught by Fodor et. al. 1991, Science 251: 767-773. Use of peptide microarrays for protein kinase or protein-protein binding was taught by MacBeath and Schreiber 2000, Science 289: 1760-1763. Here glass slides were chemically activated to covalently bind peptides, and various peptides were spotted onto the slides using conventional spotting equipment. The peptides formed a covalent bond with the derivatized glass. Alternative methods to attach peptides to solid supports are taught by U.S. Pat. No. 6,150,153, which teaches the use of polyethyleneimine layers to facilitate peptide linkages. U.S. Pat. No. 4,762,881 teaches the use of incorporating an artificial benzoylphenylalanine into a peptide and allowing the peptide to attach to a solid substrate having an active hydrogen (such as polystyrene) using ultraviolet light. U.S. Pat. No. 4,681,870 teaches methods for derivatizing silica surfaces to introduce amino or carboxyl groups, and then coupling proteins to these groups. U.S. Pat. Nos. 5,527,681 and 5,679,773 teach methods for immobilized polymer synthesis and display suitable for microarrays, and various fluorescent-labeling methods to detect proteolytic cleavage.

For protease substrate microarrays, the peptides on the microarray will further contain detection moieties (fluorescent tags, fluorescent quenchers, etc.) to generate a detectable signal corresponding to the level of proteolytic cleavage of the particular peptide zone in question. The peptides are bound to the surface of the solid support (either covalently or non-covalently) to the extent sufficient to prevent diffusion of the bound peptides upon application of liquid sample, and subsequent digestion and processing steps. In use, the completed microarray is exposed to a liquid sample, which contains a coagulation factor XII (poly)peptide under study. The sample will typically be covered with an optional cover to help distribute the sample evenly over the array, and to prevent evaporation. Typically the cover will be of a transparent flat material, such as a glass or plastic cover slip, to enable observation of the peptide zones during the course of the digestion reaction. During the protease digestion reaction, peptides with differential sequences or different modifications will typically be digested to a differential amount. The detectable signal generated by the detection moieties attached to each peptide region will be interrogated, typically at multiple time points during the digestion reaction. This conveys information as to the relative proteolytic activity of the studied coagulation factor XII (poly)peptide in the presence of a potential protease modulator or inhibitor, thus providing information on the suitability of the modulator for modulating, eventually inhibiting coagulation factor XII activity. Optionally, at the end of the reaction, a non-specific protease or a non-specific labeled moiety reacting agent may be added to the microarray to serve as a positive or negative control.

In a preferred embodiment of the present invention's method of identifying a modulator compound, the coagulation factor XII (poly)peptide of step (a) is present in cell culture or cell culture supernatant or in a subject's sample or purified from any of these sources. The cell culture could be for example a cell culture in which a coagulation factor XII (poly)peptide is recombinantly expressed or a culture of cells, for example hepatocytes, and preferably of human origin, that naturally express coagulation factor XII. The subject's sample could be for example blood plasma, and the subject could be either affected or non-affected by hereditary angioedema type III.

In another preferred embodiment of the present invention's method of identifying a modulator compound, said testing is performed by assessing the physical interaction between a coagulation factor XII (poly)peptide and the modulator and/or the effect of the modulator on the function of said coagulation factor XII (poly)peptide.

The person skilled in the art knows of various methods for detecting the interaction between a protein and a potential binding partner or modulator. One such method, for example, may be based on the testing of potential binding partners which are spotted onto a solid support. If bound to a solid support, incubation of said potential binding partners with a solution containing, for example, coagulation factor XII (poly)peptide might identify positions on the solid support, occupied with candidate binding partners. Binding of, for example coagulation factor XII (poly)peptide(s) to said binding partner may be detected by various methods known in the art. For example, binding of coagulation factor XII to a binding partner could be visualized by incubating the solid support with a labeled antibody specific for coagulation factor XII. Preferred methods comprise biacore based detection methods, ELISA based methods.

It is also envisaged here, that the (poly)peptide targeted by the potential modulator can be - instead of a coagulation factor XII (poly)peptide - a (poly)peptide functionally related, upstream or downstream within the contact system, with coagulation factor XII, i.e. interacting with coagulation factor XII. Nevertheless, as further envisaged here, this may cause a modulation of coagulation factor XII activity.

A modulator may be based on known compounds which may also be modified in order to adapt the compound to the requirements of the specific (poly)peptide to be targeted. The modulator can be, for example, a small molecule, an aptamer, or an antibody (vide infra).

Preferably, the modulator is a small molecule or small molecular compound and may be selected by screening a library of small molecules ("small molecule library"). The term "small molecule" or "small molecular compound" refers to a compound having a relative molecular weight of not more than 1000 D and preferably of not more than 500 D. It can be of organic or anorganic nature. A large number of small molecule libraries, which are commercially available, are known in the art. Thus, for example, a modulator may be any of the compounds contained in such a library or a modified compound derived from a compound contained in such a library. Preferably, such a modulator binds to the targeted (poly)peptide encoded by the coagulation factor XII gene with sufficient specificity, wherein sufficient specificity means preferably a dissociation constant (Kd) of less than 500nM, more preferable less than 200nM, still more preferable less than 50nM, even more preferable less than 10nM and most preferable less than 1nM.

It is also envisaged to design small molecular compounds using so called molecular modeling methods. Small molecular compounds can be for example peptide derived. Preferred are compounds which mimic the transition state of substrates of coagulation factor XII. Suitable compounds may be, for example, peptide-derived substrates which do not contain a cleavable peptide bond. Preferably, such compounds contain a cleavage site of a natural substrate of coagulation factor XII, wherein the peptide bond between P1 and P1' is replaced by a non-cleavable bond.

The peptide-based compounds and others, like compounds based on heterocyclic structures, may be for example known inhibitors of serine proteases or new compounds or compounds derived from preexisting inhibitors by derivatization. Preferably, such compounds are designed by computer modeling, wherein computer modeling means using virtual-screening tools for the search of compounds that bind, for example, to the substrate binding site of coagulation factor XII by using homology-modeling tools. Generally, these methods rely on the three-dimensional structure of proteins, preferably of proteins crystallized together with a substrate. More preferably, the substrate is replaced with a candidate modulator or inhibitor.

The design of molecules with particular structural relationships to part of a protein molecule like coagulation factor XII is well established and described in the literature (see for example Cochran 2000, Chem. Biol. 7, 85-94; Grzybowski et al. 2002, Acc. Chem. Res. 35, 261-269; Velasquez-Campoy et al. 2001, Arch. Biochem. Biophys. 380, 169-175; D'Aquino et al. 2000, Proteins: Struc. Func. Genet. Suppl. 4, 93-107.). Any of these so-called "molecular modeling" methods for rational drug design can be used to find a modulator of coagulation factor XII. Most of these molecular modeling methods take into consideration the shape, charge distribution and the distribution of hydrophobic groups, ionic groups and hydrogen bonds in the site of interest of the protein molecule. Using this information, that can be derived e.g. from the crystal structure of proteins and protein-substrate complexes, these methods either suggest improvements to existing proposed molecules, construct new molecules on their own that are expected to have good binding affinity, screen through virtual compound libraries for such molecules, or otherwise support the interactive design of new drug compounds *in silico*. Programs such as GOLD (G. Jones, et al., Development and J. Mol. Biol., 267, 727-748 (1997)); FLEXX (B. Kramer et al., Structure, Functions, and Genetics, Vol. 37, pp. 228-241, 1999); FLEXE (M. Rarey et al., JMB, 261,470-489 (1996)) DOCK (Kuntz, I.D. Science 257: 1078-1082, 1992); AUTODOCK (Morris et al., (1998), J. Computational Chemistry, 19: 1639-1662) are virtual screening programs designed to calculate the binding position and conformation as well as the corresponding binding energy of an organic compound to a protein. These programs are specially trimmed to allow a great number of "dockings", that is calculations of the conformation with the highest binding energy of a compound to a binding site, per time unit. Their binding energy is not always a real value, but can be statistically related to a real binding energy through a validation procedure. These methods lead to molecules, termed here "hits" that have to be evaluated by experimental biochemical, structural-biological, molecular-biological or physiological methods for their expected biological activity. The term "molecular modeling" or "molecular modeling techniques" refers to techniques that generate one or more 3D models of a ligand binding site or other structural feature of a macromolecule. Molecular modeling techniques can be performed manually, with the aid of a computer, or with a combination of these. Molecular modeling techniques can be applied for example to the atomic coordinates to derive a range of 3D models and to investigate the structure of ligand binding sites. A variety of molecular modeling methods are available to the skilled person for use according to the invention (G.Klebe and H.Gohlke, Angew.Chem.Int.Ed.2002, 41, 2644 - 2676; Jun Zeng: Combinatorial Chemistry & High Throughput Screening, 2000, 3, 355-362; Andrea G Cochran, Current Opinion in Chemical Biology 2001, 5:654-659).

In a preferred embodiment, the modulator is an inhibitor of coagulation factor XII activity, selected from the group consisting of: (a) an aptamer or inhibitory antibody or fragment or derivative thereof, specifically binding to a coagulation factor XII (poly)peptide and/or specifically inhibiting a coagulation factor XII activity; (b) a small molecule inhibitor of coagulation factor XII and/or coagulation factor XII activity; and (c) a serine protease inhibitor selected from group (I) consisting of wild-type and modified or engineered proteinaceous inhibitors of serine proteases including C1 esterase inhibitor, antithrombin III, α2-antiplasmin, α1-antitrypsin, ovalbumin serpins, and α2-macroglobulin, or selected from group (II) of Kunitz-type inhibitors including bovine pancreatic trypsin inhibitor.

The inhibitor can be an aptamer, preferably an aptamer specifically binding to coagulation factor XII. The term "aptamer" refers to RNA and also DNA molecules capable of binding target proteins with high affinity and specificity, comparable with the affinity and specificity of monoclonal antibodies. Methods for obtaining or identifying aptamers specific for a desired target are known in the art. Preferably, these methods may be based on the "systematic evolution of ligands by exponential enrichment" (SELEX) process (Ellington and Szostak, Nature, 1990, 346: 818-822; Tuerk and Gold, 1990, Science 249: 505-510; Fitzwater & Polisky, 1996, Methods Enzymol. 267: 275-301). Various chemical modifications, for example the use of 2'-fluoropyrimidines in the starting library and the attachment of a polyethylene glycol to the 5' end of an aptamer can be used to ensure stability and to enhance bioavailability of aptamers (see e.g. Toulme 2000, Current Opinion in Molecular Therapeutics 2: 318-324).

The inhibitor can also be an antibody or fragment or derivative thereof. As used herein, the term "antibody or fragment or derivative thereof" relates to a polyclonal antibody, monoclonal antibody, chimeric antibody, single chain antibody, single chain Fv antibody, human antibody, humanized antibody or Fab fragment specifically binding to coagulation factor XII and/or to a mutant of coagulation factor XII.

The antibodies described herein may be prepared by any of a variety of methods known in the art. For example, polyclonal antibodies may be induced by administration of purified protein, a coagulation factor XII (poly)peptide or an antigenic fragment thereof, to a host animal.

As pointed out above, the antibody may also be a monoclonal antibody. Such monoclonal antibodies can be prepared using hybridoma technology (Köhler et al., Nature 256:495 (1975); Köhler et al., Eur. J. Immunol. 6:511 (1976); Köhler et al., Eur. J. Immunol. 6:292 (1976); Hammerling et al., in: Monoclonal Antibodies and T-Cell Hybridomas, Elsevier, N.Y., 1981, pp. 563-681). In general, such procedures involve immunizing an animal (preferably a mouse) with a coagulation factor XII protein antigen. The splenocytes of such immunized mice are extracted and fused with a suitable myeloma cell line. Any suitable myeloma cell line may be employed in accordance with the present invention; however, it is preferable to employ the parent myeloma cell line (SP2/0), available from the American Type Culture Collection, Rockville, Maryland. After fusion, the resulting hybridoma cells are selectively maintained in HAT medium, and then cloned by limiting dilution as described by Wands et al. 1981 (Gastroenterology 80:225-232). The hybridoma cells obtained through such a selection are then assayed to identify clones which secrete antibodies capable of binding the coagulation factor XII protein antigen.

It will be appreciated that Fab and F(ab')₂ and other fragments of the antibodies of the present invention may be used according to the methods disclosed herein. Such fragments are typically produced by proteolytic cleavage, using enzymes such as papain (to produce Fab fragments) or pepsin (to produce F(ab')₂ fragments).

For *in vivo* use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. See, for review, Morrison, *Science* 229:1202 (1985); Oi et al., *BioTechniques 4*:214 (1986); Cabilly et al., U.S. Patent No. 4,816,567; Taniguchi et al., EP 171496; Morrison et al., EP 173494; Neuberger et al., WO 8601533; Robinson et al., WO 8702671; Boulianne *et al., Nature 312*:643 (1984); Neuberger *et al., Nature 314*:268 (1985).

Preferably, the antibodies specifically bind a coagulation factor XII (poly)peptide and include IgG (including IgG1, IgG2, IgG3, and IgG4), IgA (including IgA1 and IgA2), IgD, IgE, or IgM, and IgY. As used herein, the term "antibody" is meant to include whole antibodies, including single-chain whole antibodies, and antigen-binding fragments thereof. Most preferably the antibodies are human antigen binding antibody fragments and include, but are not limited to, Fab, Fab' and F(ab')₂, Fd, single-chain Fvs (scFv), single-chain antibodies, disulfide-linked Fvs (sdFv) and fragments comprising either a V_{L} or V_{H} domain. The antibodies may be from any animal origin including birds and mammals. Preferably, the antibodies are human, murine, rabbit, goat, guinea pig, camel, horse, or chicken.

"Specific binding" of antibodies may be described, for example, in terms of their cross-reactivity. Preferably, specific antibodies are antibodies that do not bind polypeptides with less than 98%, less than 95%, less than 90%, less than 85%, less than 80%, less than 75%, less than 70% and less than 65% identity (as calculated using methods known in the art) to a (poly)peptide encoded by the coagulation factor XII gene. Antibodies may, however, also be described or specified in terms of their binding affinity. Preferred binding affinities include those with a dissociation constant or Kd less than 5X10⁻⁶M, 10⁻⁶M, 5X10⁻⁷M, 10⁻⁷M, 5X10⁻⁸M, 10⁻⁸M, 5X10⁻⁹M, 10⁻⁹M, 5X10⁻¹⁰M, 10⁻¹⁰M, 5X10⁻¹¹M, 10⁻¹¹M, 5X10⁻¹²M, 10⁻¹²M, 5X10⁻¹³M, 10⁻¹³M, 5X10⁻¹⁴M, 10⁻¹⁴M, 5X10⁻¹⁵M, and 10⁻¹⁵M.

Further, the inhibitor can be a "small molecule" or "small molecular compound". As pointed out above, the term "small molecule" refers to a compound having a relative molecular weight of not more than 1000 D and preferably of not more than 500 D. Said compound may be of differing chemical nature, for example, it may be peptide-based or based on heterocyclic structures. Small molecule inhibitors of serine proteases have been extensively reviewed for example by Leung et al. 2000 (J. Med. Chem. 43: 305-341) and Walker & Lynas 2001 (Cell. Mol. Life Sci. 58: 596-624). Substances discussed by these authors include, for example, (i) peptide-based inhibitors, like phosphorus-based inhibitors (including α-aminoalkyl diphenylphosphonate esters and mixed phosphonate esters), fluorine-containing inhibitors (including for example trifluoromethyl ketones [as well as analogues containing the trifluoromethyl ketone moiety with lower peptidic characteristics], difluoromethyl ketone-based and pentafluoroethyl ketone-based inhibitors), inhibitors based on peptidyl boronic acids (including, for example, boroArg- or boroLys- or boro-methoxy-propylglycine- or boroPro-containing substances), inhibitors based on so-called 'inverse substrates' (including, for example, compounds containing a p-methoxybenzoic acid function), and peptide-based inhibitors with novel functional groups (including, for example, compounds with C-terminal electron-withdrawing groups based on α-keto heterocycles, like α-keto benzoxazoles or α-keto thiazoles); (ii) natural product-derived inhibitors, like cyclotheonamides (macrocyclic pentapeptides analogues), aeruginosins, and radiosumin; (iii) inhibitors based on heterocyclic and other nonpeptide scaffolds, like N-hydroxysuccinimide heterocycles and related compounds, compounds based on the isocoumarin scaffold, and β-lactam - based inhibitors (including, for example, cephalosporin-derived compounds and analogues of monocyclic and bicyclic β-lactams); and (iv) metal-potentiated compounds, like compounds based on bis(5-amidino-2-benzimidazolyl)methane (BABIM). All these (types of) substances, as well as derivatives thereof, are considered to be applicable for the purposes of the present invention.

Any of the known protease inhibitors may be useful for developing modulators or inhibitory modulators of coagulation factor XII activity, although inhibitors of serine proteases may be particularly useful. Any of the known compounds may be modified, for example in order to change their binding characteristics or their specificity.

With respect to natural or engineered proteinaceous inhibitors of serine proteases, selective changes or modifications of the natural inhibitory characteristics, of the natural specificity have been achieved, for example, with P2 mutants of C1 inhibitor (Zahedi et al. 2001, J. Immunol. 167: 1500-1506), a P1 mutant of α1-antitrypsin (Schapira et al. 1985, J. Clin. Invest. 76: 645-647), various P1-P2-P3 mutants of α1-antitrypsin (Sulikowski et al. 2002, Protein Science 11: 2230-2236), a P1-P2 mutant of α1-antitrypsin (Schapira et al. 1987, J. Clin. Invest. 80: 582-585), various P3-P4 mutants of bovine pancreatic trypsin inhibitor (Grzesiak et al. 2000, J. Biol. Chem. 275: 33346-33352), among them one P3 mutant with high specificity for factor XIIa.

Particularly with respect to (a) and (b), it is also envisaged that the "inhibitor of coagulation factor XII activity" could be a compound that does not primarily target a coagulation factor XII (poly)peptide, but still inhibits coagulation factor XII activity, for example by inhibiting the activation of coagulation factor XII due to interference with an activating protein. The present invention also relates to a method of identifying a compound modulating coagulation factor XII expression and/or secretion which is suitable as a medicament or lead compound for a medicament for the treatment and/or prevention of hereditary angioedema type III, the method comprising the steps of: (a) in vitro contacting a cell that expresses or is capable of expressing coagulation factor XII with a potential modulator of expression and/or secretion; and (b) testing for altered expression and/or secretion, wherein the modulator is (i) a small molecule compound, an aptamer or an antibody or fragment or derivative thereof, specifically modulating expression and/or secretion of coagulation factor XII; or (ii) a siRNA or shRNA, a ribozyme, or an antisense nucleic acid molecule specifically hybridizing to a nucleic acid molecule encoding coagulation factor XII or regulating the expression of coagulation factor XII. "Specific hybridization" means that the siRNA, shRNA, ribozyme or antisense nucleic acid molecule hybridizes to the targeted nucleic acid molecule, encoding coagulation factor XII or regulating its expression. Preferably, "specific hybridization" also means that no other genes or transcripts are affected.

A modulating compound will affect expression and/or secretion of coagulation factor XII. The skilled person knows a number of techniques for monitoring an effect on protein expression or secretion. For example, protein expression may be monitored by using techniques such as western blotting, immunofluorescence or immunoprecipitation. Alternatively, expression may also, for example, be monitored by analyzing the amount of RNA transcribed from a coagulation factor XII gene.

The term "contacting a cell" refers to the introduction of a potential modulator compound into a cell. As far as the compound is a nucleic acid molecule, the contacting may be performed by any of the known transfection techniques such as electroporation, calcium phosphate transfection, lipofection and the like. However, the nucleic acid may also be entered into the cell by virus based vector systems.

As used herein, the term "siRNA" means "short interfering RNA", the term "shRNA" refers to "short hairpin RNA". In RNA interference, small interfering RNAs (siRNA) bind the targeted mRNA in a sequence-specific manner, facilitating its degradation and thus preventing translation of the encoded protein. Transfection of cells with siRNAs can be achieved, for example, by using lipophilic agents (among them Oligofectamine™ and Transit-TKO™) and also by electroporation.

Methods for the stable expression of small interfering RNA or short hairpin RNA in mammalian, also in human cells are known to the person skilled in the art and are described, for example, by Paul et al. 2002 (Nature Biotechnology 20: 505-508), Brummelkamp et al. 2002 (Science 296: 550-553), Sui et al. 2002 (Proc. Natl. Acad. Sci. U.S.A. 99: 5515-5520), Yu et al. 2002 (Proc. Natl. Acad. Sci. U.S.A. 99: 6047-6052), Lee et al. 2002 (Nature Biotechnology 20: 500-505), Xia et al. 2002 (Nature Biotechnology 20: 1006-1010). It has been shown by several studies that an RNAi approach is suitable for the development of a potential treatment of dominantly inherited diseases by designing a siRNA that specifically targets the disease-associated mutant allele, thereby selectively silencing expression from the mutant gene (Miller et al. 2003, Proc. Natl. Acad. Sci. U.S.A. 100: 7195-7200; Gonzalez-Alegre et al. 2003, Ann. Neurol. 53: 781-787).

The siRNA molecules are essentially double-stranded but may comprise 3' or 5' overhangs. They may also comprise sequences that are not identical or essentially identical with the target gene but these sequences must be located outside of the sequence of identity. The sequence of identity or substantial identity is at least 14 and more preferably at least 19 nucleotides long. It preferably does not exceed 23 nucleotides. Optionally, the siRNA comprises two regions of identity or substantial identity that are interspersed by a region of non-identity. The term "substantial identity" refers to a region that has one or two mismatches of the sense strand of the siRNA to the targeted mRNA or 10 to 15% over the total length of siRNA to the targeted mRNA mismatches within the region of identity. Said mismatches may be the result of a nucleotide substitution, addition, deletion or duplication etc. dsRNA longer than 23 but no longer than 40 bp may also contain three or four mismatches.

The interference of the siRNA with the targeted mRNA has the effect that transcription/translation is reduced by at least 50%, preferably at least 75%, more preferred at least 90%, still more preferred at least 95%, such as at least 98% and most preferred at least 99%.

Further, the modulator can be an antisense nucleic acid molecule specifically hybridizing to a nucleic acid molecule encoding coagulation factor XII or regulating the expression of coagulation factor XII. The term "antisense nucleic acid molecule" refers to a nucleic acid molecule which can be used for controlling gene expression. The underlying technique, antisense technology, can be used to control gene expression through antisense DNA or RNA or through triple-helix formation. Antisense techniques are discussed, for example, in Okano, J. Neurochem. 56: 560 (1991); "Oligodeoxynucleotides as Antisense Inhibitors of Gene Expression." CRC Press, Boca Raton, FL (1988), or in: Phillips MI (ed.), Antisense Technology, Methods in Enzymology, Vol. 313, Academic Press, San Diego (2000). Triple helix formation is discussed in, for instance, Lee et al., Nucleic Acids Research 6: 3073 (1979); Cooney et al., Science 241: 456 (1988); and Dervan et al., Science 251: 1360 (1991). The methods are based on binding of a target polynucleotide to a complementary DNA or RNA. For example, the 5' coding portion of a polynucleotide that encodes a coagulation factor XII (poly)peptide may be used to design an antisense RNA oligonucleotide of from about 10 to 40 base pairs in length. A DNA oligonucleotide is designed to be complementary to a gene region involved in transcription thereby preventing transcription and the production of coagulation factor XII. The antisense RNA oligonucleotide hybridizes to the mRNA *in vivo* and blocks translation of the mRNA molecule into coagulation factor XII polypeptide.

The term "ribozyme" refers to RNA molecules with catalytic activity (see, e.g., Sarver et al, Science 247:1222-1225 (1990)); however, DNA catalysts (deoxyribozymes) are also known. Ribozymes and their potential for the development of new therapeutic tools are discussed, for example, by Steele et al. 2003 (Am. J. Pharmacogenomics 3: 131-144) and by Puerta-Femandez et al. 2003 (FEMS Microbiology Reviews 27: 75-97). While ribozymes that cleave mRNA at site specific recognition sequences can be used to destroy coagulation factor XII mRNAs, the use of trans-acting hairpin or hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Haseloff and Gerlach, Nature 334:585-591 (1988). There are numerous potential hammerhead ribozyme cleavage sites within the nucleotide sequence of the coagulation factor XII mRNA which will be apparent to the person skilled in the art. Preferably, the ribozyme is engineered so that the cleavage recognition site is located near the 5' end of the coagulation factor XII mRNA; i.e., to increase efficiency and minimize the intracellular accumulation of non-functional mRNA transcripts. RNase P is another ribozyme approach used for the selective inhibition of pathogenic RNAs. Ribozymes may be composed of modified oligonucleotides (e.g. for improved stability, targeting, etc.) and should be delivered to cells which express coagulation factor XII. DNA constructs encoding the ribozyme may be introduced into the cell in the same manner as described above for the introduction of other nucleic acid molecules. A preferred method of delivery involves using a DNA construct "encoding" the ribozyme under the control of a strong constitutive promoter, such as, for example, pol III or pol II promoter, so that transfected cells will produce sufficient quantities of the ribozyme to destroy endogenous coagulation factor XII messages and inhibit translation. Since ribozymes unlike antisense molecules, are catalytic, a lower intracellular concentration is generally required for efficiency. Ribozyme-mediated RNA repair is another therapeutic option applying ribozyme technologies (Watanabe & Sullenger 2000, Adv. Drug Deliv. Rev. 44: 109-118) and may also be useful for the purpose of the present invention. To this end, catalytic group I introns can be employed in a trans-splicing reaction to replace a defective segment of target mRNA in order to alleviate a mutant phenotype.

In a preferred embodiment of the method of the present invention, coagulation factor XII is a disease-associated mutant of coagulation factor XII. As pointed out above, in order to determine whether or not a mutation is disease-associated, the person skilled in the art may, for example, compare the frequency of a specific sequence change, for example in the coagulation factor XII gene, in patients affected by HAE type III with the frequency in appropriately chosen control individuals and conclude from a statistically significantly deviating frequency in the patient group that said mutation is a disease-associated mutation.
In another preferred embodiment of the present invention, said modulator is selective for a disease-associated mutant of coagulation factor XII, the method comprising (a) comparing the effect of the modulator on wild-type and disease-associated coagulation factor XII activity or their expression and/or secretion; and (b) selecting a compound which (i) modulates disease-associated coagulation factor XII activity or its expression and/or secretion and which (ii) does not affect wild-type coagulation factor XII activity or its expression and/or secretion. By using this method, the skilled person can determine whether a modulating compound is a general modulator of coagulation factor XII or selective for disease-associated coagulation factor XII. It is also possible and envisaged that a modulator affects preferably disease-associated coagulation factor XII, and partially, but to a lesser extent, also wild-type coagulation factor XII.

In yet another preferred embodiment of the present invention's methods, the disease-associated mutant or mutation is: (a) a mutant located in the fibronectin type II domain, within the region of amino acid position 1 to 76, and/or a mutation located in the nucleic acid sequence encoding the fibronectin type II domain, within mRNA position 107 to 334; (b) a mutant located in the EGF-like domain 1, within the region of amino acid position 77 to 113, and/or a mutation located in the nucleic acid sequence encoding the EGF-like domain 1, within mRNA position 335 to 445; (c) a mutant located in the fibronectin type I domain, within the region of amino acid position 114 to 157, and/or a mutation located in the nucleic acid sequence encoding the fibronectin type I domain, within mRNA position 446 to 577; (d) a mutant located in the EGF-like domain 2, within the region of amino acid position 158 to 192, and/or a mutation located in the nucleic acid sequence encoding the EGF-like domain 2, within mRNA position 578 to 682; (e) a mutant located in the kringle domain, within the region of amino acid position 193 to 276, and/or a mutation located in the nucleic acid sequence encoding the kringle domain, within mRNA position 683 to 934; (f) a mutant located in the proline-rich region, within the region of amino acid position 277 to 331, and/or a mutation located in the nucleic acid sequence encoding the proline-rich region, within mRNA position 935 to 1099; (g) a mutant located in the region of proteolytic cleavage sites, within the region of amino acid position 332 to 353, and/or a mutation located in the nucleic acid sequence encoding the region of proteolytic cleavage sites, within mRNA position 1100 to 1165; (h) a mutant located in the serine protease domain, within the region of amino acid position 354 to 596, and/or a mutation located in the nucleic acid sequence encoding the serine protease domain, within mRNA position 1166 to 1894; (i) a mutant located in the signal peptide, within the region of amino acid position -19 to -1, and/or a mutation located in the nucleic acid sequence encoding the signal peptide, within mRNA position 50 to 106; (j) a mutation located in the untranslated regions (UTRs) of coagulation factor XII mRNA, within mRNA position 1 to 49 and/or 1895 to 2048; (k) a mutation located in an intron of the coagulation factor XII gene; and/or (I) a mutation located in a flanking regulatory genomic sequence of the coagulation factor XII gene, within the region encompassing 4000bp upstream of the transcription initiation site of the coagulation factor XII gene and/or within the region encompassing 3000bp downstream of the nucleotide sequence representing the 3'-UTR of the coagulation factor XII mRNA.
The above numbering of amino acid residues of human coagulation factor XII refers to the numbering as given for example in Cool & MacGillivray 1987 (J. Biol. Chem. 262: 13662-13673). The numbering of mRNA positions refers to GenBank acc. no. NM_000505.2. Introns of the coagulation factor XII gene are preferably introns one to thirteen as given for example in the Seattle data (http://pga.gs.washington.edu/data/f12/f12.ColorFasta.html) or in the UCSC Genome Browser/July 2003 human reference sequence/chr5:176,810,093-176,817,530. Also according to the July 2003 human refence sequence of the UCSC Genome Browser, flanking regulatory sequences of the coagulation factor XII gene, as given above, encompass nucleotide positions chr5:176,817,531 to 176,821,030 and nucleotide positions chr5:176,807,093 to 176,810,092.

In a preferred embodiment, the present invention's method comprises the additional step of producing the modulator identified in said methods.

In another preferred embodiment, the present invention's method comprises in vitro testing of a sample of a blood donor for determining whether the blood of said donor or components thereof may be used for transfusion to a patient in need thereof, wherein a positive testing indicates a predisposition for hereditary angioedema type III, excluding the transfusion of blood or components thereof from said donor.

The present invention also relates to the use of (a) a (poly)peptide encoded by the coagulation factor XII gene or a fragment thereof, (b) a modulator of coagulation factor XII identified by any of the methods of claims 13 to 21; (c) a nucleic acid molecule capable of expressing coagulation factor XII or a fragment thereof; and/or (d) a nucleic acid molecule capable of expressing a modulator of coagulation factor XII activity or its expression and/or secretion, for the preparation of a pharmaceutical composition for the treatment and/or prevention of hereditary angioedema type III. Said modulator of coagulation factor XII may be any of the modulating compounds identified by the methods of the present invention or any of the modulating compounds disclosed in the present invention. As such, the modulator may be affecting the expression from the coagulation factor XII gene or may modulate the secretion or function of coagulation factor XII. Preferably, the modulating compound is an inhibitor of coagulation factor XII activity or of its expression or secretion. The use of (a) and (c) may be envisaged, for example, with the purpose of a vaccination, either protein-based or DNA-based, to stimulate an immune response against coagulation factor XII (vide infra).

The active components of a pharmaceutical composition such as, e.g. a small molecular compound or an antibody, will be formulated and dosed in a fashion consistent with good medical practice, taking into account the clinical condition of the individual patient, the site of delivery of pharmaceutical composition, the method of administration, the scheduling of administration, and other factors known to practitioners. The "effective amount" of the components of the pharmaceutical composition for purposes herein is thus determined by such considerations.

As a general proposition, the total pharmaceutically effective amount of for example a proteinaceous compound administered parenterally per dose will be in the range of about 1 µg/kg/day to 10 mg/kg/day of patient body weight, although, as noted above, this will be subject to therapeutic discretion. The length of treatment needed to observe changes and the interval following treatment for responses to occur appears to vary depending on the desired effect. Pharmaceutical compositions may be administered orally, rectally, parenterally, intracistemally, intravaginally, intraperitoneally, topically (as by powders, ointments, drops or transdermal patch), bucally, or as an oral or nasal spray. By "pharmaceutically acceptable carrier" is meant a non-toxic solid, semisolid or liquid filler, diluent, encapsulating material or formulation auxiliary of any type. The term "parenteral" as used herein refers for example to modes of administration which include intravenous, intramuscular, intraperitoneal, intrastemal, subcutaneous and intraarticular injection and infusion.

The pharmaceutical composition is also suitably administered by sustained-release systems. Suitable examples of sustained-release compositions include semipermeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., *Biopolymers 22*:547-556 (1983)), poly (2- hydroxyethyl methacrylate (R. Langer et al., *J. Biomed. Mater. Res. 15*:167-277 (1981), and R. Langer, *Chem. Tech. 12*:98-105 (1982)), ethylene vinyl acetate (R. Langer et al., Id.) or poly-D- (-)-3-hydroxybutyric acid (EP 133,988). Sustained-release compositions also include for example liposomally entrapped components. Liposomes containing the active components of the pharmaceutical composition are prepared by methods known per se: DE 3,218,121; Epstein et al., Proc. Natl. Acad. Sci. (USA) 82:3688-3692 (1985); Hwang et al., Proc. Natl. Acad. Sci. (USA) 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal therapy.

Components to be used for therapeutic administration must be sterile. Sterility is readily accomplished by filtration through sterile filtration membranes (e.g., 0.2 micron membranes). Therapeutic compositions generally are placed into a container having a sterile access port, for example, an intravenous solution bag or vial having a stopper pierceable by a hypodermic injection needle.

It is further envisaged in a preferred embodiment of the present invention's use, that said coagulation factor XII or said (poly)peptide is a mutant coagulation factor XII or mutant (poly)peptide or a fragment thereof. In one embodiment, the mutant is a disease-associated mutant of coagulation factor XII or a fragment thereof, which may be used, for example, for preparation of a vaccine to stimulate an immune response. In such a case, a fragment of coagulation factor XII would comprise at least 5, 6, 7, 8 or 9 consecutive amino acid residues of coagulation factor XII to provide an effective immunogen. Preferably, for this purpose the fragment would be a fragment comprising the mutant position of the disease-associated coagulation factor XII (poly)peptide. The use of modified, chimeric peptide constructs and other methods for creating a sufficient immunogenicity are known in the art (see e.g. Rittershaus et al. 2000, Arterioscler. Thromb. Vasc. Biol. 20:2106-2112). Alternatively, it is conceivable to engineer coagulation factor XII in such a way that the resulting mutant can for example displace a disease-associated mutant coagulation factor XII (poly)peptide from one of its interaction partners. Administering such a recombinant, i.e. mutant coagulation factor XII construct to a host may therefore be useful in treating, eventually also in preventing HAE type III. With respect to a modulator used for the preparation of a pharmaceutical composition and/or a nucleic acid molecule expressing a modulator it is envisaged here that the targeted coagulation factor XII (poly)peptide, or gene or mRNA species, is or contains a disease-associated mutant or mutation.

In a more preferred embodiment of the present invention's use, it is envisaged that the mutant is or is based on: (a) a mutant located in the fibronectin type II domain, within the region of amino acid position 1 to 76, and/or a mutation located in the nucleic acid sequence encoding the fibronectin type II domain, within mRNA position 107 to 334; (b) a mutant located in the EGF-like domain 1, within the region of amino acid position 77 to 113, and/or a mutation located in the nucleic acid sequence encoding the EGF-like domain 1, within mRNA position 335 to 445; (c) a mutant located in the fibronectin type I domain, within the region of amino acid position 114 to 157, and/or a mutation located in the nucleic acid sequence encoding the fibronectin type I domain, within mRNA position 446 to 577; (d) a mutant located in the EGF-like domain 2, within the region of amino acid position 158 to 192, and/or a mutation located in the nucleic acid sequence encoding the EGF-like domain 2, within mRNA position 578 to 682; (e) a mutant located in the kringle domain, within the region of amino acid position 193 to 276, and/or a mutation located in the nucleic acid sequence encoding the kringle domain, within mRNA position 683 to 934; (f) a mutant located in the proline-rich region, within the region of amino acid position 277 to 331, and/or a mutation located in the nucleic acid sequence encoding the proline-rich region, within mRNA position 935 to 1099; (g) a mutant located in the region of proteolytic cleavage sites, within the region of amino acid position 332 to 353, and/or a mutation located in the nucleic acid sequence encoding the region of proteolytic cleavage sites, within mRNA position 1100 to 1165; (h) a mutant located in the serine protease domain, within the region of amino acid position 354 to 596, and/or a mutation located in the nucleic acid sequence encoding the serine protease domain, within mRNA position 1166 to 1894; (i) a mutant located in the signal peptide, within the region of amino acid position -19 to -1, and/or a mutation located in the nucleic acid sequence encoding the signal peptide, within mRNA position 50 to 106; (j) a mutation located in the untranslated regions (UTRs) of coagulation factor XII mRNA, within mRNA position 1 to 49 and/or 1895 to 2048; (k) a mutation located in an intron of the coagulation factor XII gene; and/or (I) a mutation located in a flanking regulatory genomic sequence of the coagulation factor XII gene, within the region encompassing 4000bp upstream of the transcription initiation site of the coagulation factor XII gene and/or within the region encompassing 3000bp downstream of the nucleotide sequence representing the 3'-UTR of the coagulation factor XII mRNA. Numbering of sequences etc. is as outlined earlier (vide supra).

In a more preferred embodiment of the present invention's use, it is envisaged that the modulator is an inhibitor of coagulation factor XII, its activity, its expression and/or its secretion, comprising: (a) an aptamer or an inhibitory antibody or fragment or derivative thereof, specifically binding to and/or specifically inhibiting the activity of (i) disease-associated coagulation factor XII or (ii) wild-type and disease-associated coagulation factor XII; (b) a small molecule inhibitor of (i) disease-associated coagulation factor XII and/or disease-associated coagulation factor XII activity; or (ii) wild-type and disease-associated coagulation factor XII and/or wild-type and disease-associated coagulation factor XII activity; (c) a serine protease inhibitor of (i) disease-associated coagulation factor XII or of (ii) wild-type and disease-associated coagulation factor XII selected from a first group consisting of wild-type and modified or engineered proteinaceous inhibitors of serine proteases including C1 esterase inhibitor, antithrombin III, α2-antiplasmin, α1-antitrypsin, ovalbumin serpins, and α2-macroglobulin, or selected from a second group consisting of Kunitz-type inhibitors including bovine pancreatic trypsin inhibitor; or (d) a siRNA or shRNA, a ribozyme or an antisense nucleic acid molecule specifically hybridizing to a nucleic acid molecule encoding coagulation factor XII or regulating the expression of coagulation factor XII, either affecting (i) disease-associated coagulation factor XII or (ii) wild-type and disease-associated coagulation factor XII. In general, it may be a preferable type of treatment to target specifically the disease-associated mutant coagulation factor XII, its activity, expression and/or secretion. However, it may also be possible to use an inhibitor that targets wild-type as well as disease-associated mutant coagulation factor XII, their activity, expression or secretion; such an option appears particularly reasonable whenever the treatment is not a long-term or ultralong-term treatment.

The present invention also relates to a method of gene therapy in a mammal, characterized by administering an effective amount of a nucleic acid molecule capable of expressing in the mammal: (a) siRNA or shRNA, a ribozyme or an antisense nucleic acid molecule specifically hybridizing to a nucleic acid molecule encoding coagulation factor XII or regulating its expression; (b) an aptamer or an inhibitory antibody or fragment or derivative thereof, specifically binding coagulation factor XII (poly)peptide; (c) coagulation factor XII or a fragment thereof; or (d) a serine protease inhibitor selected from group (i) consisting of wild-type and modified or engineered proteinaceous inhibitors of serine proteases including C1 esterase inhibitor, antithrombin III, α2-antiplasmin, α1-antitrypsin, ovalbumin serpins, and α2-macroglobulin, or selected from group (ii) of Kunitz-type inhibitors including bovine pancreatic trypsin inhibitor.

The gene therapy method relates to the introduction of nucleic acid sequences, DNA, RNA and/or antisense DNA or RNA sequences, into a mammal. This method requires a nucleic acid construct capable of expressing in the mammal (a) siRNA or shRNA, a ribozyme, or an antisense nucleic acid molecule specifically hybridizing to a nucleic acid molecule encoding or regulating the expression of coagulation factor XII; (b) an aptamer or an inhibitory antibody or fragment or derivative thereof, specifically binding coagulation factor XII (poly)peptide; (c) coagulation factor XII or a fragment thereof; or (d) a proteinaceous serine protease inhibitor, for example C1 esterase inhibitor, antithrombin III, α2-antiplasmin, α2-macroglobulin, α1-antitrypsin, an ovalbumin serpin, or a Kunitz-type inhibitor, modified or engineered in such a way to specifically inhibit coagulation factor XII, preferably disease-associated mutant coagulation factor XII, and any other genetic elements necessary for the expression of the desired (poly)peptide or nucleic acid molecule by the target tissue. Such gene therapy and delivery techniques are known in the art; see, for example, WO90/11092, which is herein incorporated by reference, or: M. I. Phillips (Ed.): Gene Therapy Methods. Methods in Enzymology, Vol. 346, Academic Press, San Diego 2002. Thus, for example, cells from a patient may be engineered ex vivo with a nucleic acid construct comprising a promoter operably linked to the nucleic acid molecule corresponding to the molecule to be introduced, with the engineered cells then being provided to a patient to be treated. Such methods are well-known in the art. For example, see Belldegrun, A., et al., J. Natl. Cancer Inst. 85: 207-216 (1993); Ferrantini, M. et al., Cancer Research 53: 1107-1112 (1993); Ferrantini, M. et al., J. Immunology 153: 4604-4615 (1994); Kaido, T., et al., Int. J. Cancer 60: 221-229 (1995); Ogura, H., et al., Cancer Research 50: 5102-5106 (1990); Santodonato, L., et al., Human Gene Therapy 7:1-10 (1996); Santodonato, L., et al., Gene Therapy 4:1246-1255 (1997); and Zhang, J.-F. et al., Cancer Gene Therapy 3: 31-38 (1996)), which are herein incorporated by reference. The cells which are engineered may be, for example, blood or liver cells. The nucleic acid construct used in gene therapy can be delivered by any method that delivers injectable materials to the cells of an animal, such as, injection into the interstitial space of tissues (heart, muscle, skin, lung, liver, and the like). The nucleic acid molecule used in gene therapy may be delivered in a pharmaceutically acceptable liquid or aqueous carrier.

The nucleic acid molecules may be delivered as a naked nucleic acid molecule. The term "naked" nucleic acid molecule, DNA or RNA refers to sequences that are free from any delivery vehicle that acts to assist, promote or facilitate entry into the cell, including viral sequences, viral particles, liposome formulations, lipofectin or precipitating agents and the like. However, the nucleic acid molecules used in gene therapy can also be delivered in liposome formulations and lipofectin formulations and the like that can be prepared by methods well known to those skilled in the art. Such methods are described, for example, in U.S. Patent Nos. 5,593,972, 5,589,466, and 5,580,859, which are herein incorporated by reference.

The vector constructs used in the gene therapy method are preferably constructs that will not integrate into the host genome nor will they contain sequences that allow for replication. Appropriate vectors include pWLNEO, pSV2CAT, pOG44, pXT1 and pSG available from Stratagene; pSVK3, pBPV, pMSG and pSVL available from Pharmacia; and pEF1/V5, pcDNA3.1, and pRc/CMV2 available from Invitrogen. Other suitable vectors will be readily apparent to the skilled artisan. Any strong promoter known to those skilled in the art can be used for driving the expression from the nucleic acid molecule used in gene therapy. Suitable promoters include adenoviral promoters, such as the adenoviral major late promoter; or heterologous promoters, such as the cytomegalovirus (CMV) promoter; the respiratory syncytial virus (RSV) promoter; inducible promoters, such as the MMT promoter, the metallothionein promoter; heat shock promoters; the albumin promoter; the ApoAl promoter; human globin promoters; viral thymidine kinase promoters, such as the Herpes Simplex thymidine kinase promoter; retroviral LTRs; the b-actin promoter; and human growth hormone promoters. The promoter also may be the native promoter of coagulation factor XII or of any of the polypeptides expressed in gene therapy. Unlike other gene therapy techniques, one major advantage of introducing naked nucleic acid sequences into target cells is the transitory nature of the nucleic acid molecule synthesis in the cells. Studies have shown that non-replicating DNA sequences can be introduced into cells to provide production of the desired polypeptide for periods of up to six months.

The nucleic acid molecules used in gene therapy can be delivered to the interstitial space of tissues within an animal, including of muscle, skin, brain, lung, liver, spleen, bone marrow, thymus, heart, lymph, blood, bone, cartilage, pancreas, kidney, gall bladder, stomach, intestine, testis, ovary, uterus, rectum, nervous system, eye, gland, and connective tissue. Interstitial space of the tissues comprises the intercellular fluid, mucopolysaccharide matrix among the reticular fibers of organ tissues, elastic fibers in the walls of vessels or chambers, collagen fibers of fibrous tissues, or that same matrix within connective tissue ensheathing muscle cells or in the lacunae of bone. They may be conveniently delivered by injection into the tissues comprising these cells. They are preferably delivered to and expressed in persistent, non-dividing cells which are differentiated, although delivery and expression may be achieved in non-differentiated or less completely differentiated cells, such as, for example, stem cells of blood or skin fibroblasts. In vivo muscle cells are particularly competent in their ability to take up and express polynucleotides.

For the naked nucleic acid sequence injection, an effective dosage amount of DNA or RNA will be in the range of from about 0.0005 mg/kg body weight to about 50 mg/kg body weight. Preferably the dosage will be from about 0.005 mg/kg to about 20 mg/kg and more preferably from about 0.05 mg/kg to about 5 mg/kg. Of course, as the artisan of ordinary skill will appreciate, this dosage will vary according to the tissue site of injection. The appropriate and effective dosage of nucleic acid molecules can readily be determined by those of ordinary skill in the art and may depend on the condition being treated and the route of administration. The preferred route of administration is by the parenteral route of injection into the interstitial space of tissues. However, other parenteral routes may also be used, such as, inhalation of an aerosol formulation particularly for delivery to lungs or bronchial tissues, throat or mucous membranes of the nose.

The naked nucleic acid molecules are delivered by any method known in the art, including, but not limited to, direct needle injection at the delivery site, intravenous injection, topical administration, catheter infusion, and so-called "gene guns". These delivery methods are known in the art. The constructs may also be delivered with delivery vehicles such as viral sequences, viral particles, liposome formulations, lipofectin, precipitating agents, etc.

Liposomal preparations for use in the instant invention include cationic (positively charged), anionic (negatively charged) and neutral preparations. However, cationic liposomes are particularly preferred because a tight charge complex can be formed between the cationic liposome and the polyanionic nucleic acid. Cationic liposomes have been shown to mediate intracellular delivery of plasmid DNA (Felgner et al., Proc. Natl. Acad. Sci. USA (1987) 84:7413-7416, which is herein incorporated by reference); mRNA (Malone et al., Proc. Natl. Acad. Sci. USA (1989) 86:6077-6081, which is herein incorporated by reference); and purified transcription factors (Debs et al., J. Biol. Chem. (1990) 265:10189-10192, which is herein incorporated by reference), in functional form. Cationic liposomes are readily available. For example, N[1-2,3-dioleyloxy)propyl]-N,N,N-triethylammonium (DOTMA) liposomes are particularly useful and are available under the trademark Lipofectin, from GIBCO BRL, Grand Island, N.Y. (See, also, Felgner et al., Proc. Natl Acad. Sci. USA (1987) 84:7413-7416). Other commercially available liposomes include transfectace (DDAB/DOPE) and DOTAP/DOPE (Boehringer). Other cationic liposomes can be prepared from readily available materials using techniques well known in the art. See, e.g. PCT Publication No. WO 90/11092 (which is herein incorporated by reference) for a description of the synthesis of DOTAP (1,2-bis(oleoyloxy)-3-(trimethylammonio)propane) liposomes. Preparation of DOTMA liposomes is explained in the literature, see, e.g., Felgner et al., Proc. Natl. Acad. Sci. USA 84:7413-7417, which is herein incorporated by reference. Similar methods can be used to prepare liposomes from other cationic lipid materials. Similarly, anionic and neutral liposomes are readily available, such as from Avanti Polar Lipids (Birmingham, Ala.), or can be easily prepared using readily available materials. Such materials include phosphatidyl, choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art. For example, commercially available dioleoylphosphatidyl choline (DOPC), dioleoylphosphatidyl glycerol (DOPG), and dioleoylphosphatidyl ethanolamine (DOPE) can be used in various combinations to make conventional liposomes, with or without the addition of cholesterol. Thus, for example, DOPG/DOPC vesicles can be prepared by drying 50 mg each of DOPG and DOPC under a stream of nitrogen gas into a sonication vial. The sample is placed under a vacuum pump overnight and is hydrated the following day with deionized water. The sample is then sonicated for 2 hours in a capped vial, using a Heat Systems model 350 sonicator. Alternatively, negatively charged vesicles can be prepared without sonication to produce multilamellar vesicles or by extrusion through nucleopore membranes to produce unilamellar vesicles of discrete size. Other methods are known and available to those of skill in the art.

Generally, the ratio of nucleic acid to liposomes will be from about 10:1 to about 1:10. Preferably, the ratio will be from about 5:1 to about 1:5. More preferably, the ratio will be about 3:1 to about 1:3. Still more preferably, the ratio will be about 1:1.

In certain embodiments, cells are engineered, ex vivo or in vivo, using a retroviral particle containing RNA which comprises a sequence encoding any of the nucleic acid molecules or (poly)peptides used in the method of gene therapy. Retroviruses from which the retroviral plasmid vectors may be derived include, but are not limited to, Moloney Murine Leukemia Virus, spleen necrosis virus, Rous sarcoma Virus, Harvey Sarcoma Virus, avian leukosis virus, gibbon ape leukemia virus, human immunodeficiency virus, Myeloproliferative Sarcoma Virus, and mammary tumor virus. The retroviral plasmid vector is employed to transduce packaging cell lines to form producer cell lines. Examples of packaging cells which may be transfected include, but are not limited to, the PE501, PA317, R-2, R-AM, PA12, T19-14X, VT-19-17-H2, RCRE, RCRIP, GP+E-86, GP+envAm12, and DAN cell lines as described in Miller, Human Gene Therapy 1:5-14 (1990), which is incorporated herein by reference in its entirety. The vector may transduce the packaging cells through any means known in the art. Such means include, but are not limited to, electroporation, the use of liposomes, and CaPO₄ precipitation. In one alternative, the retroviral plasmid vector may be encapsulated into a liposome, or coupled to a lipid, and then administered to a host. The producer cell line generates infectious retroviral vector particles which include the nucleic acid molecule encoding the (poly)peptide or the therapeutically active nucleic acid, such as siRNA, intended to be used for gene therapy. Such retroviral vector particles then may be employed, to transduce eukaryotic cells, either in vitro or in vivo.

In certain other embodiments, cells are engineered, ex vivo or in vivo, with a nucleic acid molecule to be used in gene therapy, contained in an adenovirus vector. Adenovirus can be manipulated such that it expresses a construct of interest, and at the same time is inactivated in terms of its ability to replicate in a normal lytic viral life cycle. Adenovirus expression is achieved without integration of the viral DNA into the host cell chromosome, thereby alleviating concerns about insertional mutagenesis. Furthermore, adenoviruses have been used as live enteric vaccines for many years with an excellent safety profile (Schwartz, A. R. et al. (1974) Am. Rev. Respir. Dis.109:233-238). Finally, adenovirus mediated gene transfer has been demonstrated in a number of instances including transfer of alpha-1-antitrypsin and CFTR to the lungs of cotton rats (Rosenfeld, M. A. et al. (1991) Science 252:431-434; Rosenfeld et al., (1992) Cell 68:143-155). Furthermore, extensive studies to attempt to establish adenovirus as a causative agent in human cancer were uniformly negative (Green, M. et al. (1979) Proc. Natl. Acad. Sci. USA 76:6606). Suitable adenoviral vectors useful in the present invention are described, for example, in Kozarsky and Wilson, Curr. Opin. Genet. Devel. 3:499-503 (1993); Rosenfeld et al., Cell 68:143-155 (1992); Engelhardt et al., Human Genet. Ther. 4:759-769 (1993); Yang et al., Nature Genet. 7:362-369 (1994); Wilson et al., Nature 365:691-692 (1993); and U.S. Patent No. 5,652,224, which are herein incorporated by reference. For example, the adenovirus vector Ad2 is useful and can be grown in human 293 cells. These cells contain the E1 region of adenovirus and constitutively express E1a and E1b, which complement the defective adenoviruses by providing the products of the genes deleted from the vector. In addition to Ad2, other varieties of adenovirus (e.g., Ad3, Ad5, and Ad7) are also useful in the present invention. Preferably, the adenoviruses used in the present invention are replication deficient. Replication deficient adenoviruses require the aid of a helper virus and/or packaging cell line to form infectious particles. The resulting virus is capable of infecting cells and can express a gene of interest which is operably linked to a promoter, but cannot replicate in most cells. Replication deficient adenoviruses may be deleted in one or more of all or a portion of the following genes: Ela, E1b, E3, E4, E2a, or L1 through L5.

The present invention also relates to a non-human transgenic animal, comprising as a transgene: (a) a gene encoding human disease-associated coagulation factor XII; (b) (i) a gene encoding human disease-associated coagulation factor XII and (ii) a gene encoding human wild-type coagulation factor XII; (c) a nucleic acid molecule causing an altered expression of human coagulation factor XII and a gene encoding human wild-type coagulation factor XII; and/or (d) a species-specific coagulation factor XII gene which is specifically altered to contain a human disease-associated mutation.

Said transgenic animal of (a) to (d) will be very important, for example, for studying the pathophysiological consequences of certain coagulation factor XII alterations, and for the screening of new medicaments effective in the treatment and/or prevention of hereditary angioedema type III. Preferably, said animal is a mammalian animal, including, but not limited to, rat, mouse, cat, hamster, dog, rabbit, pig, or monkey, but can also be, for example, C. elegans or a fish, such as Torpedo fish.

The non-human transgenic animal of (b) will be valuable for example for studying a heterozygous situation, including possible dominant negative effects of a disease-associated mutation. Further it may allow to investigate potential differential effects of a medicament, including any of the modulators discussed above, on wild-type and disease-associated human coagulation factor XII. The non-human transgenic animal of (c) may allow for example to study the consequences and potential treatment of a mutated nucleic acid that leads to an altered expression of human coagulation factor XII. As envisaged here, such a mutation could relate for example to a nucleic acid molecule which in the human genome is physically unrelated to the coagulation factor XII gene. It is also envisaged that, for example in case of a mutation at a highly conserved position or within a functionally conserved motif, the human disease or disease predisposition can be imitated in the animal by altering the animal's species-specific coagulation factor XII gene to contain a human disease-associated mutation.

A method for the production of a transgenic non-human animal, for example transgenic mouse, comprises introduction of the desired polynucleotide, for example a nucleic acid encoding human wild-type or disease-associated mutant coagulation factor XII, or targeting vector into a germ cell, an embryonic cell, stem cell or an egg or a cell derived therefrom. Production of transgenic embryos and screening of those can be performed, e.g., as described by A. L. Joyner Ed., Gene Targeting, A Practical Approach (1993), Oxford University Press. The DNA of the embryonal membranes of embryos can be analyzed using, e.g., Southern blots with an appropriate probe. A general method for making transgenic non-human animals is described in the art, see for example WO 94/24274. For making transgenic non-human organisms (which include homologously targeted non-human animals), embryonal stem cells (ES cells) are preferred. Murine ES cells, such as AB-1 line grown on mitotically inactive SNL76/7 cell feeder layers (McMahon and Bradley, Cell 62: 1073-1085 (1990)), essentially as described in: Teratocarcinomas and Embryonic Stem Cells: A Practical Approach. E. J. Robertson, ed. (Oxford: IRL Press), 1987, pp. 71-112, may be used for homologous gene targeting. Other suitable ES lines include, but are not limited to, the E14 line (Hooper et al., Nature 326: 292-295 (1987)), the D3 line (Doetschman et al., J. Embryol. Exp. Morph. 87: 27-45 (1985)), the CCE line (Robertson et al., Nature 323: 445-448 (1986)), the AK-7 line (Zhuang et al., Cell 77: 875-884 (1994) which is incorporated by reference herein). The success of generating a mouse line from ES cells bearing a specific targeted mutation depends on the pluripotence of the ES cells (i. e., their ability, once injected into a host developing embryo, such as a blastocyst or morula, to participate in embryogenesis and contribute to the germ cells of the resulting animal). The blastocysts containing the injected ES cells are allowed to develop in the uteri of pseudopregnant nonhuman females and are bom as chimeric animals. The resultant transgenic animals are chimeric for cells having either the recombinase or reporter loci and are backcrossed and screened for the presence of the correctly targeted transgene (s) by PCR or Southern blot analysis on tail biopsy DNA of offspring so as to identify transgenic animals heterozygous for either the recombinase or reporter locus/loci.

Methods for producing transgenic flies, such as Drosophila melanogaster are also described in the art, see for example US-A-4,670,388, Brand & Perrimon, Development (1993) 118: 401-415; and Phelps & Brand, Methods (April 1998) 14: 367-379. Transgenic worms such as C. elegans can be generated as described in Mello, et al., (1991) Efficient gene transfer in C.elegans: extrachromosomal maintenance and integration of transforming sequences. Embo J 10, 3959-70, Plasterk, (1995) Reverse genetics: from gene sequence to mutant worm. Methods Cell Biol 48, 59-80.

In a preferred embodiment of the present invention, the non-human transgenic animal additionally expresses siRNA or shRNA, a ribozyme or an antisense nucleic acid molecule specifically hybridizing to the transgene(s) or to the altered species-specific gene contained in the transgenic animal. Preferably, said transgene(s) is/are of human origin. Such an approach can be useful, for example, for studying options for treatment and/or prevention for example by using RNA interference.

It may also be desirable to inactivate coagulation factor XII protein expression or function at a certain stage of development and/or life of the transgenic animal. This can be achieved by using, for example, tissue specific, developmental and/or cell regulated and/or inducible promoters which drive the expression of, e.g., an antisense or ribozyme directed against a mRNA encoding a coagulation factor XII (poly)peptide. A suitable inducible system is for example tetracycline-regulated gene expression as described, e.g., by Gossen and Bujard (Proc. Natl. Acad. Sci. 89 USA (1992), 5547-5551) and Gossen et al. (Trends Biotech. 12 (1994), 58-62). Similar, the expression of a mutant coagulation factor XII protein may be controlled by such regulatory elements.

In another preferred embodiment, the non-human transgenic animal's native species-specific genes encoding coagulation factor XII are inactivated. The term "inactivation" means reversible or irreversible inactivation. Appropriate methods to obtain such an inactivation are well known in the art. Such an approach may be useful in order to eliminate any effects of the animal's species-specific coagulation factor XII genes when studying for example the pathophysiological effects and/or the possible therapeutic targeting of the human transgene(s).

The present invention also relates to the use of any of the transgenic animals of the present invention, for screening for compounds for use in the diagnosis, prevention and/or treatment of hereditary angioedema type III.

Finally, the present invention also relates to a kit for use in diagnosis of hereditary angioedema type III or a susceptibility or predisposition thereto, said kit comprising: (a) at least one nucleic acid molecule capable of hybridizing under stringent conditions to a nucleic acid molecule encoding or regulating the expression of coagulation factor XII; (b) an antibody or an aptamer specific for coagulation factor XII or a fragment thereof and/or a disease-associated mutant of these; (c) a restriction enzyme capable of discriminating between wild-type and disease-associated mutant nucleic acid encoding or regulating the expression of coagulation factor XII; and/or (d) a pair of primers complementary to nucleic acid regulating the expression of coagulation factor XII or encoding wild-type and/or disease-associated coagulation factor XII; and optionally instructions for use. The nucleic acid molecule(s) of (a) may be suitable for example for use as probes or primers. Preferably, the kit will also provide means for detection of a reaction, e.g. nucleotide label detection means, labeled secondary antibodies or size detection means. The various compounds of the kit may be packed in one or more containers, optionally dissolved in suitable buffer for storage.

## Claims

1. A method of diagnosing hereditary angioedema type III (HAE III) or a predisposition thereto in a subject being suspected of having developed or of having a predisposition to develop a hereditary angioedema type III or in a subject being suspected of being a carrier for hereditary angioedema type III, the method comprising determining in vitro from a biological sample of said subject the presence or absence of a disease-associated mutation in a nucleic acid molecule regulating the expression of or encoding coagulation factor XII; wherein the presence of such a mutation is indicative of a hereditary angioedema type III or a predisposition thereto.

2. The method of claim 1, wherein said determination comprises hybridizing under stringent conditions to said nucleic acid molecule at least one pair of nucleic acid probes, the first probe of said pair being complementary to the wild-type sequence of said nucleic acid molecule and the second probe of said pair being complementary to the mutant sequence of said nucleic acid molecule, wherein a perfect match, the presence of stable hybridization, between (i) the first hybridization probe and the target nucleic acid molecule indicates the presence of a wild-type sequence, and (ii) the second hybridization probe and the target nucleic acid molecule, indicates the presence of a mutant sequence, wherein the first hybridization probe and the second hybridization probe allow a differential detection.

3. The method of claim 1, said method comprising hybridizing under stringent conditions to said nucleic acid molecule a hybridization probe specific for a mutant sequence.

4. The method of any one of claims 1 to 3, comprising a step of nucleic acid amplification and/or nucleic acid sequencing.

5. The method of any one of claims 1 to 4, wherein the method is or comprises an allele discrimination method selected from the group consisting of allele-specific hybridization, allele-specific primer extension including allele-specific PCR, allele-specific oligonucleotide ligation, allele-specific cleavage of a flap probe and/or allele-specific cleavage using a restriction endonuclease.

6. The method of any one of claims 1 to 5, comprising a detection method selected from the group consisting of fluorescence detection, time-resolved fluorescence, fluorescence resonance energy transfer (FRET), fluorescence polarization, colorimetric methods, mass spectrometry, (chemi)luminescence, electrophoretical detection and electrical detection methods.

7. The method of any one of claims 1 to 6, wherein the probe or the subject's nucleic acid molecule is attached to a solid support.

8. A method of diagnosing hereditary angioedema type III (HAE III) or a predisposition thereto in a subject being suspected of having developed or of having a predisposition to develop a hereditary angioedema type III or in a subject being suspected of being a carrier for hereditary angioedema type III, the method comprising assessing the presence, amount and/or activity of coagulation factor XII in said subject and including the steps of:
(a) determining from a biological sample of said subject in vitro, the presence, amount and /or activity of:
(i.) a (poly)peptide encoded by the coagulation factor XII gene;
(ii.) a substrate of the (poly)peptide of (i); or
(iii.) a (poly)peptide processed by the substrate mentioned in (ii);
(b) comparing said presence, amount and/or activity with that determined from a reference sample; and
(c) diagnosing, based on the difference between the samples compared in step (b), the pathological condition of a hereditary angioedema type III or a predisposition thereto.

9. The method of any one of claims 1 to 8, wherein the biological sample consists of or is taken from hair, skin, mucosal surfaces, body fluids, including blood, plasma, serum, urine, saliva, sputum, tears, liquor cerebrospinalis, semen, synovial fluid, amniotic fluid, milk, lymph, pulmonary sputum, bronchial secretion, or stool.

10. The method of claim 8 or 9, wherein said presence, amount and/or activity is determined by using an antibody or an aptamer, wherein the antibody or aptamer is specific for (a) a (poly)peptide encoded by the coagulation factor XII gene; (b) a substrate of the (poly)peptide of (a); or (c) a (poly)peptide processed by the substrate mentioned in (b).

11. The method of claim 10, wherein said antibody or aptamer is specific for a (poly)peptide encoded by the coagulation factor XII gene.

12. The method of any one of claims 8 or 9, wherein the presence, amount and/or activity of the (poly)peptide(s) encoded by the coagulation factor XII gene is determined in (a) a coagulation assay; or in (b) a functional amidolytic assay; or in (c) a mitogenic assay; or in (d) a binding assay measuring binding of a (poly)peptide encoded by the coagulation factor XII gene to a binding partner.

13. A method of identifying a compound modulating coagulation factor XII activity which is suitable as a medicament or a lead compound for a medicament for the treatment and/or prevention of hereditary angioedema type III, the method comprising the steps of:
(a) in vitro contacting a coagulation factor XII (poly)peptide or a functionally related (poly)peptide with the potential modulator; and
(b) testing for modulation of coagulation factor XII activity,
wherein modulation of coagulation factor XII activity is indicative of a compound's suitability as a medicament or a lead compound for a medicament for the treatment and/or prevention of hereditary angioedema type III.

14. The method of claim 13, wherein the coagulation factor XII (poly)peptide of step (a) is present in cell culture or cell culture supernatant or in a subject's sample or purified from any of these sources.

15. The method of claim 13 or 14, wherein said testing is performed by assessing the physical interaction between a coagulation factor XII (poly)peptide and the modulator and/or the effect of the modulator on the function of said coagulation factor XII (poly)peptide.

16. The method of any one of claims 13 to 15, wherein the modulator is an inhibitor of coagulation factor XII activity, selected from the group consisting of:
(a) an aptamer or inhibitory antibody or fragment or derivative thereof, specifically binding to a coagulation factor XII (poly)peptide and/or specifically inhibiting a coagulation factor XII activity;
(b) a small molecule inhibitor of coagulation factor XII and/or coagulation factor XII activity; and
(c) a serine protease inhibitor selected from group (I) consisting of wild-type and modified or engineered proteinaceous inhibitors of serine proteases including C1 esterase inhibitor, antithrombin III, α2-antiplasmin, α1-antitrypsin, ovalbumin serpins, and α2-macroglobulin, or selected from group (II) of Kunitz-type inhibitors including bovine pancreatic trypsin inhibitor.

17. A method of identifying a compound modulating coagulation factor XII expression and/or secretion which is suitable as a medicament or lead compound for a medicament for the treatment and/or prevention of hereditary angioedema type III, the method comprising the steps of:
(a) in vitro contacting a cell that expresses or is capable of expressing coagulation factor XII with a potential modulator of expression and/or secretion; and
(b) testing for altered expression and/or secretion,
wherein the modulator is (i) a small molecule compound, an aptamer or an antibody or fragment or derivative thereof, specifically modulating expression and/or secretion of coagulation factor XII; or (ii) a siRNA or shRNA, a ribozyme, or an antisense nucleic acid molecule specifically hybridizing to a nucleic acid molecule encoding coagulation factor XII or regulating the expression of coagulation factor XII.

18. The method of any one of claims 13 to 17, wherein coagulation factor XII is a disease-associated mutant of coagulation factor XII.

19. The method of any one of claims 13 to 18, wherein said modulator is selective for a disease-associated mutant of coagulation factor XII, the method comprising (a) comparing the effect of the modulator on wild-type and disease-associated coagulation factor XII activity or their expression and/or secretion; and (b) selecting a compound which (i) modulates disease-associated coagulation factor XII activity or its expression and/or secretion and which (ii) does not affect wild-type coagulation factor XII activity or its expression and/or secretion.

20. The method of any one of claims 1 to 19, wherein the disease-associated mutant or mutation is:
(a) a mutant located in the fibronectin type II domain, within the region of amino acid position 1 to 76, and/or a mutation located in the nucleic acid sequence encoding the fibronectin type II domain, within mRNA position 107 to 334;
(b) a mutant located in the EGF-like domain 1, within the region of amino acid position 77 to 113, and/or a mutation located in the nucleic acid sequence encoding the EGF-like domain 1, within mRNA position 335 to 445;
(c) a mutant located in the fibronectin type I domain, within the region of amino acid position 114 to 157, and/or a mutation located in the nucleic acid sequence encoding the fibronectin type I domain, within mRNA position 446 to 577;
(d) a mutant located in the EGF-like domain 2, within the region of amino acid position 158 to 192, and/or a mutation located in the nucleic acid sequence encoding the EGF-like domain 2, within mRNA position 578 to 682;
(e) a mutant located in the kringle domain, within the region of amino acid position 193 to 276, and/or a mutation located in the nucleic acid sequence encoding the kringle domain, within mRNA position 683 to 934;
(f) a mutant located in the proline-rich region, within the region of amino acid position 277 to 331, and/or a mutation located in the nucleic acid sequence encoding the proline-rich region, within mRNA position 935 to 1099;
(g) a mutant located in the region of proteolytic cleavage sites, within the region of amino acid position 332 to 353, and/or a mutation located in the nucleic acid sequence encoding the region of proteolytic cleavage sites, within mRNA position 1100 to 1165;
(h) a mutant located in the serine protease domain, within the region of amino acid position 354 to 596, and/or a mutation located in the nucleic acid sequence encoding the serine protease domain, within mRNA position 1166 to 1894;
(i) a mutant located in the signal peptide, within the region of amino acid position -19 to -1, and/or a mutation located in the nucleic acid sequence encoding the signal peptide, within mRNA position 50 to 106;
(j) a mutation located in the untranslated regions (UTRs) of coagulation factor XII mRNA, within mRNA position 1 to 49 and/or 1895 to 2048;
(k) a mutation located in an intron of the coagulation factor XII gene; and/or
(I) a mutation located in a flanking regulatory genomic sequence of the coagulation factor XII gene, within the region encompassing 4000bp upstream of the transcription initiation site of the coagulation factor XII gene and/or within the region encompassing 3000bp downstream of the nucleotide sequence representing the 3'-UTR of the coagulation factor XII mRNA.

21. The method of any one of claims 13 to 20, comprising the additional step of producing the modulator identified in said methods.

22. The method of any one of claims 1 to 12, comprising in vitro testing of a sample of a blood donor for determining whether the blood of said donor or components thereof may be used for transfusion to a patient in need thereof,
wherein a positive testing indicates a predisposition for hereditary angioedema type III, excluding the transfusion of blood or components thereof from said donor.

23. Use of (a) a (poly)peptide encoded by the coagulation factor XII gene or a fragment thereof, (b) a modulator of coagulation factor XII identified by any of the methods of claims 13 to 21; (c) a nucleic acid molecule capable of expressing coagulation factor XII or a fragment thereof; and/or (d) a nucleic acid molecule capable of expressing a modulator of coagulation factor XII activity or its expression and/or secretion, for the preparation of a pharmaceutical composition for the treatment and/or prevention of hereditary angioedema type III.

24. The use of claim 23, wherein said coagulation factor XII or said (poly)peptide is a mutant coagulation factor XII or mutant (poly)peptide or a fragment thereof.

25. The use of claim 24, wherein said mutant is or is based on:
(a) a mutant located in the fibronectin type II domain, within the region of amino acid position 1 to 76, and/or a mutation located in the nucleic acid sequence encoding the fibronectin type II domain, within mRNA position 107 to 334;
(b) a mutant located in the EGF-like domain 1, within the region of amino acid position 77 to 113, and/or a mutation located in the nucleic acid sequence encoding the EGF-like domain 1, within mRNA position 335 to 445;
(c) a mutant located in the fibronectin type I domain, within the region of amino acid position 114 to 157, and/or a mutation located in the nucleic acid sequence encoding the fibronectin type I domain, within mRNA position 446 to 577;
(d) a mutant located in the EGF-like domain 2, within the region of amino acid position 158 to 192, and/or a mutation located in the nucleic acid sequence encoding the EGF-like domain 2, within mRNA position 578 to 682
(e) a mutant located in the kringle domain, within the region of amino acid position 193 to 276, and/or a mutation located in the nucleic acid sequence encoding the kringle domain, within mRNA position 683 to 934;
(f) a mutant located in the proline-rich region, within the region of amino acid position 277 to 331, and/or a mutation located in the nucleic acid sequence encoding the proline-rich region, within mRNA position 935 to 1099;
(g) a mutant located in the region of proteolytic cleavage sites, within the region of amino acid position 332 to 353, and/or a mutation located in the nucleic acid sequence encoding the region of proteolytic cleavage sites, within mRNA position 1100 to 1165;
(h) a mutant located in the serine protease domain, within the region of amino acid position 354 to 596, and/or a mutation located in the nucleic acid sequence encoding the serine protease domain, within mRNA position 1166 to 1894;
(i) a mutant located in the signal peptide, within the region of amino acid position -19 to -1, and/or a mutation located in the nucleic acid sequence encoding the signal peptide, within mRNA position 50 to 106;
(j) a mutation located in the untranslated regions (UTRs) of coagulation factor XII mRNA, within mRNA position 1 to 49 and/or 1895 to 2048;
(k) a mutation located in an intron of the coagulation factor XII gene; and/or
(I) a mutation located in a flanking regulatory genomic sequence of the coagulation factor XII gene, within the region encompassing 4000bp upstream of the transcription initiation site of the coagulation factor XII gene and/or within the region encompassing 3000bp downstream of the nucleotide sequence representing the 3'-UTR of the coagulation factor XII mRNA.

26. The use of any one of claims 23 to 25, wherein said modulator is an inhibitor of coagulation factor XII, its activity, its expression and/or its secretion, comprising:
(a) an aptamer or an inhibitory antibody or fragment or derivative thereof, specifically binding to and/or specifically inhibiting the activity of (i) disease-associated coagulation factor XII or (ii) wild-type and disease-associated coagulation factor XII;
(b) a small molecule inhibitor of (i) disease-associated coagulation factor XII and/or disease-associated coagulation factor XII activity; or (ii) wild-type and disease-associated coagulation factor XII and/or wild-type and disease-associated coagulation factor XII activity;
(c) a serine protease inhibitor of (i) disease-associated coagulation factor XII or of (ii) wild-type and disease-associated coagulation factor XII selected from a first group consisting of wild-type and modified or engineered proteinaceous inhibitors of serine proteases including C1 esterase inhibitor, antithrombin III, α2-antiplasmin, α1-antitrypsin, ovalbumin serpins, and α2-macroglobulin, or selected from a second group consisting of Kunitz-type inhibitors including bovine pancreatic trypsin inhibitor; or
(d) a siRNA or shRNA, a ribozyme or an antisense nucleic acid molecule specifically hybridizing to a nucleic acid molecule encoding coagulation factor XII or regulating the expression of coagulation factor XII, either affecting (i) disease-associated coagulation factor XII or (ii) wild-type and disease-associated coagulation factor XII.

27. A method of gene therapy in a mammal, **characterized by** administering an effective amount of a nucleic acid molecule capable of expressing in the mammal:
(a) siRNA or shRNA, a ribozyme or an antisense nucleic acid molecule specifically hybridizing to a nucleic acid molecule encoding coagulation factor XII or regulating its expression;
(b) an aptamer or an inhibitory antibody or fragment or derivative thereof, specifically binding coagulation factor XII (poly)peptide;
(c) coagulation factor XII or a fragment thereof; or
(d) a serine protease inhibitor selected from group (i) consisting of wild-type and modified or engineered proteinaceous inhibitors of serine proteases including C1 esterase inhibitor, antithrombin III, α2-antiplasmin, α1-antitrypsin, ovalbumin serpins, and α2-macroglobulin, or selected from group (ii) of Kunitz-type inhibitors including bovine pancreatic trypsin inhibitor.

28. A non-human transgenic animal, comprising as a transgene:
(a) a gene encoding human disease-associated coagulation factor XII;
(b) (i) a gene encoding human disease-associated coagulation factor XII and (ii) a gene encoding human wild-type coagulation factor XII;
(c) a nucleic acid molecule causing an altered expression of human coagulation factor XII and a gene encoding human wild-type coagulation factor XII; and/or
(d) a species-specific coagulation factor XII gene which is specifically altered to contain a human disease-associated mutation.

29. The non-human transgenic animal of claim 28, additionally expressing siRNA or shRNA, a ribozyme or an antisense nucleic acid molecule specifically hybridizing to said human gene(s) of (a) 28(a), (b) 28(b)(i) or 28(b), (c) to the nucleic acid molecule of claim 28(c), or (d) to the altered species-specific gene of 28(d).

30. The non-human transgenic animal of claim 28 or 29, wherein the animal's native species-specific genes encoding coagulation factor XII are inactivated.

31. Use of the transgenic animal of any one of claims 28 to 30, for screening for compounds for use in the diagnosis, prevention and/or treatment of hereditary angioedema type III.

32. A kit for use in diagnosis of hereditary angioedema type III or a susceptibility or predisposition thereto, said kit comprising:
(a) at least one nucleic acid molecule capable of hybridizing under stringent conditions to a nucleic acid molecule encoding or regulating the expression of coagulation factor XII;
(b) an antibody or an aptamer specific for coagulation factor XII or a fragment thereof and/or a disease-associated mutant of these;
(c) a restriction enzyme capable of discriminating between wild-type and disease-associated mutant nucleic acid encoding or regulating the expression of coagulation factor XII; and/or
(d) a pair of primers complementary to nucleic acid regulating the expression of coagulation factor XII or encoding wild-type and/or disease-associated coagulation factor XII;
and optionally instructions for use.


SEQUENCE LISTING

<110>  Dewald, Georg

<120>  Hereditary Angioedema Type III

<130>  G 3152 EP

<140>  04 01 1790.5
<141>  2004-05-18

<160>  5

<170>  PatentIn version 3.1

<210>  1
<211>  2048
<212>  RNA
<213>  Homo sapiens

<220>
<221>  misc_feature
<223>  /note="translation product as given in GenBank accession no. NM_0
       00505.2"

<400>  1
cuauugaucu ggacuccugg auaggcagcu ggaccaacgg acggacgcca ugagggcucu      60
gcugcuccug ggguuccugc uggugagcuu ggagucaaca cuuucgauuc caccuuggga     120
agcccccaag gagcauaagu acaaagcuga agagcacaca gucguucuca cugucaccgg     180
ggagcccugc cacuuccccu uccaguacca ccggcagcug uaccacaaau guacccacaa     240
gggccggcca ggcccucagc ccuggugugc uaccaccccc aacuuugauc aggaccagcg     300
auggggauac uguuuggagc ccaagaaagu gaaagaccac ugcagcaaac acagccccug     360
ccagaaagga gggaccugug ugaacaugcc aagcggcccc cacugucucu guccacaaca     420
ccucacugga aaccacugcc agaaagagaa gugcuuugag ccucagcuuc uccgguuuuu     480
ccacaagaau gagauauggu auagaacuga gcaagcagcu guggccagau gccagugcaa     540

ggguccugau gcccacugcc agcggcuggc cagccaggcc ugccgcacca acccgugccu 600
ccaugggggu cgcugccuag agguggaggg ccaccgccug ugccacugcc cggugggcua 660
caccggaccc uucugcgacg uggacaccaa ggcaagcugc uaugauggcc gcgggcucag 720
cuaccgcggc cuggccagga ccacgcucuc gggugcgccc ugucagccgu gggccucgga 780
ggccaccuac cggaacguga cugccgagca agcgcggaac uggggacugg gcggccacgc 840
cuucugccgg aacccggaca acgacauccg cccguggugc uucgugcuga accgcgaccg 900
gcugagcugg gaguacugcg accuggcaca gugccagacc ccaacccagg cggcgccucc 960
gaccccggug uccccuaggc uucauguccc acucaugccc gcgcagccgg caccgccgaa 1020
gccucagccc acgacccgga ccccgccuca gucccagacc ccgggagccu ugccggcgaa 1080
gcgggagcag ccgccuuccc ugaccaggaa cggcccacug agcugcgggc agcggcuccg 1140
caagagucug ucuucgauga cccgcgucgu uggcgggcug guggcgcuac gcggggcgca 1200
ccccuacauc gccgcgcugu acuggggcca caguuucugc gccggcagcc ucaucgcccc 1260
cugcugggug cugacggccg cucacugccu gcaggaccgg cccgcacccg aggaucugac 1320
gguggugcuc ggccaggaac gccguaacca cagcugugag ccgugccaga cguuggccgu 1380
gcgcuccuac cgcuugcacg aggccuucuc gcccgucagc uaccagcacg accuggcucu 1440
guugcgccuu caggaggaug cggacggcag cugcgcgcuc cugucgccuu acguucagcc 1500
ggugugccug ccaagcggcg ccgcgcgacc cuccgagacc acgcucugcc agguggccgg 1560
cuggggccac caguucgagg gggcggagga auaugccagc uuccugcagg aggcgcaggu 1620
accguuccuc ucccuggagc gcugcucagc cccggacgug cacggauccu ccauccuccc 1680
cggcaugcuc ugcgcagggu uccucgaggg cggcaccgau gcgugccagg gugauuccgg 1740
aggcccgcug gugugugagg accaagcugc agagcgccgg cucacccugc aaggcaucau 1800
cagcugggga ucgggcugug gugaccgcaa caagccaggc gucuacaccg auguggccua 1860
cuaccuggcc uggauccggg agcacaccgu uuccugauug cucagggacu caucuuuccc 1920
uccuugguga uuccgcagug agagaguggc uggggcaugg aaggcaagau ugugucccau 1980
ucccccagug cggccagcuc cgcgccagga uggcgaggaa cucaauaaag ugcuuugaaa 2040
augcugag 2048

<210> 2
<211> 615
<212> PRT
<213> Homo sapiens

<220>
<221> MISC_FEATURE
<223> /note="translation product as given in GenBank accession no. NM_0

00505.2; the length of 615 residues corresponds to the sum of a s
ignal peptide of length 19 residues (-19 to -1) and a mature prot
ein of length 596 amino acid residues (1 to 596)"

<400> 2

Met Arg Ala Leu Leu Leu Leu Gly Phe Leu Leu Val Ser Leu Glu Ser
1               5                   10                  15

Thr Leu Ser Ile Pro Pro Trp Glu Ala Pro Lys Glu His Lys Tyr Lys
            20                  25                  30

Ala Glu Glu His Thr Val Val Leu Thr Val Thr Gly Glu Pro Cys His
        35                  40                  45

Phe Pro Phe Gln Tyr His Arg Gln Leu Tyr His Lys Cys Thr His Lys
    50                  55                  60

Gly Arg Pro Gly Pro Gln Pro Trp Cys Ala Thr Thr Pro Asn Phe Asp
65                  70                  75                  80

Gln Asp Gln Arg Trp Gly Tyr Cys Leu Glu Pro Lys Lys Val Lys Asp
                85                  90                  95

His Cys Ser Lys His Ser Pro Cys Gln Lys Gly Gly Thr Cys Val Asn
            100                 105                 110

Met Pro Ser Gly Pro His Cys Leu Cys Pro Gln His Leu Thr Gly Asn
        115                 120                 125

His Cys Gln Lys Glu Lys Cys Phe Glu Pro Gln Leu Leu Arg Phe Phe
    130                 135                 140

His Lys Asn Glu Ile Trp Tyr Arg Thr Glu Gln Ala Ala Val Ala Arg
145                 150                 155                 160

Cys Gln Cys Lys Gly Pro Asp Ala His Cys Gln Arg Leu Ala Ser Gln
                165                 170                 175

Ala Cys Arg Thr Asn Pro Cys Leu His Gly Gly Arg Cys Leu Glu Val
            180                 185                 190

Glu Gly His Arg Leu Cys His Cys Pro Val Gly Tyr Thr Gly Pro Phe
        195                 200                 205

Cys Asp Val Asp Thr Lys Ala Ser Cys Tyr Asp Gly Arg Gly Leu Ser
    210                 215                 220

Tyr Arg Gly Leu Ala Arg Thr Thr Leu Ser Gly Ala Pro Cys Gln Pro
225                 230                 235                 240

Trp Ala Ser Glu Ala Thr Tyr Arg Asn Val Thr Ala Glu Gln Ala Arg
                245                 250                 255

Asn Trp Gly Leu Gly Gly His Ala Phe Cys Arg Asn Pro Asp Asn Asp
            260                 265                 270

Ile Arg Pro Trp Cys Phe Val Leu Asn Arg Asp Arg Leu Ser Trp Glu
        275                 280                 285

Tyr Cys Asp Leu Ala Gln Cys Gln Thr Pro Thr Gln Ala Ala Pro Pro
    290                 295                 300

Thr Pro Val Ser Pro Arg Leu His Val Pro Leu Met Pro Ala Gln Pro
305                 310                 315                 320

Ala Pro Pro Lys Pro Gln Pro Thr Thr Arg Thr Pro Pro Gln Ser Gln
                325                 330                 335

Thr Pro Gly Ala Leu Pro Ala Lys Arg Glu Gln Pro Pro Ser Leu Thr
            340                 345                 350

Arg Asn Gly Pro Leu Ser Cys Gly Gln Arg Leu Arg Lys Ser Leu Ser
        355                 360                 365

Ser Met Thr Arg Val Val Gly Gly Leu Val Ala Leu Arg Gly Ala His
    370                 375                 380

Pro Tyr Ile Ala Ala Leu Tyr Trp Gly His Ser Phe Cys Ala Gly Ser
385                 390                 395                 400

Leu Ile Ala Pro Cys Trp Val Leu Thr Ala Ala His Cys Leu Gln Asp
                405                 410                 415

Arg Pro Ala Pro Glu Asp Leu Thr Val Val Leu Gly Gln Glu Arg Arg
            420                 425                 430

Asn His Ser Cys Glu Pro Cys Gln Thr Leu Ala Val Arg Ser Tyr Arg
        435                 440                 445

Leu His Glu Ala Phe Ser Pro Val Ser Tyr Gln His Asp Leu Ala Leu
    450                 455                 460

Leu Arg Leu Gln Glu Asp Ala Asp Gly Ser Cys Ala Leu Leu Ser Pro
465                 470                 475                 480

Tyr Val Gln Pro Val Cys Leu Pro Ser Gly Ala Ala Arg Pro Ser Glu
                485                 490                 495

Thr Thr Leu Cys Gln Val Ala Gly Trp Gly His Gln Phe Glu Gly Ala
            500                 505                 510

Glu Glu Tyr Ala Ser Phe Leu Gln Glu Ala Gln Val Pro Phe Leu Ser
        515                 520                 525

Leu Glu Arg Cys Ser Ala Pro Asp Val His Gly Ser Ser Ile Leu Pro
    530                 535                 540

Gly Met Leu Cys Ala Gly Phe Leu Glu Gly Gly Thr Asp Ala Cys Gln
545                 550                 555                 560

Gly Asp Ser Gly Gly Pro Leu Val Cys Glu Asp Gln Ala Ala Glu Arg
                565                 570                 575

Arg Leu Thr Leu Gln Gly Ile Ile Ser Trp Gly Ser Gly Cys Gly Asp
            580                 585                 590

Arg Asn Lys Pro Gly Val Tyr Thr Asp Val Ala Tyr Tyr Leu Ala Trp
        595                 600                 605

Ile Arg Glu His Thr Val Ser
    610                 615

<210>  3

<211>  10616

<212>  DNA

<213>  Homo sapiens


<220>

<221>  misc_feature

<223>  /note="translation product as given in GenBank accession no. AF53
       8691.1"


<400>  3
acatgctctg tgcttagtaa ccccagtgca acttttttgc tttcccaaaa gttctggcaa      60

aagtcccaag ctagcacttt aattggccta aattgtgtat atgcttatct ctgaaccaat     120

cactgtggat tagagatgtc atgctctgat tgaccagacc taggccacat ctctagccct     180

agctctgagg gtagagttgg cagcactaga gcccatggaa gaagtaagag aggagtcgtt     240

gctaaaggaa aaatcaaagt gtcattaccg aaccaggaca gatgctgggc agcacatgtg     300

caccccgtct tcttctcatg ttccagctgc acatcttagt gccccttggt ttagcacttt     360

tctcattaaa tcatttgctt tcttgcctca cttcctgtgg ttggtagaat gctaagatgg     420

ccccaagatc tctacccctg gtgtttgcac acctcccagt tattctgtca aacatgaatg     480

tagatgcttc tgtgaaagaa ttttgcacat gtaatttaag tcccaaattg tttgacctta     540

aaataaggag aatggcaggg ccaggcatgg tggctcatac ctgtaatccc agcactttgg     600

gaggccaagg cgggcagatc acgaggtcag gagatcgaga ccatcctggc taacacagtg 660
aaaccccatc tctactaaaa atacaaaaaa ttagctgggc gtggtggcgg gtgcctgtat 720
tcccagctac ccaggaggct gaggcaggag aatggcgtga acccgggagg cgtagcttgc 780
agtgagccaa gatcgtgcca ctgcactcca gcctgggtga cagagccaga ctctgtctca 840
aaaaaaaaaa aaaaaaggag aatggctttg gtgggcctga cctagtcagg tgagttctta 900
aaaggcgaca catggcccgg tgcagtggct caggcctgta atcccagcac tttgggaggc 960
cgaggcgggt ggatcacgag gtcaggagat cgagaccatc ctggctaaca tggtgaaacc 1020
ccgtctctac taaaaagaca aaaaattagc tgggcgtggt ggtgggctcc tgtagtccca 1080
gctactcggg aggctgaggc aggagaatgg cgtgaacccg ggaggcggag cttgcagtga 1140
gcggagattg cgccactgca ctccagcctg ggcgacagag cgagactccg tctcaaaaaa 1200
aaaaaaaaaa aaaaagaaaa ttaaaagtgg gtattgttgt aagatgctga gtttatggta 1260
gtttgttaca tgacaataga aaatgaacac acttcacagt ggactccaag atccccatga 1320
tctttgatct ccttaacctc ctgatctcca caggacccag agcataagaa tgtcccttct 1380
tctgcttcca gtcccactat ctagaaaaga gaggaggagc ccagctcttc atttcacccc 1440
cacccacaaa ctcccaactt tccggccctc aaggggtgac caaggaagtt gctccacttg 1500
gctttccaca aacagcctgt gccccaccag gctcaggagg gcagcttgac caatctctat 1560
ttccaagacc tttggccagt cctattgatc tggactcctg gataggcagc tggaccaacg 1620
gacggacgcc atgagggctc tgctgctcct ggggttcctg ctggtgagct tggagtcaac 1680
actttcggtg agtgctgtgg gaaccaggat tgtcccagga ttgttctggg gggtcgctat 1740
cacagccatg agccatggcc tctgctcatg acctgtgggt ccaggtgact aggaggccta 1800
tgtggaaagg tgaggccagc ccggaaggcc caggcagagg agacagacaa ccagactggg 1860
tggatacaag ggcacagcct gcatttctgg gggagatggg ccttaagaag acaacggggg 1920
gaggtagaaa gggtttgggt cttgggaaga aatctctgca tttctgggct gtgagaggaa 1980
gctgcagact agcaacagat cggtggcagg ctatgactta tagtcagttc cctgccttct 2040
tctctccctt gtagattcca ccttgggaag cccccaagga gcataagtac aaagctgaag 2100
agcacacagt cggtaagtgg cctggctcct cctcccggga acccttgggt ggggatgtgt 2160
atggtgcagt gtgtgcagtc tcagggcagt ctagtctagt gcctacctgg tgctaggtct 2220
tatgcccatg ggcactagag tgatcgtgag ctgtgtgatc cttgagggca gggtatgggc 2280
tgtgtctaag tgcccacgag cctggctcgg agcaggtgct tgagatatgt gctgctggcg 2340
ccatcacacc tgggctcctg ccagccttcc tcagtttccc cagcttctcc ccttcttttc 2400
ctttccccag tacgtctcat gggcatcatt catgccacac agaggccagg gccttcaatg 2460
ggcaaggaag gatcaagagc ttgtctctgg catctgaatg cctctgaagc ccagctttat 2520
cacttatgag ctgggtgact ctgggcgagg gatttgagtt ctccaagctt caatttcccc 2580
ttctgtgaaa ccaggttgat aacagtaaac ctcttagggt tgttgagaag ggaaacccat 2640

gtgaggtatt cagcccatca cctggtgcat ggaaatgctt tacaaatatt agcttttatt 2700
atgaaactac cttttagatg aagggtacct gccatttccc ccttcctcaa gctctgccat 2760
agctccccat tgctttcatt cttccagaca ctaaattacc tacatgccag gcatggtggc 2820
tcatgcctgt aatcccagca ctttgggagg ccaaggtcgg tggatcatga ggtcaggagt 2880
tcgagaccag cctggccaac atggtgaaat gctgtctcta ctaaaagtac aaaaattagc 2940
caggcatggt ggcatgcgcc agtagtccca gctactcggg aggctgaggc agaagaattg 3000
cttgaacctg ggaggtgaag gttgcagtga acgaagatca caccattgca ctccagcttg 3060
ggcaacacag caagactccg tctcaaaaaa aaaaaaaaaa tttacctaga gtgtggcaca 3120
tagcagggcc tgtgaaccag atggacctta ccctggtggg cctgacttgg tggggttgag 3180
tctctaagca tggcgttgag gcccagcaca ttccaaccct ggactccctc agcctcctct 3240
cttcacccca cacccaaaag tttctcctct ctcttgcctt acccaaactt ggtgccctat 3300
ccttgcctaa tcccctgcct aaggtccccc tcctctctgt ccgtccatcc catctgcatc 3360
tttttttttt tttgagatgg agtctcgctc tgtcccctag gctagagtgc aatggcgcga 3420
tcttggctca ttgcaacctc cgcctcctgg gttcaagcga ttctctgcct cagcctcccg 3480
agttgctggg attacaggca cacaacttca tgctcagcta atttttgtat tttttagtag 3540
agacagggtt tcaccatgtt ggccaggctg gtctcgaact cctgccctca ggtggtccgc 3600
ccaccttagc ctcccaaagt gctgggatta caggcgtgag ccaccgcgcc tggcccccat 3660
ttgcatctta aaggtccatc tcagatccat ttccatttac tgtcctagtt ctggtttggt 3720
ccttggcaag tgcactttgc cttgaacaaa atagtggcaa aagcttattg agcaggtact 3780
ttgtgccaga cactgctcag catttcatgg cattatctca tgaagcccca cgacaattcc 3840
tctgaagaag acacaggcaa ttctcattat tcgcgatggt tatgttctat aaaatcacag 3900
tgaacattga actagcaaac agtattaggt tcctgtgagc ctctggtcac aacattttca 3960
tcaaccaaca gcatataatc tggttttatg tatgattctg tttaaagaca ttttatttag 4020
tatatgtgtt gctgattcat caatgctaag ctgatggcac tatagcacac acctgaatca 4080
agtgtctaac acacgctttc tccctaaggt agccttcttg tgcttaggaa ctacacagct 4140
cttcagcagg aggctcagag gccatttcca aaagccaaat ccccagcaaa agcacaaagt 4200
gtgaaaaacg ttgcactaag tagactgaga aggacactca ttcaatagga gagctgaaac 4260
aagcagcagc agcgtgacgc cttgttgaac cttaactggg aatgtgcaaa tttttcactg 4320
ctctgtgcat gcccacaaat ggccatgaaa acatttcaag tattgacttg ggagttacaa 4380
ataaaattca gcaagtaggc acattctcaa tgtagaacca gagaagaatg aggatcaact 4440
gtactattat tactgccgtt ttacagataa ggaaaccaag gctcagatca gagtggttaa 4500
cagtgacttc aacattcaac aagtattatt aagtgcctac tttgtggcaa gtgctcttcc 4560
tggccttggg actgaagact tacccaaggt cacacagcta gcaggttgtg gagtcaggag 4620
tctactccag ctatctgact cctgaaccca agtttttttt tttttcttta agatggagtc 4680

tcactctgtc acccaggctg gagtgcagtg gcgcgatctc ggctcactgc aagctccgcc 4740
tcccgggttc acaccattct cctgcctcgg cctcccgagt agctgggact acaggcacct 4800
gccaccaccc ccagctaatt tttttgtatt tttagtagag agggggtttc actgtattag 4860
ccaggatggt cttgatctcc tgacctcgtg atctgcccgc cttggcctcc caaagtgctg 4920
ggattacagg cttgagccac cgcgcccggc cctgaaccca acttttagag cagaaagtgt 4980
tttcaatgca cagcgacctt tttgagggtc tgtccttttc ctgaccagac cctgagggac 5040
agtgcctgag cagttgagta caggggaagt cctcagagag tgtgttgtcc ctgcagttct 5100
cactgtcacc ggggagccct gccacttccc cttccagtac caccggcagc tgtaccacaa 5160
atgtacccac aagggccggc caggccctca gccctggtaa gactacgcag aggagttgga 5220
gcaggggcct gggagacatg taccctgcct gtccttctgt ccaaggaact ctgcttggag 5280
agaggggact gtgatagggc agggtgggcc aggcccctgg gtagagcagg gaagccttgt 5340
ctctttctac aggtgtgcta ccacccccaa ctttgatcag gaccagcgat ggggatactg 5400
tttggagccc aagaaagtga aaggtgctac acacagcctc tggggtggcc tggggctctc 5460
tcctcccgcc tcattactct cctggtatca ccagacccca cacacctggg attctggacc 5520
cagccccttc tctccctcca caataccctt tggaagtcca gagggagagt tctgggaagg 5580
agtggtccca ttttgcaggt gggtaaacca agcttggaaa cttggagtag caaggtcaca 5640
aggcaagtag gttcaagaag ggccttggcc cccagctgtg tgactcagct ccctgctctt 5700
ccttccacca tgtccatctc tcagaccact gcagcaaaca cagcccctgc cagaaaggag 5760
ggacctgtgt gaacatgcca agcggccccc actgtctctg tccacaacac ctcactggaa 5820
accactgcca gaaaggtgag gagatgtgga ggacctgggc ggggtgctgg gggacagggg 5880
caaccctggg cctacagaat aggttgctgg atactcggag acttggcatg gtcctagact 5940
ctcctgagac cactatccct ctttgtcccc agagaagtgc tttgagcctc agcttctccg 6000
gtttttccac aagaatgaga tatggtatag aactgagcaa gcagctgtgg ccagatgcca 6060
gtgcaagggt cctgatgccc actgccagcg gctggccagc cagggtgagc agatggttgg 6120
gaacgggcca gggaggagcg tcaggaagac aggctggcag gaggccgggt ggtgtgccgg 6180
gaaggagagc tctctggggg ggtctttagg cccaggggtg gctcactgcg ttccctcccc 6240
aagcctgccg caccaacccg tgcctccatg ggggtcgctg cctagaggtg gagggccacc 6300
gcctgtgcca ctgcccggtg ggctacaccg gacccttctg cgacgtgggt gagtgagggt 6360
ctggggcaag cagaaggcca gcccccaggt gggacgggct tgccaggaag gaggagggag 6420
agtgcggaaa gcagatgaga gggaggcagg agagcccagc cttggctgcc cagggagccc 6480
cctttctcct cagacaccaa ggcaagctgc tatgatggcc gcgggctcag ctaccgcggc 6540
ctggccagga ccacgctctc gggtgcgccc tgtcagccgt gggcctcgga ggccacctac 6600
cggaacgtga ctgccgagca agcgcggaac tggggactgg gcggccacgc cttctgccgg 6660
tgcgccgcgt ggggctgggt gacccctccg ccccagggct ccgggctccc ggcgctctaa 6720

cggcgccccg tcgtgtggct acaggaaccc ggacaacgac atccgcccgt ggtgcttcgt 6780
gctgaaccgc gaccggctga gctgggagta ctgcgacctg gcacagtgcc agaccccaac 6840
ccaggcggcg cctccgaccc cggtgtcccc taggcttcat gtcccactca tgcccgcgca 6900
gccggcaccg ccgaagcctc agcccacgac ccggaccccg cctcagtccc agaccccggg 6960
aggttaggaa gtgggggggg gaaggaggag ccgagagggc gccgggcgag ctagattccg 7020
gccagccggc cgcgggctcc ccgtcctcag cccctgctcc tccacagcct tgccggcgaa 7080
gcgggagcag ccgccttccc tgaccaggaa cggcccactg agctgcgggc agcggctccg 7140
caagagtctg tcttcgatga cccgcgtcgt tggcgggctg gtggcgctac gcggggcgca 7200
cccctacatc gccgcgctgt actggggcca cagtttctgc gccggcagcc tcatcgcccc 7260
ctgctgggtg ctgacggccg ctcactgcct gcaggaccgg cgagtacccg cccgcccaga 7320
gccgccccag gggccgcggc tcctccgtct cccagcgcag cttccacgct gcacccgaac 7380
ccgtgcccta ccttctcccg ccccaccctt ctttccacgc ccctccggag ctcccgggga 7440
ggaagctgga acacgggatt ggggttcggg agcagggggc ttccccagaa cgcttgtggc 7500
caggtctgag agcgctgcct ctcccctacc ctccccgcag gcccgcaccc gaggatctga 7560
cggtggtgct cggccaggaa cgccgtaacc acagctgtga gccgtgccag acgttggccg 7620
tgcgctccta ccgcttgcac gaggccttct cgcccgtcag ctaccagcac gacctgggtg 7680
cgtgggggcg ccccgcgggg acgggaagag agcttggggc cccggcgtcc ccgcctcacg 7740
ctcctctccg cccgggttag ctctgttgcg ccttcaggag gatgcggacg gcagctgcgc 7800
gctcctgtcg ccttacgttc agccggtgtg cctgccaagc ggcgccgcgc gaccctccga 7860
gaccacgctc tgccaggtgg ccggctgggg ccaccagttc gagggtaggc acaactgcta 7920
ggggcagggg taggggagga gacctttgat cactgggtta ggcggaagaa gcccgcgact 7980
ttggtatcgt tccgggtgcc tacagaatgg gtggcgctga cctgatgggt tgtgagaatg 8040
tgtaggtgaa tcccaggtag aatcccaggg cctgggattc actgctggga tccccaaatc 8100
tcctggggat acagggagaa tcgaacttgc tcttggttcc ctctgggcgc cgggctgcaa 8160
aggccaacta ggacgctggc cccgcgctcc gggctagtgt gggagccagg ttctgcgact 8220
ctggatgggt ggtgggggag gggtttctgt ttccgctccg cccattcaaa tcctggcttt 8280
tctctggacc tcagcctcct tgcctatgaa attgaattaa tggcacctcc tccccttcgg 8340
gcttgctgcg agagaggaag ggcatgagtg ggtttacaag cgcctggagc agctttgtcc 8400
atcgtccggg cggcaagcgt tgtcagatgg ggtgtgaaga aggcgctctg tgttcgcagg 8460
ggcggaggaa tatgccagct tcctgcagga ggcgcaggta ccgttcctct ccctggagcg 8520
ctgctcagcc ccggacgtgc acggatcctc catcctcccc ggcatgctct gcgcagggtt 8580
cctcgagggc ggcaccgatg cgtgccaggt gagctcttag cccggttggc gcccttcccc 8640
gaggccgtca ggcacaaatc tcaggtccac agcgctgagc tgcgtgtttc cgacccaggg 8700
tgattccgga ggcccgctgg tgtgtgagga ccaagctgca gagcgccggc tcaccctgca 8760

aggcatcatc agctggggat cgggctgtgg tgaccgcaac aagccaggcg tctacaccga 8820
tgtggcctac tacctggcct ggatccggga gcacaccgtt tcctgattgc tcagggactc 8880
atctttccct ccttggtgat tccgcagtga gagagtggct ggggcatgga aggcaagatt 8940
gtgtcccatt cccccagtgc ggccagctcc gcgccaggat ggcgcaggaa ctcaataaag 9000
tgctttgaaa atgctgagaa ggaaagctct tttcttcatg ggtcccgccg ggaaatgcca 9060
agacagaaaa gcgattcaca gcttctccac agctctcaga gaacaaggtc tatgagatct 9120
taacgtgcaa aatctagatg ccagcccagc taatgtttac tgagcctagg atactgtata 9180
ccaagccctg tgcaaggaga agctgcatgt tattccttat gagaaactaa cattttgttt 9240
acagagcagt agttctcaga ccatacatta agatcacttg gggagcgttt tgagccaatc 9300
tatgcccaag ttccacctca gaccaattaa atcagtatgt ctagggatgg ggcatgggta 9360
gtggtatatt tgtaaaactc cccagataat tccatgtaca gccaaggttg agaatcgtgg 9420
ttagaaatac ttagcattgg ccgggcgcgg tggctcacgc ctgtaatcct agcactttaa 9480
gaggccaagg caggtggatt gctcaggagt tcgaaaccag cctgggcaac acgatgaaac 9540
cccgtctcta ctaaaataca agaaattagc cgggcacggc ggcgtgcgcc tgtagtccca 9600
gctactcagg aggctgaggc aggagaatca cttgaaccgg caggaaggaa ggaaggaagg 9660
aacagaggga gggaaagaga gagacagaaa gaaaagaaaa aagaaaatag aaaaaaagag 9720
cattgactgt ggcgtggacc ctaagggctg ggtgacatat cgttgtcccc accccaacac 9780
gcactagtgt agtgggtctg agagtccctt ggctagcagt accatcacca gggaacttgt 9840
tacacataac aaattctcgg gctacacttt atactgctga acagaaagtc tggggtgggg 9900
cccagcaatc tgtttaacag ccttgcgggg gattctgatg ttctctcatg cttaagaacc 9960
acaatctggg ggttgaatgg ttggttccct tacaagtgaa ggtctggctg tccagacaca 10020
acatcctttt ttcacaaaac cagcttttta aaattaaaaa tagattggcc agatgcggtg 10080
gttcacgcct gtaatctcgt cactttgaga ggctgaggcg ggaggattgt ttgagctcaa 10140
gacttcctga cccgcctggg caacatagtg agacctcatc tcaaaaaaat tttttttaat 10200
taaaatttgt ttttgctttt ttagagacgg ggcctcgctc tgtggctcag gctggcgtgc 10260
agcgacacga tcctataata gtttactata atctcgctac tgagttcaag cgatccgccc 10320
gcctcggcct cccaaagcgc tgggattaca ggagtgagcc gctgcgctct gccaaaccca 10380
tcctacagga taaccttaga actgcgacag cactaaacgc ccacgcccca cgtgccccag 10440
cctgggtggt cgctccggga cggcgccttg tgtgacgtca cagccccgcc cagcctgcct 10500
cacagcgccg caggccttcc ccgcgtggcg cctctatatt tccccgagag gtgcgaggcg 10560
gctgggcgca ctcggagcgc gatgggcgac tggaaggtct acatcagtgc agtgct 10616

<210> 4
<211> 2420
<212> DNA

<213>  Homo sapiens

<220>

<221>  misc_feature

<223>  /note="Extended upstream genomic sequence (extending the upstream
       sequence given in AF538691.1), corresponding to (taken from) UCS
       C Genome Browser/July 2003 human reference sequence/ >chr5:176819
       111-176821530 (reverse complement)"

<400>  4
   agccgggtgt ggtggctcac gcctgtaatt ccagcacttc gggaggccga ggtgggcaga      60
   tcatgaggtc aggagttcga gaccaacctg gccaacatgg tgaagccctg gtctctacta     120
   aggagacaaa aaattagccg ggcatggtgg cgtgggcctg taatggcagc tacttgggag     180
   gctgaggcag gagaatcact tgaacctggg aggtggaggt tgtagtgagc cgagatcgca     240
   ccatggcagt ccagtttggg cgacagcgcg agactccatc tcaaataaat aactaaataa     300
   aaataataat aaagtaccct ttcaccacca cagctgggga ggggtgatca caaaatgaaa     360
   ctgggagagg aagaggaagg gagatggatg ttgaacatcc aaatgaccat taatgtccac     420
   ctgacacctc atctcacact ccctacaagg caccttatgc tcaccacatt ccccatttga     480
   cacgctgcat tcaggcttct gtgcagttgt attactcact ctgcctgcaa acccctactt     540
   gtggcgaaga cctgttcaaa aggccccttc catgtagagg aacagctgcc ttctccccaa     600
   gaggaaggag ccaatgtggg agtggcctta gggccagggt ctatactcag gagcaatcaa     660
   gaactgacag agccaggcat ggtggcatgc acctgtaatc ccagctattc aggaggatga     720
   agcgagagga tcacttgcgc ccagaagttc caggctagcc tgggcaactt aataagaccg     780
   agtctctttg aaacaacaaa aaaggtgaca aaggtagttt cgctttaatg actcgtcaat     840
   atatctaata gtttcacttt catcatggag ttaacagctg tcacgtcaat gactttgaaa     900
   ccagttacct ttgaaaccag tggttggaaa ggtgcccctc ctctcctgcc ctcctctttc     960
   tgtgtttctg ttccaagaag tcttccacag cctgcctctc ttccagcaga attaataaat    1020
   tgctacactc agtcttcatt tcccataaat tactttgtat aacttaggac tttgtataac    1080
   tgaaagtgac agaagtccac tcaaattggt tttattggct catataactg agaagtctat    1140
   tttttttttt tttggagata cagtctcagt ctgcttccca gactggagtg cagtggcaca    1200
   atcacggctc acagcatttt ggacctctgg gctcaagcaa tcctcctgcc tcagactcac    1260
   aagtagccgg aactacaggc acgcgccacc atgcctggct aatgttaaat ttttttggag    1320
   agatgggatc tcactatgtt gcctacatca gtctggaact cctgggctca agtgatcctc    1380
   aagtgatccc cagcctccca gagtgttggg attacaggcg tgagccactg tgcctagcca    1440
   taactgacca agtcttaagg atacttctag ttttaggatg taggctctca aagtcatgag    1500
   aaatcaatcc atcagtacta gttctcaaat gtcgtgtaac aaatcaccct aacttgtggc    1560
   ttaaagcaac aacatttaat gatgatttat cacagtttct gatggtcagt tttgccttga 1620
   ggtctttcat aagattgcaa gccaaatgtc agcgagggct gcagtatcca cttccaaggc 1680
   agcttattca tgtggctgtc caattggcac tggctgtcag cagggtgctt tcctttctcc 1740
   ctacatgggc ttctctgcag ggctgcatgt gtgtcctcac aacacagtgg ctggcttcct 1800
   ccagagtgag caacctaaga gaccgaggca gaaactgcaa tgtcttttat gacctgggct 1860
   tggaagtcca acaccctcac ttctgccata tttttttttt tttgagacag tccctgtcgc 1920
   ccaggctgga gtgcaatggc acaatctctg ctcactacaa cctccacttc ctgggttcaa 1980
   gcgattctcc tgcctcagcc tcacgggtag ctgggattac aggcacacgc cactacgccc 2040
   ggctaatttt tgtatttttc atagagatgg ggtttggcca acacgttggc caggccggtc 2100
   ttgaactcct gacctagagt gatctgcctg ccttggcatc ccaaagtgct gggattacag 2160
   gtgtgagcca ccacacccag ccacttctgt catattctga tggacacaca gaacagccct 2220
   aactcaatgt gggaagacac catacaaggg catgaatcct aggtgctgag gatcaatggg 2280
   ggctgtcttt gaggctgtct accacagcat ctttccatcc ttcctgccct gtttgctttg 2340
   cttttcctat gtgtaggctt cattctcaaa caggccctcc ctagagagtg acaaaggtga 2400
   tcatcaatgt gttcagaccc 2420

<210> 5

<211> 1402

<212> DNA

<213> Homo sapiens

<220>

<221> misc_feature

<223> /note="Extended downstream genomic sequence (extending the downst
ream sequence given in AF538691.1), corresponding to (taken from)
UCSC Genome Browser/July 2003 human reference sequence/ >chr5:17
6807094-176808495 (reverse complement)"

<400> 5
   gcgggaccag cgcatcgacg acgtggccat cgtgggccat gcggacaaca gctgcgtgtg 60
   ggcttcgcgg cccgggggcc tgctggcggc catctcgccg caggaggtgg gcgtgctcac 120
   ggggccggac aggcacacct tcctgcaggc gggcctgagc gtgggggccc gccgctgctg 180
   cgtcatccgc gaccacctgc tggccgaggg tgacggcgtg ctggacgcac gcaccaaggg 240
   gctggacgcg cgcgccgtgt gcgtgggccg tgcgccgcgc gcgctcctgg tgctaatggg 300
   ccgacgcggc gtacatgggg gcatcctcaa caagacggtg cacgaactca tacgcgggct 360
   gcgcatgcag ggcgcctagc cggccagcca ggccgcccac tggtagcgcg ggccaaataa 420
   actgtgacct gggcgcggct ggctcctcct ccacttgcgc ggtgggggga gttgtaaata 480
   aggaaactgg tctttgcaag acggttacct ggtggagccg ggattttgag tctagaggct 540
   gccaggcccc tgtgccctac accctgctct cccatggacg ccttgcagag gctcctggcc 600
   tgactgctgc tccttggcgc gttcccaggg tcctagggac tccgcagctg aggaagagtc 660
   caagggtggg ggcttctcaa agtctgtttc agccttagcg tcctttctca gagatattcc 720
   cacacattag gcaggacaag taaagggagc cccctcccca tcccgcgaac acctctcccc 780
   atcagggtgt caggctggag gccaatttgc tcctcccccc tccactcata cctcaagcac 840
   tagcaagttg tgagtgggtg acaggatggg cttggtggct tgtaaagcag ttctggggct 900
   cacaggcctc tgcatctctg cccacattcc tccaagggga gcctactgag agggctcatg 960
   tccaagacca tcgcaattgg gtttgagacc ttacatcctg ccttccccag gccttcgaaa 1020
   aggccccgca ggagtccctg gactagaggg aggaactctg gcatccctac ccgggagtct 1080
   cactctgcag gcctcagttt cagggtgacc tatggaggag gggaattgaa aaagcttggg 1140
   taagtttgag gcctggttta ttgcccaaag atagtggaca aaagtgggag ggagggtgtc 1200
   tggtccctgc cctccatgtg ctggggccca gggcatggcc tctcttgccc acccccaccc 1260
   ttcccgtccc ctcccccagc ggccctgatg gcagacccca cctgtcactt attctggagc 1320
   cctgatctta tcccagcagg aaggagtgat gtgtggctga ggtgggtgaa tttagagggc 1380
   agagggagac cagaggaagt tc 1402

SEQUENCE LISTING

<110> Dewald, Georg

<120> Hereditary Angioedema Type III

<130> G 3152 EP

<140> 04 01 1790.5
<141> 2004-05-18

<160> 5

<170> PatentIn version 3.1

<210> 1
<211> 2048
<212> RNA
<213> Homo sapiens (Comment: corresponding to (taken from) GenBank accession no. NM_000505.2)

<400> 1
cuauugaucu ggacuccugg auaggcagcu ggaccaacgg acggacgcca ugagggcucu 60
gcugcuccug ggguuccugc uggugagcuu ggagucaaca cuuucgauuc caccuuggga 120
agcccccaag gagcauaagu acaaagcuga agagcacaca gucguucuca cugucaccgg 180
ggagcccugc cacuuccccu uccaguacca ccggcagcug uaccacaaau guacccacaa 240
gggccggcca ggcccucagc ccuggugugc uaccaccccc aacuuugauc aggaccagcg 300
auggggauac uguuuggagc ccaagaaagu gaaagaccac ugcagcaaac acagccccug 360
ccagaaagga gggaccugug ugaacaugcc aagcggcccc cacugucucu guccacaaca 420
ccucacugga aaccacugcc agaaagagaa gugcuuugag ccucagcuuc uccgguuuuu 480
ccacaagaau gagauauggu auagaacuga gcaagcagcu guggccagau gccagugcaa 540
ggguccugau gcccacugcc agcggcuggc cagccaggcc ugccgcacca acccgugccu 600
ccaugggggu cgcugccuag agguggaggg ccaccgccug ugccacugcc cgguggggua 660
caccggaccc uucugcgacg uggacaccaa ggcaagcugc uaugauggcc gcgggcucag 720
cuaccgcggc cuggccagga ccacgcucuc gggugcgccc ugucagccgu gggccucgga 780

ggccaccuac cggaacguga cugccgagca agcgcggaac uggggacugg gcggccacgc 840
cuucugccgg aacccggaca acgacauccg cccguggugc uucgugcuga accgcgaccg 900
gcugagcugg gaguacugcg accuggcaca gugccagacc ccaacccagg cggcgccucc 960
gaccccggug uccccuaggc uucauguccc acucaugccc gcgcagccgg caccgccgaa 1020
gccucagccc acgacccgga ccccgccuca gucccagacc ccgggagccu ugccggcgaa 1080
gcgggagcag ccgccuuccc ugaccaggaa cggcccacug agcugcgggc agcggcuccg 1140
caagagucug ucuucgauga cccgcgucgu uggcgggcug guggcgcuac gcggggcgca 1200
ccccuacauc gccgcgcugu acuggggcca caguuucugc gccggcagcc ucaucgcccc 1260
cugcugggug cugacggccg cucacugccu gcaggaccgg cccgcacccg aggaucugac 1320
gguggugcuc ggccaggaac gccguaacca cagcugugag ccgugccaga cguuggccgu 1380
gcgcuccuac cgcuugcacg aggccuucuc gcccgucagc uaccagcacg accuggcucu 1440
guugcgccuu caggaggaug cggacggcag cugcgcgcuc cugucgccuu acguucagcc 1500
ggugugccug ccaagcggcg ccgcgcgacc cuccgagacc acgcucugcc agguggccgg 1560
cuggggccac caguucgagg gggcggagga auaugccagc uuccugcagg aggcgcaggu 1620
accguuccuc ucccuggagc gcugcucagc cccggacgug cacggauccu ccauccuccc 1680
cggcaugcuc ugcgcagggu uccucgaggg cggcaccgau gcgugccagg gugauuccgg 1740
aggcccgcug gugugugagg accaagcugc agagcgccgg cucacccugc aaggcaucau 1800
cagcugggga ucgggcugug gugaccgcaa caagccaggc gucuacaccg auguggccua 1860
cuaccuggcc uggauccggg agcacaccgu uuccugauug cucagggacu caucuuuccc 1920
uccuugguga uuccgcagug agagaguggc uggggcaugg aaggcaagau ugugucccau 1980
ucccccagug cggccagcuc cgcgccagga uggcgaggaa cucaauaaag ugcuuugaaa 2040
augcugag 2048

<210> 2

<211> 615

<212> PRT

<213> Homo sapiens (Comment: translation product as given in GenBank accession no. NM_000505.2; the length of 615 residues corresponds to the sum of a signal peptide of length 19 residues (-19 to -1) and a mature protein of length 596 amino acid residues (1 to 596))

<400> 2

Met Arg Ala Leu Leu Leu Leu Gly Phe Leu Leu Val Ser Leu Glu Ser
1               5                   10                  15

Thr Leu Ser Ile Pro Pro Trp Glu Ala Pro Lys Glu His Lys Tyr Lys
            20                  25                  30

Ala Glu Glu His Thr Val Val Leu Thr Val Thr Gly Glu Pro Cys His
        35                  40                  45

Phe Pro Phe Gln Tyr His Arg Gln Leu Tyr His Lys Cys Thr His Lys
    50                  55                  60

Gly Arg Pro Gly Pro Gln Pro Trp Cys Ala Thr Thr Pro Asn Phe Asp
65                  70                  75                  80

Gln Asp Gln Arg Trp Gly Tyr Cys Leu Glu Pro Lys Lys Val Lys Asp
                85                  90                  95

His Cys Ser Lys His Ser Pro Cys Gln Lys Gly Gly Thr Cys Val Asn
            100                 105                 110

Met Pro Ser Gly Pro His Cys Leu Cys Pro Gln His Leu Thr Gly Asn
        115                 120                 125

His Cys Gln Lys Glu Lys Cys Phe Glu Pro Gln Leu Leu Arg Phe Phe
    130                 135                 140

His Lys Asn Glu Ile Trp Tyr Arg Thr Glu Gln Ala Ala Val Ala Arg
145                 150                 155                 160

Cys Gln Cys Lys Gly Pro Asp Ala His Cys Gln Arg Leu Ala Ser Gln
                165                 170                 175

Ala Cys Arg Thr Asn Pro Cys Leu His Gly Gly Arg Cys Leu Glu Val
            180                 185                 190

Glu Gly His Arg Leu Cys His Cys Pro Val Gly Tyr Thr Gly Pro Phe
        195                 200                 205

Cys Asp Val Asp Thr Lys Ala Ser Cys Tyr Asp Gly Arg Gly Leu Ser
    210                 215                 220

Tyr Arg Gly Leu Ala Arg Thr Thr Leu Ser Gly Ala Pro Cys Gln Pro
225                 230                 235                 240

Trp Ala Ser Glu Ala Thr Tyr Arg Asn Val Thr Ala Glu Gln Ala Arg
                245                 250                 255

Asn Trp Gly Leu Gly Gly His Ala Phe Cys Arg Asn Pro Asp Asn Asp
            260                 265                 270

Ile Arg Pro Trp Cys Phe Val Leu Asn Arg Asp Arg Leu Ser Trp Glu
        275                 280                 285

Tyr Cys Asp Leu Ala Gln Cys Gln Thr Pro Thr Gln Ala Ala Pro Pro
    290                 295                 300

Thr Pro Val Ser Pro Arg Leu His Val Pro Leu Met Pro Ala Gln Pro
305                 310                 315                 320

Ala Pro Pro Lys Pro Gln Pro Thr Thr Arg Thr Pro Pro Gln Ser Gln
                325                 330                 335

Thr Pro Gly Ala Leu Pro Ala Lys Arg Glu Gln Pro Pro Ser Leu Thr
            340                 345                 350

Arg Asn Gly Pro Leu Ser Cys Gly Gln Arg Leu Arg Lys Ser Leu Ser
        355                 360                 365

Ser Met Thr Arg Val Val Gly Gly Leu Val Ala Leu Arg Gly Ala His
    370                 375                 380

Pro Tyr Ile Ala Ala Leu Tyr Trp Gly His Ser Phe Cys Ala Gly Ser
385                 390                 395                 400

Leu Ile Ala Pro Cys Trp Val Leu Thr Ala Ala His Cys Leu Gln Asp
                405                 410                 415

Arg Pro Ala Pro Glu Asp Leu Thr Val Val Leu Gly Gln Glu Arg Arg
            420                 425                 430

Asn His Ser Cys Glu Pro Cys Gln Thr Leu Ala Val Arg Ser Tyr Arg
        435                 440                 445

Leu His Glu Ala Phe Ser Pro Val Ser Tyr Gln His Asp Leu Ala Leu
    450                 455                 460

Leu Arg Leu Gln Glu Asp Ala Asp Gly Ser Cys Ala Leu Leu Ser Pro
465                 470                 475                 480

Tyr Val Gln Pro Val Cys Leu Pro Ser Gly Ala Ala Arg Pro Ser Glu
                485                 490                 495

Thr Thr Leu Cys Gln Val Ala Gly Trp Gly His Gln Phe Glu Gly Ala
            500                 505                 510

Glu Glu Tyr Ala Ser Phe Leu Gln Glu Ala Gln Val Pro Phe Leu Ser
        515                 520                 525

Leu Glu Arg Cys Ser Ala Pro Asp Val His Gly Ser Ser Ile Leu Pro
    530                 535                 540

Gly Met Leu Cys Ala Gly Phe Leu Glu Gly Gly Thr Asp Ala Cys Gln
545                 550                 555                 560

Gly Asp Ser Gly Gly Pro Leu Val Cys Glu Asp Gln Ala Ala Glu Arg
                565                 570                 575

Arg Leu Thr Leu Gln Gly Ile Ile Ser Trp Gly Ser Gly Cys Gly Asp
            580                 585                 590

Arg Asn Lys Pro Gly Val Tyr Thr Asp Val Ala Tyr Tyr Leu Ala Trp
        595                 600                 605

Ile Arg Glu His Thr Val Ser
    610                 615

<210>  3

<211>  10616

<212>  DNA

<213>  Homo sapiens (Comment: corresponding to (taken from) GenBank accession
no. AF538691.1)

<400>  3
   acatgctctg tgcttagtaa ccccagtgca acttttttgc tttcccaaaa gttctggcaa      60
   aagtcccaag ctagcacttt aattggccta aattgtgtat atgcttatct ctgaaccaat     120
   cactgtggat tagagatgtc atgctctgat tgaccagacc taggccacat ctctagccct     180
   agctctgagg gtagagttgg cagcactaga gcccatggaa gaagtaagag aggagtcgtt     240
   gctaaaggaa aaatcaaagt gtcattaccg aaccaggaca gatgctgggc agcacatgtg     300
   caccccgtct tcttctcatg ttccagctgc acatcttagt gccccttggt ttagcacttt     360
   tctcattaaa tcatttgctt tcttgcctca cttcctgtgg ttggtagaat gctaagatgg     420
   ccccaagatc tctacccctg gtgtttgcac acctcccagt tattctgtca aacatgaatg     480
   tagatgcttc tgtgaaagaa ttttgcacat gtaatttaag tcccaaattg tttgacctta     540
   aaataaggag aatggcaggg ccaggcatgg tggctcatac ctgtaatccc agcactttgg     600
   gaggccaagg cgggcagatc acgaggtcag gagatcgaga ccatcctggc taacacagtg     660
   aaaccccatc tctactaaaa atacaaaaaa ttagctgggc gtggtggcgg gtgcctgtat     720
   tcccagctac ccaggaggct gaggcaggag aatggcgtga acccgggagg cgtagcttgc     780
   agtgagccaa gatcgtgcca ctgcactcca gcctgggtga cagagccaga ctctgtctca     840
   aaaaaaaaaa aaaaaaggag aatggctttg gtgggcctga cctagtcagg tgagttctta     900
   aaaggcgaca catggcccgg tgcagtggct caggcctgta atcccagcac tttgggaggc     960
   cgaggcgggt ggatcacgag gtcaggagat cgagaccatc ctggctaaca tggtgaaacc    1020
   ccgtctctac taaaaagaca aaaaattagc tgggcgtggt ggtgggctcc tgtagtccca    1080
   gctactcggg aggctgaggc aggagaatgg cgtgaacccg ggaggcggag cttgcagtga    1140
   gcggagattg cgccactgca ctccagcctg ggcgacagag cgagactccg tctcaaaaaa    1200
   aaaaaaaaaa aaaaagaaaa ttaaaagtgg gtattgttgt aagatgctga gtttatggta    1260
   gtttgttaca tgacaataga aaatgaacac acttcacagt ggactccaag atccccatga    1320
   tctttgatct ccttaacctc ctgatctcca caggacccag agcataagaa tgtcccttct 1380
   tctgcttcca gtcccactat ctagaaaaga gaggaggagc ccagctcttc atttcacccc 1440
   cacccacaaa ctcccaactt tccggccctc aaggggtgac caaggaagtt gctccacttg 1500
   gctttccaca aacagcctgt gccccaccag gctcaggagg gcagcttgac caatctctat 1560
   ttccaagacc tttggccagt cctattgatc tggactcctg gataggcagc tggaccaacg 1620
   gacggacgcc atgagggctc tgctgctcct ggggttcctg ctggtgagct tggagtcaac 1680
   actttcggtg agtgctgtgg gaaccaggat tgtcccagga ttgttctggg gggtcgctat 1740
   cacagccatg agccatggcc tctgctcatg acctgtgggt ccaggtgact aggaggccta 1800
   tgtggaaagg tgaggccagc ccggaaggcc caggcagagg agacagacaa ccagactggg 1860
   tggatacaag ggcacagcct gcatttctgg gggagatggg ccttaagaag acaacggggg 1920
   gaggtagaaa gggtttgggt cttgggaaga aatctctgca tttctgggct gtgagaggaa 1980
   gctgcagact agcaacagat cggtggcagg ctatgactta tagtcagttc cctgccttct 2040
   tctctccctt gtagattcca ccttgggaag cccccaagga gcataagtac aaagctgaag 2100
   agcacacagt cggtaagtgg cctggctcct cctcccggga acccttgggt ggggatgtgt 2160
   atggtgcagt gtgtgcagtc tcagggcagt ctagtctagt gcctacctgg tgctaggtct 2220
   tatgcccatg ggcactagag tgatcgtgag ctgtgtgatc cttgagggca gggtatgggc 2280
   tgtgtctaag tgcccacgag cctggctcgg agcaggtgct tgagatatgt gctgctggcg 2340
   ccatcacacc tgggctcctg ccagccttcc tcagtttccc cagcttctcc ccttcttttc 2400
   ctttccccag tacgtctcat gggcatcatt catgccacac agaggccagg gccttcaatg 2460
   ggcaaggaag gatcaagagc ttgtctctgg catctgaatg cctctgaagc ccagctttat 2520
   cacttatgag ctgggtgact ctgggcgagg gatttgagtt ctccaagctt caatttcccc 2580
   ttctgtgaaa ccaggttgat aacagtaaac ctcttagggt tgttgagaag ggaaacccat 2640
   gtgaggtatt cagcccatca cctggtgcat ggaaatgctt tacaaatatt agcttttatt 2700
   atgaaactac cttttagatg aagggtacct gccatttccc ccttcctcaa gctctgccat 2760
   agctccccat tgctttcatt cttccagaca ctaaattacc tacatgccag gcatggtggc 2820
   tcatgcctgt aatcccagca ctttgggagg ccaaggtcgg tggatcatga ggtcaggagt 2880
   tcgagaccag cctggccaac atggtgaaat gctgtctcta ctaaaagtac aaaaattagc 2940
   caggcatggt ggcatgcgcc agtagtccca gctactcggg aggctgaggc agaagaattg 3000
   cttgaacctg ggaggtgaag gttgcagtga acgaagatca caccattgca ctccagcttg 3060
   ggcaacacag caagactccg tctcaaaaaa aaaaaaaaaa tttacctaga gtgtggcaca 3120
   tagcagggcc tgtgaaccag atggacctta ccctggtggg cctgacttgg tggggttgag 3180
   tctctaagca tggcgttgag gcccagcaca ttccaaccct ggactccctc agcctcctct 3240
   cttcacccca cacccaaaag tttctcctct ctcttgcctt acccaaactt ggtgccctat 3300
   ccttgcctaa tcccctgcct aaggtccccc tcctctctgt ccgtccatcc catctgcatc 3360
   tttttttttt tttgagatgg agtctcgctc tgtcccctag gctagagtgc aatggcgcga 3420
   tcttggctca ttgcaacctc cgcctcctgg gttcaagcga ttctctgcct cagcctcccg 3480
   agttgctggg attacaggca cacaacttca tgctcagcta atttttgtat tttttagtag 3540
   agacagggtt tcaccatgtt ggccaggctg gtctcgaact cctgccctca ggtggtccgc 3600
   ccaccttagc ctcccaaagt gctgggatta caggcgtgag ccaccgcgcc tggcccccat 3660
   ttgcatctta aaggtccatc tcagatccat ttccatttac tgtcctagtt ctggtttggt 3720
   ccttggcaag tgcactttgc cttgaacaaa atagtggcaa aagcttattg agcaggtact 3780
   ttgtgccaga cactgctcag catttcatgg cattatctca tgaagcccca cgacaattcc 3840
   tctgaagaag acacaggcaa ttctcattat tcgcgatggt tatgttctat aaaatcacag 3900
   tgaacattga actagcaaac agtattaggt tcctgtgagc ctctggtcac aacattttca 3960
   tcaaccaaca gcatataatc tggttttatg tatgattctg tttaaagaca ttttatttag 4020
   tatatgtgtt gctgattcat caatgctaag ctgatggcac tatagcacac acctgaatca 4080
   agtgtctaac acacgctttc tccctaaggt agccttcttg tgcttaggaa ctacacagct 4140
   cttcagcagg aggctcagag gccatttcca aaagccaaat ccccagcaaa agcacaaagt 4200
   gtgaaaaacg ttgcactaag tagactgaga aggacactca ttcaatagga gagctgaaac 4260
   aagcagcagc agcgtgacgc cttgttgaac cttaactggg aatgtgcaaa tttttcactg 4320
   ctctgtgcat gcccacaaat ggccatgaaa acatttcaag tattgacttg ggagttacaa 4380
   ataaaattca gcaagtaggc acattctcaa tgtagaacca gagaagaatg aggatcaact 4440
   gtactattat tactgccgtt ttacagataa ggaaaccaag gctcagatca gagtggttaa 4500
   cagtgacttc aacattcaac aagtattatt aagtgcctac tttgtggcaa gtgctcttcc 4560
   tggccttggg actgaagact tacccaaggt cacacagcta gcaggttgtg gagtcaggag 4620
   tctactccag ctatctgact cctgaaccca agtttttttt tttttcttta agatggagtc 4680
   tcactctgtc acccaggctg gagtgcagtg gcgcgatctc ggctcactgc aagctccgcc 4740
   tcccgggttc acaccattct cctgcctcgg cctcccgagt agctgggact acaggcacct 4800
   gccaccaccc ccagctaatt tttttgtatt tttagtagag agggggtttc actgtattag 4860
   ccaggatggt cttgatctcc tgacctcgtg atctgcccgc cttggcctcc caaagtgctg 4920
   ggattacagg cttgagccac cgcgcccggc cctgaaccca acttttagag cagaaagtgt 4980
   tttcaatgca cagcgacctt tttgagggtc tgtccttttc ctgaccagac cctgagggac 5040
   agtgcctgag cagttgagta caggggaagt cctcagagag tgtgttgtcc ctgcagttct 5100
   cactgtcacc ggggagccct gccacttccc cttccagtac caccggcagc tgtaccacaa 5160
   atgtacccac aagggccggc caggccctca gccctggtaa gactacgcag aggagttgga 5220
   gcaggggcct gggagacatg taccctgcct gtccttctgt ccaaggaact ctgcttggag 5280
   agaggggact gtgatagggc agggtgggcc aggcccctgg gtagagcagg gaagccttgt 5340
   ctctttctac aggtgtgcta ccacccccaa ctttgatcag gaccagcgat ggggatactg 5400
   tttggagccc aagaaagtga aaggtgctac acacagcctc tggggtggcc tggggctctc 5460
   tcctcccgcc tcattactct cctggtatca ccagacccca cacacctggg attctggacc 5520
   cagccccttc tctccctcca caataccctt tggaagtcca gagggagagt tctgggaagg 5580
   agtggtccca ttttgcaggt gggtaaacca agcttggaaa cttggagtag caaggtcaca 5640
   aggcaagtag gttcaagaag ggccttggcc cccagctgtg tgactcagct ccctgctctt 5700
   ccttccacca tgtccatctc tcagaccact gcagcaaaca cagcccctgc cagaaaggag 5760
   ggacctgtgt gaacatgcca agcggccccc actgtctctg tccacaacac ctcactggaa 5820
   accactgcca gaaaggtgag gagatgtgga ggacctgggc ggggtgctgg gggacagggg 5880
   caaccctggg cctacagaat aggttgctgg atactcggag acttggcatg gtcctagact 5940
   ctcctgagac cactatccct ctttgtcccc agagaagtgc tttgagcctc agcttctccg 6000
   gtttttccac aagaatgaga tatggtatag aactgagcaa gcagctgtgg ccagatgcca 6060
   gtgcaagggt cctgatgccc actgccagcg gctggccagc cagggtgagc agatggttgg 6120
   gaacgggcca gggaggagcg tcaggaagac aggctggcag gaggccgggt ggtgtgccgg 6180
   gaaggagagc tctctggggg ggtctttagg cccaggggtg gctcactgcg ttccctcccc 6240
   aagcctgccg caccaacccg tgcctccatg ggggtcgctg cctagaggtg gagggccacc 6300
   gcctgtgcca ctgcccggtg ggctacaccg gacccttctg cgacgtgggt gagtgagggt 6360
   ctggggcaag cagaaggcca gcccccaggt gggacgggct tgccaggaag gaggagggag 6420
   agtgcggaaa gcagatgaga gggaggcagg agagcccagc cttggctgcc cagggagccc 6480
   cctttctcct cagacaccaa ggcaagctgc tatgatggcc gcgggctcag ctaccgcggc 6540
   ctggccagga ccacgctctc gggtgcgccc tgtcagccgt gggcctcgga ggccacctac 6600
   cggaacgtga ctgccgagca agcgcggaac tggggactgg gcggccacgc cttctgccgg 6660
   tgcgccgcgt ggggctgggt gacccctccg ccccagggct ccgggctccc ggcgctctaa 6720
   cggcgccccg tcgtgtggct acaggaaccc ggacaacgac atccgcccgt ggtgcttcgt 6780
   gctgaaccgc gaccggctga gctgggagta ctgcgacctg gcacagtgcc agaccccaac 6840
   ccaggcggcg cctccgaccc cggtgtcccc taggcttcat gtcccactca tgcccgcgca 6900
   gccggcaccg ccgaagcctc agcccacgac ccggaccccg cctcagtccc agaccccggg 6960
   aggttaggaa gtgggggggg gaaggaggag ccgagagggc gccgggcgag ctagattccg 7020
   gccagccggc cgcgggctcc ccgtcctcag cccctgctcc tccacagcct tgccggcgaa 7080
   gcgggagcag ccgccttccc tgaccaggaa cggcccactg agctgcgggc agcggctccg 7140
   caagagtctg tcttcgatga cccgcgtcgt tggcgggctg gtggcgctac gcggggcgca 7200
   cccctacatc gccgcgctgt actggggcca cagtttctgc gccggcagcc tcatcgcccc 7260
   ctgctgggtg ctgacggccg ctcactgcct gcaggaccgg cgagtacccg cccgcccaga 7320
   gccgccccag gggccgcggc tcctccgtct cccagcgcag cttccacgct gcacccgaac 7380
   ccgtgcccta ccttctcccg ccccaccctt ctttccacgc ccctccggag ctcccgggga 7440
   ggaagctgga acacgggatt ggggttcggg agcagggggc ttccccagaa cgcttgtggc 7500
   caggtctgag agcgctgcct ctcccctacc ctccccgcag gcccgcaccc gaggatctga 7560
   cggtggtgct cggccaggaa cgccgtaacc acagctgtga gccgtgccag acgttggccg 7620
   tgcgctccta ccgcttgcac gaggccttct cgcccgtcag ctaccagcac gacctgggtg 7680
   cgtgggggcg ccccgcgggg acgggaagag agcttggggc cccggcgtcc ccgcctcacg 7740
   ctcctctccg cccgggttag ctctgttgcg ccttcaggag gatgcggacg gcagctgcgc 7800
   gctcctgtcg ccttacgttc agccggtgtg cctgccaagc ggcgccgcgc gaccctccga 7860
   gaccacgctc tgccaggtgg ccggctgggg ccaccagttc gagggtaggc acaactgcta 7920
   ggggcagggg taggggagga gacctttgat cactgggtta ggcggaagaa gcccgcgact 7980
   ttggtatcgt tccgggtgcc tacagaatgg gtggcgctga cctgatgggt tgtgagaatg 8040
   tgtaggtgaa tcccaggtag aatcccaggg cctgggattc actgctggga tccccaaatc 8100
   tcctggggat acagggagaa tcgaacttgc tcttggttcc ctctgggcgc cgggctgcaa 8160
   aggccaacta ggacgctggc cccgcgctcc gggctagtgt gggagccagg ttctgcgact 8220
   ctggatgggt ggtgggggag gggtttctgt ttccgctccg cccattcaaa tcctggcttt 8280
   tctctggacc tcagcctcct tgcctatgaa attgaattaa tggcacctcc tccccttcgg 8340
   gcttgctgcg agagaggaag ggcatgagtg ggtttacaag cgcctggagc agctttgtcc 8400
   atcgtccggg cggcaagcgt tgtcagatgg ggtgtgaaga aggcgctctg tgttcgcagg 8460
   ggcggaggaa tatgccagct tcctgcagga ggcgcaggta ccgttcctct ccctggagcg 8520
   ctgctcagcc ccggacgtgc acggatcctc catcctcccc ggcatgctct gcgcagggtt 8580
   cctcgagggc ggcaccgatg cgtgccaggt gagctcttag cccggttggc gcccttcccc 8640
   gaggccgtca ggcacaaatc tcaggtccac agcgctgagc tgcgtgtttc cgacccaggg 8700
   tgattccgga ggcccgctgg tgtgtgagga ccaagctgca gagcgccggc tcaccctgca 8760
   aggcatcatc agctggggat cgggctgtgg tgaccgcaac aagccaggcg tctacaccga 8820
   tgtggcctac tacctggcct ggatccggga gcacaccgtt tcctgattgc tcagggactc 8880
   atctttccct ccttggtgat tccgcagtga gagagtggct ggggcatgga aggcaagatt 8940
   gtgtcccatt cccccagtgc ggccagctcc gcgccaggat ggcgcaggaa ctcaataaag 9000
   tgctttgaaa atgctgagaa ggaaagctct tttcttcatg ggtcccgccg ggaaatgcca 9060
   agacagaaaa gcgattcaca gcttctccac agctctcaga gaacaaggtc tatgagatct 9120
   taacgtgcaa aatctagatg ccagcccagc taatgtttac tgagcctagg atactgtata 9180
   ccaagccctg tgcaaggaga agctgcatgt tattccttat gagaaactaa cattttgttt 9240
   acagagcagt agttctcaga ccatacatta agatcacttg ggagcgtttt tgagccaatc 9300
   tatgcccaag ttccacctca gaccaattaa atcagtatgt ctagggatgg ggcatgggta 9360
   gtggtatatt tgtaaaactc cccagataat tccatgtaca gccaaggttg agaatcgtgg 9420
   ttagaaatac ttagcattgg ccgggcgcgg tggctcacgc ctgtaatcct agcactttaa 9480
   gaggccaagg caggtggatt gctcaggagt tcgaaaccag cctgggcaac acgatgaaac 9540
   cccgtctcta ctaaaataca agaaattagc cgggcacggc ggcgtgcgcc tgtagtccca 9600
   gctactcagg aggctgaggc aggagaatca cttgaaccgg caggaaggaa ggaaggaagg 9660
   aacagaggga gggaaagaga gagacagaaa gaaaagaaaa aagaaaatag aaaaaaagag 9720
   cattgactgt ggcgtggacc ctaagggctg ggtgacatat cgttgtcccc accccaacac 9780
   gcactagtgt agtgggtctg agagtccctt ggctagcagt accatcacca gggaacttgt 9840
   tacacataac aaattctcgg gctacacttt atactgctga acagaaagtc tggggtgggg 9900
   cccagcaatc tgtttaacag ccttgcgggg gattctgatg ttctctcatg cttaagaacc 9960
   acaatctggg ggttgaatgg ttggttccct tacaagtgaa ggtctggctg tccagacaca 10020
   acatcctttt ttcacaaaac cagcttttta aaattaaaaa tagattggcc agatgcggtg 10080
   gttcacgcct gtaatctcgt cactttgaga ggctgaggcg ggaggattgt ttgagctcaa 10140
   gacttcctga cccgcctggg caacatagtg agacctcatc tcaaaaaaat tttttttaat 10200
   taaaatttgt ttttgctttt ttagagacgg ggcctcgctc tgtggctcag gctggcgtgc 10260
   agcgacacga tcctataata gtttactata atctcgctac tgagttcaag cgatccgccc 10320
   gcctcggcct cccaaagcgc tgggattaca ggagtgagcc gctgcgctct gccaaaccca 10380
   tcctacagga taaccttaga actgcgacag cactaaacgc ccacgcccca cgtgccccag 10440
   cctgggtggt cgctccggga cggcgccttg tgtgacgtca cagccccgcc cagcctgcct 10500
   cacagcgccg caggccttcc ccgcgtggcg cctctatatt tccccgagag gtgcgaggcg 10560
   gctgggcgca ctcggagcgc gatgggcgac tggaaggtct acatcagtgc agtgct 10616

<210> 4

<211> 2420

<212> DNA

<213> Homo sapiens (Comment: Extended upstream genomic sequence (extending the upstream sequence given in AF538691.1), corresponding to (taken from) UCSC Genome Browser/July 2003 human reference sequence/ >chr5:176819111-176821530 (reverse complement))

<400> 4
agccgggtgt ggtggctcac gcctgtaatt ccagcacttc gggaggccga ggtgggcaga 60

tcatgaggtc aggagttcga gaccaacctg gccaacatgg tgaagccctg gtctctacta 120

aggagacaaa aaattagccg ggcatggtgg cgtgggcctg taatggcagc tacttgggag 180

gctgaggcag gagaatcact tgaacctggg aggtggaggt tgtagtgagc cgagatcgca 240

ccatggcagt ccagtttggg cgacagcgcg agactccatc tcaaataaat aactaaataa 300

aaataataat aaagtaccct ttcaccacca cagctgggga ggggtgatca caaaatgaaa 360

ctgggagagg aagaggaagg gagatggatg ttgaacatcc aaatgaccat taatgtccac 420

ctgacacctc atctcacact ccctacaagg caccttatgc tcaccacatt ccccatttga 480

cacgctgcat tcaggcttct gtgcagttgt attactcact ctgcctgcaa acccctactt 540
gtggcgaaga cctgttcaaa aggccccttc catgtagagg aacagctgcc ttctccccaa 600
gaggaaggag ccaatgtggg agtggcctta gggccagggt ctatactcag gagcaatcaa 660
gaactgacag agccaggcat ggtggcatgc acctgtaatc ccagctattc aggaggatga 720
agcgagagga tcacttgcgc ccagaagttc caggctagcc tgggcaactt aataagaccg 780
agtctctttg aaacaacaaa aaaggtgaca aaggtagttt cgctttaatg actcgtcaat 840
atatctaata gtttcacttt catcatggag ttaacagctg tcacgtcaat gactttgaaa 900
ccagttacct ttgaaaccag tggttggaaa ggtgcccctc ctctcctgcc ctcctctttc 960
tgtgtttctg ttccaagaag tcttccacag cctgcctctc ttccagcaga attaataaat 1020
tgctacactc agtcttcatt tcccataaat tactttgtat aacttaggac tttgtataac 1080
tgaaagtgac agaagtccac tcaaattggt tttattggct catataactg agaagtctat 1140
tttttttttt tttggagata cagtctcagt ctgcttccca gactggagtg cagtggcaca 1200
atcacggctc acagcatttt ggacctctgg gctcaagcaa tcctcctgcc tcagactcac 1260
aagtagccgg aactacaggc acgcgccacc atgcctggct aatgttaaat ttttttggag 1320
agatgggatc tcactatgtt gcctacatca gtctggaact cctgggctca agtgatcctc 1380
aagtgatccc cagcctccca gagtgttggg attacaggcg tgagccactg tgcctagcca 1440
taactgacca agtcttaagg atacttctag ttttaggatg taggctctca aagtcatgag 1500
aaatcaatcc atcagtacta gttctcaaat gtcgtgtaac aaatcaccct aacttgtggc 1560
ttaaagcaac aacatttaat gatgatttat cacagtttct gatggtcagt tttgccttga 1620
ggtctttcat aagattgcaa gccaaatgtc agcgagggct gcagtatcca cttccaaggc 1680
agcttattca tgtggctgtc caattggcac tggctgtcag cagggtgctt tcctttctcc 1740
ctacatgggc ttctctgcag ggctgcatgt gtgtcctcac aacacagtgg ctggcttcct 1800
ccagagtgag caacctaaga gaccgaggca gaaactgcaa tgtcttttat gacctgggct 1860
tggaagtcca acaccctcac ttctgccata tttttttttt tttgagacag tccctgtcgc 1920
ccaggctgga gtgcaatggc acaatctctg ctcactacaa cctccacttc ctgggttcaa 1980
gcgattctcc tgcctcagcc tcacgggtag ctgggattac aggcacacgc cactacgccc 2040
ggctaatttt tgtatttttc atagagatgg ggtttggcca acacgttggc caggccggtc 2100
ttgaactcct gacctagagt gatctgcctg ccttggcatc ccaaagtgct gggattacag 2160
gtgtgagcca ccacacccag ccacttctgt catattctga tggacacaca gaacagccct 2220
aactcaatgt gggaagacac catacaaggg catgaatcct aggtgctgag gatcaatggg 2280
ggctgtcttt gaggctgtct accacagcat ctttccatcc ttcctgccct gtttgctttg 2340
cttttcctat gtgtaggctt cattctcaaa caggccctcc ctagagagtg acaaaggtga 2400
tcatcaatgt gttcagaccc 2420

<210>  5

<211>  1402

<212>  DNA

<213>  Homo sapiens (Comment: Extended downstream genomic sequence (extending
the downstream sequence given in AF538691.1), corresponding to (taken from) UCSC
Genome Browser/July 2003 human reference sequence/ >chr5:176807094-176808495
(reverse complement))

<400>  5
gcgggaccag cgcatcgacg acgtggccat cgtgggccat gcggacaaca gctgcgtgtg      60

ggcttcgcgg cccgggggcc tgctggcggc catctcgccg caggaggtgg gcgtgctcac     120

ggggccggac aggcacacct tcctgcaggc gggcctgagc gtgggggccc gccgctgctg     180

cgtcatccgc gaccacctgc tggccgaggg tgacggcgtg ctggacgcac gcaccaaggg     240

gctggacgcg cgcgccgtgt gcgtgggccg tgcgccgcgc gcgctcctgg tgctaatggg     300

ccgacgcggc gtacatgggg gcatcctcaa caagacggtg cacgaactca tacgcgggct     360

gcgcatgcag ggcgcctagc cggccagcca ggccgcccac tggtagcgcg ggccaaataa     420

actgtgacct gggcgcggct ggctcctcct ccacttgcgc ggtgggggga gttgtaaata     480

aggaaactgg tctttgcaag acggttacct ggtggagccg ggattttgag tctagaggct     540

gccaggcccc tgtgccctac accctgctct cccatggacg ccttgcagag gctcctggcc     600

tgactgctgc tccttggcgc gttcccaggg tcctagggac tccgcagctg aggaagagtc     660

caagggtggg ggcttctcaa agtctgtttc agccttagcg tcctttctca gagatattcc     720

cacacattag gcaggacaag taaagggagc cccctcccca tcccgcgaac acctctcccc     780

atcagggtgt caggctggag gccaatttgc tcctcccccc tccactcata cctcaagcac     840

tagcaagttg tgagtgggtg acaggatggg cttggtggct tgtaaagcag ttctggggct     900

cacaggcctc tgcatctctg cccacattcc tccaagggga gcctactgag agggctcatg     960

tccaagacca tcgcaattgg gtttgagacc ttacatcctg ccttccccag gccttcgaaa    1020

aggccccgca ggagtccctg gactagaggg aggaactctg gcatccctac ccgggagtct    1080

cactctgcag gcctcagttt cagggtgacc tatggaggag ggggaattga aaagcttggg    1140

taagtttgag gcctggttta ttgcccaaag atagtggaca aaagtgggag ggagggtgtc    1200

tggtccctgc cctccatgtg ctggggccca gggcatggcc tctcttgccc acccccaccc    1260

ttcccgtccc ctcccccagc ggccctgatg gcagacccca cctgtcactt attctggagc    1320

cctgatctta tcccagcagg aaggagtgat gtgtggctga ggtgggtgaa tttagagggc    1380

agagggagac cagaggaagt tc                                             1402